(19) Europäisches Patentamt
European Patent Office
Office européen des brevets

(11) EP 1 293 568 A1

(12) EUROPEAN PATENT APPLICATION
published in accordance with Art. 158(3) EPC

(43) Date of publication:
**19.03.2003 Bulletin 2003/12**

(21) Application number: **01941123.0**

(22) Date of filing: **20.06.2001**

(51) Int Cl.⁷: **C12N 15/12**, C12P 21/02, C07K 14/47, C12N 5/10, C07K 16/18, A61K 45/00, A61P 3/12, G01N 33/566, G01N 33/50, G01N 33/15

(86) International application number:
**PCT/JP01/05263**

(87) International publication number:
**WO 01/098495 (27.12.2001 Gazette 2001/52)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.06.2000 JP 2000191088**

(71) Applicant: **Takeda Chemical Industries, Ltd.**
**Osaka-shi, Osaka 541-0045 (JP)**

(72) Inventors:
- **KUROKAWA, Tomofumi**
  **Kawake-gun Hyogo 666-0251 (JP)**
- **YAMADA, Takao**
  **Matsubara-shi, Osaka 580-0003 (JP)**
- **MORIMOTO, Shigeto**
  **Osaka-shi, Osaka 543-0001 (JP)**

(74) Representative: **Rickard, Timothy Mark Adrian**
**Takeda Patent Office,**
**11-12 Charles II Street**
**London SW1Y 4QU (GB)**

# (54) NOVEL PROTEIN AND DNA THEREOF

(57) The present invention relates to a phosphatonin protein or a salt thereof and the like.

The protein of this invention, a partial peptide thereof or a salt thereof, and DNAs encoding them can be used for obtainment of antibody and antiserum, construction of an expression system of the protein of this invention, screening of a candidate compound for pharmaceutical product using this expression system and the like.

EP 1 293 568 A1

## Description

## Technical Field

The present invention relates to a novel protein "phosphatonin" having a phosphaturic activity and/or a hypophosphatemia-inducing activity, and a DNA thereof.

## Background Art

Phosphorus plays an important role in cell metabolism, and is essential as a constituent component of nucleic acid, adenosine triphosphate (ATP), phospholipid, cell membrane and intermediate metabolite or for the storage and transport of cellular energy. In addition, calcification of bone is closely related to concentrations of phosphorus and calcium in blood, and once the homeostasis of these components is lost, the strength of the bone is seriously affected.

The phosphorus concentration in blood is elaborately balanced by the absorption from small intestine, release from bone, excretion from kidney, shift inside and outside cell and so on. In the kidney, phosphorus is passively excreted by glomerular filtration, meanwhile sodium-dependant phosphorus ($Na^+$-Pi) transporter present in the brush border membrane of renal proximal tubule actively reabsorbs phosphorus, greatly contributing to the maintenance of phosphorus concentration in blood.

Vitamin D is metabolized in the liver and the kidney into an active form, 1,25-dihydroxy vitamin D ($1,25(OH)_2D$), thereby contributing to the maintenance of phosphorus concentration in blood and calcification of bone. When vitamin D is insufficient, children in the growth period develop rickets and adults develop osteomalacia. As hereditary rickets due to a cause other than lack of vitamin D, X-linked hypophosphatemia (XLH), autosomal dominant hypophosphatemic rickets (ADHR), hereditary hypophosphatemic rickets with hypercalciuria (HHRH) and the like are mentioned. In these diseases, mutation in a causative gene causes rickets and osteomalacia that develop phosphaturia and hypophosphemia, but the mechanism are not the same and includes many unclear aspects.

In 1995, a gene located at Xp22.1, PHEX (phosphate regulating gene with homologies to endopeptidase, on the X chromosome), was cloned as a causative gene of XLH (NATURE GENETICS 11, 130-136, 1995). The PHEX gene product is one transmembrane glycoprotein consisting of 794 amino acid residues, which has a high homology with zinc metalloendopeptidase. When a blood vessel of Hyp mouse known as a model mouse of XLH and that of a normal mouse were artificially bound to provide a state where humoral factors can freely communicate (parabiosis), hypophosphemia could be induced in normal mouse (JOURNAL OF BONE AND MINERAL RESEARCH, 4, 493-500, 1989). When the kidney of a normal mouse was transplanted to Hyp mouse, hypophosphemia in Hyp mouse was not improved (JOURNAL OF CLINICAL INVESTIGATION 89, 1453-1459, 1992). From these results, an idea that the etiology of Hyp mouse is humoral phosphaturic factor produced in an organ other than the kidney has been proposed.

As a disease showing a symptom highly similar to XLH, oncogenic hypophosphatemic osteomalacia (OHO) can be mentioned. This disease is mainly caused by mesodermal tumor in bone (benign osteoblastoma, mesenchymoma ossificans), soft tissue (angioma, desmoma) and the like, which leads to significant leakage of phosphorus from the kidney, causing hypophosphemia and osteomalacia (Tumor associated rickets and osteomalacia Favus MJ (ed) AMERICAN SOCIETY FOR BONE MINERAL RESEARCH, 184-188, 1990). It is considered that the main etiology of this disease is different from that of XLH (CURRENT OPINION IN NEPHROLOGY AND HYPERTENSION 7, 367-376, 1998). As the etiology of OHO, the presence of humoral phosphaturic factor excessively secreted by a tumor has been suggested from the fact that, the symptoms of OHO disappeared completely upon removal of the tumor, transplantation of tumor of OHO patients to nude mouse or continuous administration of the tumor extract to rat induced hypophosphemia (JOURNAL OF CLINICAL ENDOCRINOLOGY AND METABOLISM 67, 46-53, 1988; JOURNAL OF PEDIATRICS 91, 56-60, 1977) and the like. In recent years, this factor is called "phosphatonin", and suggested to suppress reabsorption of phosphorus by $Na^+$-Pi transporter of renal proximal tubule in experimental systems using human or animal culture kidney cells (PROCEEDINGS OF ASSOCIATION OF AMERICAN PHYSICIANS 107, 1296-1305, 1995; BONE, 18, 159-169, 1996).

From the similarity in the pathology between XLH and OHO, it is considered that phosphatonin secreted by a tumor in OHO is also secreted in the blood in healthy subjects, which is degraded and controlled well by a PHEX gene product. A mechanism involving defective PHEX gene in XLH, and excess secretion of phosphatonin from tumor in OHO to develop hypophosphemia can be assumed. Accordingly, isolation and identification of humoral phosphaturic factor secreted by tumor of OHO patients to clarify the properties thereof are expected to greatly contribute not only to the elucidation of the relationship between OHO and XLH but of any disease caused by abnormal control of phosphorus concentration in blood.

Recent reports have documented that phosphatonin was separated from a primary culture supernatant of OHO tumor cells (NEW ENGLAND JOURNAL OF MEDICINE, 330, 1645-1649, 1994, BONE, 18, 159-169, 1996) and that a phosphatonin candidate gene was cloned (BONE, 23, S653, 1998). Considering the fact that the gene sequence of phosphatonin reported by Rowe P.S.N. (WO 99/60017) lacked 5' region including the initial methionine, and that its recombinant product showed an function opposite to the physiological function of phosphatonin, a full-length gene sequence of phosphatonin and a gene product thereof have not been clarified yet.

## Disclosure of the Invention

The present invention aims at providing a novel protein having a phosphaturic activity and/or a hypophosphatemia-inducing activity, its partial peptide or its salt, a DNA encoding said protein, recombinant vectors, transformants, methods for manufacturing said protein, pharmaceutical agents comprising said protein or DNA, antibodies against said protein, methods for screening receptor agonist/antagonist and a screening kit of receptor agonist/antagonist, a receptor agonist/antagonist obtained by said screening, methods for screening a compound having an inhibitory action on a proteinase that degrades the protein or a salt thereof, and a screening kit thereof, and a compound obtained by said screening or a salt thereof.

Isolation of a novel protein having a phosphaturic activity and/or a hypophosphatemia-inducing activity clarifies the onset mechanisms of OHO and XLH and reabsorption mechanism of phosphorus in the kidney, and leads to the development of a new pharmaceutical product useful for the prophylaxis or treatment of various diseases caused by abnormal control of phosphorus concentration in blood.

As a result of intensive studies done by the present inventors, they have succeeded in cloning cDNA having a novel base sequence, from a cDNA library derived from OHO patients. The present inventors have found that a protein encoded by the obtained cDNA is a novel protein having a phosphaturic activity and/or a hypophosphatemia-inducing activity, and they have studied further based on such finding, which resulted in the completion of the present invention.

Accordingly, the present invention provides

(1) a protein comprising an amino acid sequence identical or substantially identical to an amino acid sequence consisting of amino acid Nos. 17 - 525 of the amino acid sequence presented by SEQ ID:1, or a salt thereof,
(2) a protein comprising an amino acid sequence consisting of amino acid Nos. 17 - 525 of the amino acid sequence presented by SEQ ID:1, or a salt thereof,
(3) the protein of (1) or a salt thereof, which is a protein comprising an amino acid sequence identical or substantially identical to the amino acid sequence presented by SEQ ID:1, or a salt thereof,
(4) the protein of (3) or a salt thereof, which is a protein having the amino acid sequence presented by SEQ ID:1 or a salt thereof,
(5) the protein of (1)-(4) or a salt thereof, which is a protein having a phosphaturic activity and/or a hypophosphatemia-inducing activity,
(6) the protein of (1)-(4), which is a protein having at least one activity selected from (i) an activity that suppresses a sodium-dependent phosphorous ($Na^+$-Pi) transport activity in kidney, (ii) an activity that suppresses a 25-hydroxy vitamin $D_3$-$1_\alpha$-hydroxylase activity in kidney, and (iii) an activity that promotes a 25-hydroxy vitamin $D_3$-24-hydroxylase activity in kidney,
(7) a partial peptide of the protein of (1), or a salt thereof,
(8) a DNA comprising a DNA having a base sequence encoding the protein of (1),
(9) the DNA of (8), which has a base sequence presented by SEQ ID:2 or SEQ ID:3,
(10) a recombinant vector comprising the DNA of (8),
(11) a transformant retaining the recombinant vector of (10),
(12) a method for manufacturing the protein of (1), the partial peptide of (7) or a salt thereof, which comprises culturing the transformant of (11) to produce and accumulate the protein of (1) or the partial peptide of (7) and harvesting the same.
(13) a pharmaceutical agent comprising the protein of (1), the partial peptide of (7) or a salt thereof,
(14) a pharmaceutical agent comprising the DNA of (8),
(15) the pharmaceutical agent of (13) or (14), which is capable of regulating and improving abnormal concentration of phosphorus in blood,
(16) an antibody against the protein of (1), the partial peptide of (7) or a salt thereof,
(17) a method for quantifying the protein of (1), the partial peptide of (7) or a salt thereof, which comprises using the antibody of (16),
(18) a method for diagnosing a disease involved by the protein of (1), the partial peptide of (7) or a salt thereof, which comprises using the quantification method of (17),
(19) a method for screening a receptor agonist or antagonist, which comprises using the protein of (1), the partial peptide of (7) or a salt thereof,
(20) a screening kit of a receptor agonist or antagonist, which comprises the protein of (1), the partial peptide of (7) or a salt thereof,

(21) a receptor agonist or antagonist obtained by the screening method of (19) or by the use of the screening kit of (20),

(22) a method for screening a compound or a salt thereof having an inhibitory action on a proteinase that degrades the protein of (1) or the partial peptide of (7), which comprises using the protein of (1), the partial peptide of (7) or a salt thereof,

(23) a screening kit of a compound or a salt thereof having an inhibitory action on a proteinase that degrades the protein of (1) or the partial peptide of (7), which comprises the protein of (1), the partial peptide of (7) or a salt thereof,

(24) a compound or a salt thereof having an inhibitory action on a proteinase that degrades the protein of (I) or the partial peptide of (7), which is obtained by the screening method of (22) or by the use of the screening kit of (23), and the like.

Moreover, the present invention provides

(25) the screening method of (19), which comprises measuring and comparing the amount of the protein of (1), the partial peptide of (7) or a salt thereof bound to a receptor or a partial peptide thereof, between (i) a case where the protein of (1), the partial peptide of (7) or a salt thereof is brought into contact with the receptor or a partial peptide thereof, and (ii) a case where the protein of (1), the partial peptide of (7) or a salt thereof and a test compound are brought into contact with the receptor or a partial peptide thereof,

(26) the screening method of (19), which comprises measuring and comparing the amount of the protein of (1), the partial peptide of (7) or a salt thereof bound to a cell containing a receptor or a cell membrane fraction thereof, between (i) a case where the protein of (1), the partial peptide of (7) or a salt thereof is brought into contact with the cell containing the receptor or a cell membrane fraction thereof, and (ii) a case where the protein of (1), the partial peptide of (7) or a salt thereof and a test compound are brought into contact with the cell containing the receptor or a cell membrane fraction thereof,

(27) the screening method of (19), which comprises measuring and comparing a cell stimulating activity (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular proteins, lowering of pH and the like) via a receptor in a cell containing the receptor, an $Na^+$-Pi transport activity, a 25-hydroxy vitamin $D_3$-$1_\alpha$-hydroxylase activity, a 25-hydroxy vitamin $D_3$-24-hydroxylase activity or the like, between (i) a case where the protein of (1), the partial peptide of (7) or a salt thereof is brought into contact with the cell containing the receptor, and (ii) a case where the protein of (1), the partial peptide of (7) or a salt thereof and a test compound are brought into contact with the cell containing the receptor,

(28) a pharmaceutical agent comprising a receptor agonist obtained by the screening method of any of (19) and (25) - (27) or by the use of the screening kit of (20),

(29) the pharmaceutical agent of (28), which is an agent for the prophylaxis or treatment of hyperphosphatemia, arteriosclerosis, acute coronary syndrome, heart failure, stroke, chronic glomerulonephritis, diabetic nephropathy or kidney failure,

(30) a pharmaceutical agent comprising a receptor antagonist obtained by the screening method of any of (19) and (25) - (27) or by the use of the screening kit of (20),

(31) the pharmaceutical agent of (30), which is an agent for the prophylaxis or treatment of oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure or the like,

(32) the screening method of (22), which comprises measuring and comparing a cell stimulating activity (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular proteins, lowering of pH and the like) via a receptor in a cell containing the receptor, an $Na^+$-Pi transport activity, a 25-hydroxy vitamin $D_3$-$1\alpha$-hydroxylase activity, a 25-hydroxy vitamin $D_3$-24-hydroxylase activity or the like, between (i) a case where the protein of (1), the partial peptide of (7) or a salt thereof is brought into contact with the cell containing the receptor in the presence of a proteinase that degrades the protein of (1), the partial peptide of (7) or a salt thereof, and (ii) a case where the protein of (1), the partial peptide of (7) or a salt thereof is brought into contact with the cell containing the receptor in the presence of a proteinase that degrades the protein of (1), the partial peptide of (7) or a salt thereof and a test compound,

(33) a pharmaceutical agent comprising a compound or a salt thereof having an inhibitory action on a proteinase that degrades the protein of (1) or the partial peptide of (7), which is obtained by the screening method of (22) or (32) or by the use of the screening kit of (23),

(34) a method for screening a compound or a salt thereof that promotes or inhibits intracellular signal transduction after binding of the protein of (1), the partial peptide of (7) or a salt thereof to a receptor, which comprises using the protein of (1), the partial peptide of (7) or a salt thereof,

(35) a screening method of (34), which comprises measuring and comparing intracellular signal transduction after binding of the protein of (1), the partial peptide of (7) or a salt thereof to a receptor, between (i) a case where the protein of (1), the partial peptide of (7) or a salt thereof is brought into contact with a cell containing the receptor, and (ii) a case where the protein of (1), the partial peptide of (7) or a salt thereof and a test compound are brought into contact with the cell containing the receptor,

(36) a screening kit for a compound or a salt thereof that promotes or inhibits intracellular signal transduction after binding of the protein of (1), the partial peptide of (7) or a salt thereof to a receptor, which comprises the protein of (1), the partial peptide of (7) or a salt thereof,

(37) a compound or a salt thereof that promotes or inhibits intracellular signal transduction after binding of the protein of (1), the partial peptide of (7) or a salt thereof to a receptor, which is obtained by the screening method of (34) or (35), or by the use of the screening kit of (36),

(38) a pharmaceutical agent comprising a compound or a salt thereof that promotes or inhibits intracellular signal transduction after binding of the protein of (1), the partial peptide of (7) or a salt thereof to a receptor, which is obtained by the screening method of (34) or (35), or by the use of the screening kit of (36),

(39) the pharmaceutical agent of (38), which is an agent for the prophylaxis or treatment of oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure, hyperphosphatemia, arteriosclerosis, acute coronary syndrome, heart failure, stroke, chronic glomerulonephritis, diabetic nephropathy, kidney failure or the like, and the like.

**Brief Description of the Drawings**

Fig. 1 shows a base sequence of a DNA encoding the phosphatonin protein of the present invention, which is contained in plasmid pCR-PHOS obtained in Example 1 and an amino acid sequence of the phosphatonin protein of the present invention deduced therefrom (continued on Fig. 2).

Fig. 2 shows a base sequence of a DNA encoding the phosphatonin protein of the present invention, which is contained in plasmid pCR-PHOS obtained in Example 1 and an amino acid sequence of the phosphatonin protein of the present invention deduced therefrom (continued from Fig. 1 onto Fig. 3).

Fig. 3 shows a base sequence of a DNA encoding the phosphatonin protein of the present invention, which is contained in plasmid pCR-PHOS obtained in Example 1 and an amino acid sequence of the phosphatonin protein of the present invention deduced therefrom (continued from Fig. 2 onto Fig. 4).

Fig. 4 shows a base sequence of a DNA encoding the phosphatonin protein of the present invention, which is contained in plasmid pCR-PHOS obtained in Example 1 and an amino acid sequence of the phosphatonin protein of the present invention deduced therefrom (continued from Fig. 3).

Fig. 5 shows hydrophilicity · hydrophobicity of the phosphatonin protein of the present invention obtained in Example 1 as deduced from the amino acid sequence of the protein according to a Kyte Doolittle method.

Fig. 6 shows a plasmid construct of plasmid pTCII-mPHOS-2 obtained in Example 2.

Fig. 7 shows an SDS polyacrylamide gel electrophoresis of the phosphatonin protein of the present invention obtained in Example 3.

Fig. 8 shows HPLC analysis results of the phosphatonin protein of the present invention obtained in Example 3.

Fig. 9 shows an N-terminal sequence analysis of the phosphatonin protein of the present invention obtained in Example 3.

Fig. 10 shows an SDS polyacrylamide gel electrophoresis of phosphorylated Phosphatonin obtained in Example 3.

Fig. 11 shows a calibration curve in Phosphatonin ELISA.

Fig. 12 shows the amount of the phosphatonin protein of the present invention in human serum.

Fig. 13 shows a plasmid construct of plasmid pT-PHOSF-11 obtained in Example 6.

Fig. 14 shows an SDS polyacrylamide gel electrophoresis of the phosphatonin protein of the present invention obtained in Example 7.

Fig. 15 shows the results of activity determination of phosphatonin protein of Example 8.

The protein of this invention is a protein having an amino acid sequence identical or substantially identical to the amino acid sequence presented by SEQ ID:1, more preferably a protein having an amino acid sequence identical or substantially identical to an amino acid sequence consisting of amino acid Nos. 17 - 525 of the amino acid sequence presented by SEQ ID:1.

The protein of this invention may be derived from any type of cells of humans and warm-blooded animals (e.g. guinea pigs, rats, mice, chicken, rabbits, pigs, sheeps, bovines, horses, monkeys and the like), for example, splenocytes, neurocytes, glia cells, pancreatic β cells, bone marrow cells, mesangium cells, Langerhans cells, epidermal cells, epithelial cells, endothelial cells, fibroblasts, fiber cells, muscle cells, adipocytes, immune cells (e.g. macrophages, T cells, B cells, natural killer cells, mast cells, neutrophils, basophils, eosinophils, monocytes), megakaryocytes, synovial cells, chondrocytes, osteocytes, osteoblasts, osteoclasts, mammary gland cells, hepatocytes and interstitial cells, and also precursor cells, stem cells and cancer cells of said cells. The protein may also derived from any tissue in which said cells are present, for exmaple, the brain, each region of the brain (e.g. olfactory bulbs, amyglada, basal ganglia, hippocampus, thalamus, hypothalamus, cerebral cortex, medulla oblongata, cerebelleum), spinal cord, pituitary gland, stomach, pancreas, kidneys, liver, gonads, thyroid gland, gallbladder, bone marrow, adrenal glands, skin, muscle, lung, digestive tract (e.g. large intestine, small intestine and duodenum), blood vessels, heart, thymus, spleen, submandibular gland, peripheral blood, prostate, orchis, ovaries, placenta, uterus, bones, joints, skeletal muscles and the like. The protein may also be synthetic.

An amino acid sequence substantially identical to a 17th - 525th amino acid sequence of the amino acid sequence presented by SEQ ID:1 comprises, for example, an amino acid sequence having about 40% or more, preferably 60% or more, more preferably 80% or more, further preferably 90% or more, most preferably 95% or more homology to the amino acid sequences presented by SEQ ID:1.

Amino acid sequences substantially identical to the amino acid sequence presented by SEQ ID:1 comprises for example, an amino acid sequences having about 40% or more, preferably about 60% or more, more preferably about 80% or more, and further preferably about 90% or more, and most preferably about 95% or more homogoly to the amino acid sequences presented by SEQ ID:1.

As the protein of this invention containing an amino acid sequence substantially identical to a 17th - 525th amino acid sequence of the amino acid sequence presented by SEQ ID:1, proteins having the above-mentioned amino acid sequence substantially identical to a 17th - 525th amino acid sequence of the amino acid sequence presented by SEQ ID:1 and having substantially the same activity as that of a protein containing the 17th - 525th amino acid sequence of the amino acid sequence presented by SEQ ID:1 are preferred.

As proteins containing amino acid sequences substantially identical to the amino acid sequence of this invention presented by SEQ ID:1, for example, proteins having amino acid sequences substantially identical to the amino acid sequence presented by SEQ ID:1 and having substantially the same activity as that of the amino acid presented by SEQ ID:1 are preferred.

As to the substatially same activity, for example, phosphaturic activity, hypophosphatemia-inducing activity, $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity in kidney cells and the like are included. "Substatially same" means that the quality of the activity is same. Therefore, although it is preferable that phosphaturic activity, hypophosphatemia-inducing activity, $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity in kidney cells and the like are equivalent (e.g. about 0.5- to 2-fold), quantitative factors such as the level of activity and the molecular weight of the protein may be different.

In addition, the protein of this invention includes a protein containing (1) an amino acid sequence wherein 1 or more (e.g., 1-80, preferably about 1-20, more preferably about 1-9, more preferably several (1 or 2)) amino acids are deleted from a 17th - 525th amino acid sequence of the amino acid sequence presented by SEQ ID:1, (2) an amino acid sequence wherein 1 or more (e.g., 1-80, preferably about 1-20, more preferably about 1-9, more preferably several (1 or 2)) amino acids are added to 17th - 525th amino acid sequence of SEQ ID:1, (3) an amino acid sequence wherein 1 or more (e.g., 1-80, preferably about 1-20, more preferably about 1-9, more preferably several (1 or 2)) amino acids in a 17th - 525th amino acid sequence of the amino acid sequence presented by SEQ ID:1 are substituted by other amino acids, and the like, which are what is called a mutein.

Furthermore, the protein of this invention includes a protein containing (1) an amino acid sequence wherein 1 or more (e.g., 1-80, preferably about 1-20, more preferably about 1-9, more preferably several (1 or 2)) amino acids are deleted from the amino acid sequence presented by SEQ ID:1, (2) an amino acid sequence wherein 1 or more (e.g., 1-80, preferably about 1-20, more preferably about 1-9, more preferably several (1 or 2)) amino acids are added to the amino acid sequence presented by SEQ ID:1, (3) an amino acid sequence wherein 1 or more (e.g., 1-80, preferably about 1-20, more preferably about 1-9, more preferably several (1 or 2)) amino acids in the amino acid sequence presented by SEQ ID:1 are substituted by other amino acids, and the like, which are what is called a mutein.

When the amino acid sequence is deleted or substituted as mentioned above, the position of deletion or substitution is not particularly limited.

In this specification, the protein is presented according to the conventinal presentation manner of peptides: the left end presents the N-terminal (amino terminal) and the right end presents the C-terminal (carboxyl terminal). In the proteins of this invention including the protein containing the amino acid sequence presented by SEQ ID:1, the C-terminal may be any of carboxyl group (-COOH), carboxylate ($-COO^-$), amide ($-CONH_2$) and ester (-COOR).

For R in the esters, $C_{1-6}$ alkyl groups such as methyl, ethyl, n-propyl, isopropyl, n-butyl and the like, $C_{3-8}$ cycloalkyl groups such as cyclopentyl, cyclohexyl and the like, $C_{6-12}$ aryl groups such as phenyl, α-naphthyl and the like, $C_{7-14}$ aralkyl groups including phenyl-$C_{1-2}$ alkyl groups such as benzyl, phenethyl and the like, and α-naphthyl-$C_{1-2}$ alkyl groups such as α-naphthylmethyl are used, and pivaloyloxymethyl groups and the like, which are commonly used for oral esters, is also used.

When the protein of this invention has a carboxyl group (or carboxylate) at a site other than the C-terminal, the proteins having an amidized or esterified caroboxyl group are included in the proteins of this invention. For the ester form in this case, for example, the C-terminal esters described above and the like are used.

Furthermore, the proteins of this invention also include proteins described above in which the amino group of the N-terminal methionine residue is protected by a protecting group (e.g. $C_{1-6}$ acyl group such as formyl group, acetyl group and the like), those in which the N-terminal is cleaved in vivo and thus produced N-terminal glutamyl group is converted to pyroglutamate, those in which substituents on amino acid side chains in the molecule (e.g. -OH, -SH, amino group, imidazole group, indole group, guanidino group and the like) are protected by appropriate protecting groups (e.g. $C_{1-6}$ acyl group such as formyl group, acetyl group and the like), or complex proteins to which sugar chains are bound, so-called glycoproteins.

More specifically, as the protein of this invention, for example, a protein derived from tumor of human OHO patients, having a 17th - 525th amino acid sequence of the amino acid sequence presented by SEQ ID:1, a protein derived from tumor of human OHO patients having the amino acid sequence presented by SEQ ID:1, and the like are preferred.

The 17th - 525th amino acid sequence of the amino acid sequence presented by SEQ ID:1 is a sequence obtained by deleting secretion signal sequence from the amino acid sequence presented by SEQ ID:1.

The partial peptide of the protein of this invention may be any of peptide having the same activity as that of the aforementioned protein of this invention, for example, phosphaturic activity, hypophosphatemia-inducing activity, $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity in kidney cell and the like.

Specifically, a partial peptide having the 17th - 525th amino acid sequence, a partial peptide having the 17th - 330th amino acid sequence, a partial peptide having the 331st - 525th amino acid sequence and the like of the amino acid sequence presented by SEQ ID:1 are preferably used.

As the partial peptide of this invention, moreover, a partial peptide having an amino acid sequence substantially identical to the amino acid sequence presented by SEQ ID:1, and having a substantially same activity as that of a peptide having the amino acid sequence presented by SEQ ID:1 is preferable.

As the substantially same activity, for example, phosphaturic activity, hypophosphatemia-inducing activity, $Na^+$-Pi transport activity, 25-hydroxy vitamin $D_3$-1α-hydroxylase inhibitory activity, 25-hydroxy vitamin $D_3$-24-hydroxylase-promoting activity in kidney cell and the like are mentioned. By being substantially the same means that the activities are same in terms of properties. Therefore, it is preferable that the phosphaturic activity, hypophosphatemia-inducing activity, $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity in kidney cell be equivalent (e.g., about 0.5 to 2-fold). The levels of these activities and quantitative factors such as molecular weight of protein and the like may be different.

These partial peptides of this invention also include one that is an (competitive) inhibitory type to the protein of this invention, that is one having an inhibitory activity on the activity of the protein of this invention.

Moreover, as the partial peptide of the present invention, a partial peptide of a protein having a homology of not less than about 40%, preferably not less than 60%, more preferably not less than about 80%, further preferably not less than about 90%, most preferably not less than about 95%, with the amino acid sequence presented by SEQ ID:1 is used. More specifically, the partial peptide of the protein of this invention includes the partial peptide of the protein containing an amino acid sequence wherein 1 or more (e.g., 1-80, preferably about 1-20, more preferably about 1-9, more preferably several (1 or 2)) amino acids are deleted from the amino acid sequence presented by SEQ ID:1, an amino acid sequence wherein 1 or more (e.g., 1-80, preferably about 1-20, more preferably about 1-9, more preferably several (1 or 2)) amino acids are added to the amino acid sequence presented by SEQ ID:1, an amino acid sequence wherein 1 or more (e.g., 1-80, preferably about 1-20, more preferably about 1-9, more preferably several (1 or 2)) amino acids in the amino acid sequence presented by SEQ ID:1 are substituted by other amino acids, and the like.

Furthermore, a partial peptide having a homology of not less than about 40%, preferably not less than 60%, more preferably not less than about 80%, more preferably not less than about 90%, most preferably not less than about 95%, with the 17th - 525th amino acid sequence, the 17th - 330th amino acid sequence or the 331st - 525th amino acid sequence (hereinafter these are to be briefly referred to as amino acid sequence A) of the amino acid sequence presented by SEQ ID:1 is used. More specifically, a partial peptide includes an amino acid sequence wherein 1 or more (e.g., 1-80, preferably about 1-20, more preferably about 1-9, more preferably several (1 or 2)) amino acids are deleted from the amino acid sequence A, an amino acid sequence wherein 1 or more (e.g., 1-80, preferably about 1-20, more preferably about 1-9, more preferably several (1 or 2)) amino acids are added to the amino acid sequence A, an amino acid sequence wherein 1 or more (e.g., 1-80, preferably about 1-20, more preferably about 1-9, more preferably several (1 or 2)) amino acids in the amino acid sequence A are substituted by other amino acids, and the like.

In the partial peptides of this invention, the C-terminal may be any of carboxyl group (-COOH), carboxylate ($-COO^-$), amide ($-CONH_2$) and ester (-COOR) (R is as defined above).

Furthermore, the partial peptide of this invention also include partial peptide described above in which the amino group of the N-terminal methionine residue is protected by a protecting group (e.g. $C_{1-6}$ acyl group such as formyl group, acetyl group and the like), those in which the N-terminal is cleaved in vivo and thus produced N-terminal glutamyl group is converted to pyroglutamate, those in which substituents on amino acid side chains in the molecule (e.g. -OH, -SH, amino group, imidazole group, indole group, guanidino group and the like) are protected by appropriate protecting groups (e.g. $C_{1-6}$ acyl group such as formyl group, acetyl group and the like), or complex peptides to which sugar chains are bound, that is glycopeptides.

As the salts of the proteins or partial peptides of this invention, physiologically acceptable salts formed with acids are especially preferred. As such salt, for examples, the salts formed with inorganic acids (e.g. hydrochloric acid, phosphoric acid, hydrobromic acid, sulfuric acid), the salts formed with organic acids (e.g. acetic acid, formic acid, propionic acid, fumaric acid, maleic acid, succinic acid, tartaric acid, citric acid, malic acid, oxalic acid, benzoic acid, methanesufonic acid, benzenesulfonic acid) and the like are used.

The protein of this invention or a salt thereof can be also produced from cells, tissues or plasma of the aforementioned human and warm-blooded animals according to a protein purification method known per se, or by culturing a transformant containing a DNA encoding the protein to be mentioned below. In addition, it can be produced according to the protein synthetic method to be mentioned below or a method analogous thereto.

When the protein is produced from cells, tissues, or plasma of human or warm-blooded animals, homogenate-supernatant of cells or tissues, or plasma of human or warm-blooded animals are/is subjected to a combination of chromatography such as ammonium sulfate precipitation, ethanol precipitation, acid extraction and chromatography, such as ion-exchange chromatography, hydrophobic chromatography, hydroxyapatite chromatography, reversed-phase chromatogtraphy, lectin column chromatography, gel filtration chromatography to isolate and purify the protein.

For synthesis of the protein, its partial peptides, or its salts and amide forms of this invention, commercially available resins for protein synthesis can be used. Such resins include, for example, chloromethyl resin, hydroxymethyl resion, benzhydrylamine resin, aminomethyl resin, 4-benzyloxybenzyl alcohol resin, 4-methylbenzhydrylamine resin, PAM resin, 4-hydroxymethyl methylphenyl acetoamidomethyl resin, polyacrylamide resin, 4-(2',4'-dimethoxyphenyl-hydroxymethyl)phenoxy resin, 4-(2',4'-dimethoxyphenyl-Fmoc aminoethyl) phenoxy resin and the like. Using these resins, amino acids in which the $\alpha$-amino groups and the side-chain functional groups are appropriately protected, are condensed in the order of the sequecne of the objective protein on the resin according to the various publicly known condensation methods. At the end of the reactions, the protein is excised from the resin and the protecting groups are simultaenosuly removed. Then, intramolecular disufide bond-forming reaction is performed in a highly diluted solution to obtain the objective protein or its partial peptide, or its amide form.

For condensation of the protected amino acids described above, various activation reagent for protein synthesis can be used, but carbodiimides are particularly good. For carbodiimides, DCC, N,N'-diisopropylcarbodiimide, N-ethyl-N'-(3-dimethylaminoproryl)carbodiimide and the like are used. For activation by these reagents, the protected amino acids are added with a racemization inhibitor (e.g. HOBt, HOOBt) directly to the resin, or the protected amino acids are previously activated as corresponding acid anhydrides, HOBt esters, or HOOBt esters, then added to the resin. The solvent used for activation of the protected amino acids and condensation with the resin can be selected from solvents known to be useful in protein condensation reaction. For example, N,N-dimethylformamide, N-methylpyrrollidone, chloroform, trifluoroethanol, dimethylsulfoxide, DMF, dimethylsulfoxide, pyridine, chloroform, dioxane, methylene chloride, tetrahydrofuran, acetonitrile, ethyl acetate or N-methylpyrrollidone, appropriate mixtures of these solvents or the like is used. The reaction temperature is selected from the range known to be used in protein bonding reaction, and usually selected from the range from about -20°C to 50°C. The activated amino acid derivatives are usually used in 1.5- to 4-fold excess. The condensation is tested using ninhydrin reaction, and when the condensation is insufficient, sufficient condensation can be obtained by repeating the condensation reaction without elimination of the protecting groups. When the condensation is insufficient even after repeating the reaction, non-reacted amino acids can be acetylated using acetic anhydride or acetylimidazole.

For the protecting groups for amino group of starting material, for examples, Z, Boc, tertiary amyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like are used. For the protecting group for carboxyl group, for example, alkyl ester (e.g., ester group such as methyl, ethyl, propyl, butyl, tertiary butyl, cyclopentyl, cyclohexyl, cycloheptyl, cyclooctyl, 2-adamantyl and the like), benzyl ester, 4-nitrobenzyl ester, 4-methoxybenzyl ester, 4-chlorobenzyl ester, benzhydryl ester, phenacyl ester, benzyloxycarbonyl hydrazide, tertiary butoxycarbonyl hydrazide, tritylhydrazide and the like is used.

The hydroxyl group of serine can be protected, for example, by esterification or etherification. Groups appropriate for this esterification include, for example, lower alkanoyl groups such as acetyl group, aroyl groups such as benzoyl group, and groups derived from carbon such as benzyloxycarbonyl group and ethoxycarbonyl group. Groups appropriate for etherification include, for example, benzyl group, tetrahydropyranyl group, t-butyl group and the like.

As a protecting group of the phenolic hydroxyl group of tyrosine, for example, BzL, $Cl_2$-Bzl, 2-nitrobenzyl, Br-Z, and tertiary butyl or the like are used.

As a protecting group of the imidazole of histidine, for example, Tos, 4-methoxy-2,3,6-trimethylbenzen sulfonyl, DNP, benzyloxymethyl, Bum, Boc, Trt, Fmoc or the like are used.

For activated carboxyl groups in the starting material, for example, corresponding acid anhydride, azide, active ester [ester formed with alcohol (e.g. pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)], or the like are used. Activated amino groups used in the starting material include, for example, corresponding phosphoric amide.

For the method for removing (eliminating) the protecting groups, catalytic reduction in hydrogen gas flow in the presence of a catalyst such as Pd-black, Pd-carbon and the like, acid treatment with hydrogen fluoride anhydride, methanesulfonic acid, trifluoromethanesulfonic acid, and trofluoroacetic acid, and mixture of these acids, basic treatment with diisopropylethylamine, triethylamine, piperidine, piperazine, and the like, reduction by sodium in liquid ammonia, and the like are used. The elimination reaction by the acid treatment described above is generally performed at a temperature ranging from about -20°C to 40°C. In acid treatment, addition of a cation scavenger such as anisole, phenol, thioanisole, metacresol, paracresol, dimethylsulfide, 1,4-butanedithiol, and 1,2-ethanedithiol is effective. 2,4-dinitrophenyl group used as the protecting group of the imidazole of histidine is removed by treatment with thiophenol. Formyl group used as the protecting group of the indole of tryptophan is removed by the acid treatment in the presence of 1,2-ethanedithiol, 1,4-butanedithiol and the like, as well as alkaline treatment with diluted sodium hydroxide solution, diluted ammonia and the like.

Protection of functional groups that should not be involved in the reaction of the starting materials, protecting groups, elimination of the protecting groups, activation of functional groups involved in the reaction, and the like may be appropriately selected from publicly known groups and means.

In another method for obtaining amide form of the proteins, for example, first, the α-carboxyl group of the carboxy termial amino acid is protected by amidation, and the peptide (protein) chain is extended for a desired chain length from the amino group side. Then, a protein in which only the protecting group of the N-terminal α-amino group was removed from said peptide and a protein in which only the protecting group of the C-terminal carboxyl group is removed are produced. These two proteins are condensed in the mixed solvent described above. The details of the condensation reaction are the same as described above. After the protected protein obtained by condensation is purified, all protecting groups are removed by the method described above, and the desired crude protein is obtained. The desired protein in amide form can be obtained by purifying this crude protein using various known means and by lyophilizing the major fraction.

To obtain esterified form of the protein, the α-carboxyl group of the carboxy termial amino acid is condensed with a desired alcohols to prepare amino acid ester, and the desired esterified form of the protein can be obtained by the same procedure as in the preparation of the amide form of the protein.

The partial peptides or its salts of this invention can be manufactured by publicly known method for peptide synthesis or by cleaving the protein of this invesntion with appropriate peptidase. For the method for peptide synthesis, for example, either solid phase synthesis or liquid phase synthesis may be used. The partial peptides or amino acids that may compose the protein of this invention are condensed with the residual portion. When the product has protecting groups, the desired peptide can be obtained by eliminating the protecting groups. The publicly known condensation and elimination of protecting group include the methods described in 1) - 5) below.

1) M. Bodanszky and M.A. Ondetti: Peptide Synthesis. Interscience Publishers, New York (1966)
2) Schroeder and Luebke: The Peptide. Academic Press, New York (1965)
3) N. Izumiya, et al.: Basics and experiments of peptide synthesis, Maruzen Co. (1975)
4) H. Yajima and S. Sakakibara: Biochemical Experiment 1, Chemistry of Proteins IV, 205 (1977)
5) H. Yajima ed.: A sequel to Development of Pharmaceuticals Vol. 14, Peptide Synthesis, Hirokawa Shoten

After the reaction, moreover, the protein of this invention are purified by a combination of conventional purification methods such as solvent extraction, distillation, column chromatography, liquid chromatography, recrystallization and the like. When the protein obtained by the above methods is free form, it can be converted to an appropriate salt form by known methods. On the oher hand, when a salt form is obtained, it can be converted to the free form by known methods.

For the DNA encoding the protein of this invention, any DNA containing the base sequence encoding the protein of this invention described above can be used. Said DNA may be any of genomic DNA, genomic DNA library, cDNA derived from the cells and tissues described above, cDNA library derived from the cells and tissues described above, and synthetic DNA. For the vector used for library, bacteriophage, plasmid, cosmid, phagemid and the like may be used. The DNA may be directly amplified by reverse transcriptase polymerase chain reaction (hereinafter to be briefly referred to as RT-RCR) using mRNA fraction prepared from the cells and tissues described above.

Specifically, as the DNA encoding the protein of this invention having the amino acid sequence presented by SEQ ID:1, for example, (1) a DNA having a base sequence presented by SEQ ID:2, (2) DNA encoding a protein that hybridizes to the DNA having a base sequence presented by SEQ ID:2 and having an activity same as that of a protein having the amino acid sequence presented by SEQ ID:1, for example, phosphaturic activity, hypophosphatemia-inducing activity, $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity in kidney cell and the like, and the like are used.

For the DNA that can hybridize to the base sequence presented by SEQ ID:2, for example, DNA containing a base sequence that have about 40% or more, preferably about 60% or more, more preferably about 80% or more, further preferably about 90% or more and most preferably about 95% or more homology with the base sequence presented by SEQ ID:2 are used.

Hybridization can be performed using publicly known method or the modified method such as the method described in Molecular Cloning, 2nd (J. Sambrook et al., Cold Spring Harbor Lab. Press, 1989). When a commercial library is used, hybridization may be performed according to the attached instruction. Preferably, hybridization may be performed under a high stringent condition.

In said high stringent condition, for example, the sodium concentration is about 19 - 40 mM, preferably about 19 - 20 mM, and the temperature is about 50 - 70°C, preferably about 60 - 65°C. In the most preferred condition, the sodium concentration is about 19 mM and the temperature is about 65°C.

To be more specific, as DNA encoding the protein having the amino acid sequence presented by SEQ ID:1, DNA having a base sequence presented by SEQ ID:2 and the like are used. Moreover, as DNA containing DNA encoding the protein having the amino acid sequence presented by SEQ ID:1, for example, DNA having a base sequence presented by SEQ ID:3 DNA and the like are used.

As DNA encoding the partial peptide of this invention, any can be used as long as it contains a base sequence encoding the aforementioned partial peptide of this invention.

Specifically, as DNA encoding the partial peptide having the 17th-525th, the 17th-330th and the 331st-525th amino acid sequence of the amino acid sequence presented by SEQ ID:1 of this invention, for example, DNA having the 49th-1575th, the 49th-990th and the 991st-1575th base sequence of the base sequence presented by SEQ ID:2 and the like are used.

For the method for cloning the DNA encoding the protein or its partial peptides of this invention, the DNA is amplified by PCR using synthetic DNA primers containing a part of the base sequence of the DNA encoding the protein of this inveniton, or the DNA inserted in an appropriate vector can be selected by hydridization with the labeled DNA fragment encoding a part or entire region of the protein of this invention or synthetic DNA. Hybridization can be performed, for exmaple, by the method described in Molecular Cloning, 2nd (J. Sambrook et al., Cold Spring Harbor Lab. Press, 1989). Hybridization can also be performed when commercial libraly are used according to the method described in the attached instruction.

The cloned DNA encoding the protein or its partial peptide of this invention can be used without treatment or used after digestion with restriction enzymes or addition of linkers when necessary. Said DNA may contain the translational initiation codon ATG at the 5'-end and translational stop codon TAA, TGA, or TAG at the 3'-end. These translational initiation codon and translational stop codon can be added using an appropriate synthetic DNA adaptor.

Expression vectors for DNA encoding the protein or its partial peptide of this invention can be manufactured, for example, as follows: (i) The objective DNA fragment is excised from the DNA encoding the protein of this inveniton, and (ii) the DNA fragment is ligated to downstream of the promoter in an appropriate vector.

For the vector, *Escherichia coli*-derived plasmid (e.g. pBR322, pBR325, pUC12 pUC13), *Bacillus subtilis*-derived plasmid (e.g. pUB110, pTP5, pC194), yeast-derived plasmid (e.g. pSH19, pSH15), bacteriophages such as λ phage, animal viruses such as retrovirus, vaccinia virus and baculovirus, and pA1-11, pXT1, pRC/CMV, pRC/RSV, pcDNAI/Neo and the like are used.

Any promoter that is appropriate and corresponds to the host used for the gene expression may be used as the promoter used in this invention. For example, when animal cells are used as the host, $SR_\alpha$ promoter, SV40 promoter, LTR promoter, CMV promoter, HSV-TK promoter and the like are used.

Among them, CMV promoter, $SR_\alpha$ promoter and the like are preferred. When the host is bacteria of *Escherichia genus,* trp promoter, lac promoter, recA promoter, $\lambda P_L$ promoter, lpp promoter and the like are preferred. When the host is bacteria of *Bacillus genus,* SPO1 promoter, SPO2 promoter, penP promoter and the like are preferred. When the host is yeast, PHO5 promoter, PGK promoter, GAP promoter, ADH promoter and the like are preferred. When the host is insect cells, polyhedrin promoter, P10 promoter and the like are preferred.

In addition to the vectors described above, expression vectors contaninig enhancer, splicing signal, polyA additive signal, selection marker, and SV40 replication origin (hereinafter sometimes to be briefly referred to as SV40 ori) may be used when desired. For the selection marker, for example, dihydrofolate reductase (hereinafter sometimes to be briefly referred to as dhfr) gene [methotrexate (MTX)-resistant], ampicillin resistance gene (hereinafter sometimes to be briefly referred to as $Amp^r$), neomycin resistance gene (hereinafter sometimes to be briefly referred to as Neo, G418-resistant) and the like are used. Especially, when dhfr gene is used as the selection marker using CHO ($dhfr^-$) cells, the objective gene can be selected using thymidine-free medium.

When necessary, a signal sequence appropriate for the host is added to the N-terminal of the protein of this invention. When the host is bacteria of *Escherichia genus,* PhoA signal sequence, OmpA signal sequence and the like are used. When the host is bacteria of *Bacillus genus,* α-amylase signal sequence, subtilisin signal sequence and the like are used. When the host is yaest, $MF_\alpha$ signal sequence, SUC2 signal sequence and the like are used. When the host is animal cells, for example, insulin signal sequence, $_\alpha$-inetrferon signal sequence, the signal sequence of antibody molecule and the like can be respectively used.

Introducing the vectors containing the DNA encoding the protein of this invention constructed as described above to cell, transformants can be manufactured.

For the host, for exmaple, *Escherichia genus, Bacillus genus,* yeast, insect cells, insects, animal cells and the like are used.

Specific examples of the host of *Escherichia genus* are *Escherichia coli* K12 DH1 [Proceedings of the National Academy of Sciences of the USA (Proc. Natl. Acad. Sci. USA) Vol. 60, 160 (1968)], JM103 [Nucleic Acids Research Vol. 9, 309 (1981)], JA221 [Journal of Molecular Biology Vol. 120, 517 (1978)], HB101 [Journal of Molecular Biology Vol. 41, 459 (1969)], C600 [Genetics Vol. 39, 440 (1954)] and the like are used.

For the host of Bacillus genus, for example, *Bacillus subtilis* MI114 [Gene Vol. 24, 255 (1983)] and 207-21 [Journal of Biochemistry Vol. 95, 87 (1984)] and the like are used.

For the host of yeast, for example, *Saccharomyces* cerevisiae AH22, $AH22R^-$, NA87-11A, DKD-5D, 20B-12, *Schizosaccharomyces* pombe NCYC1913, NCYC2036, *pichia pastoris* and the like are used.

For the host of insect cells, for example, when the virus is AcNPV, Spodoptera frugiperda cells (Sf cells), MG1 cells derived from the middle gut of Trichoplusia ni, High Five™ cells derived from Trichoplusia ni eggs, Mamestra brassicae-derived cells, Estigmena acrea-derived cells or the like are used. When the virus is BmNPV, silkworm-derived cells Bombyx mori N (BmN cells) are used. For said Sf cells, for example, SF9 cells (ATCC CRL1711), Sf21 cells (Vaughn, J.L. et al., In Vitro 13, 213-217 (1977)) or the like are used.

For the host of insect, for example, silkworm larvae or the like are used [Maeda et al., Nature, Vol. 315, 592 (1985)].

For animal cells, for example, monkey COS-1, COS-7, Vero cell, Chinese hamster cells CHO (hereinafter to be briefly referred to as CHO cells), dhfr gene-deficient Chinese hamster cell CHO (hereinafter to be briefly referred to as CHO ($dhfr^-$) cells), L cells, myeloma cells, human FL cells, 293 cells, C127 cells, BALB3T3 cells, Sp-2/O cells or the like are used. Among them, CHO cells, CHO($dhfr^-$) cells, 293 cells and the like are preffered.

For transformation of bacteria of *Escherichia genus,* for example, the methods published in Proc. Natl. Acad. Sci. USA Vol. 69, 2110 (1972), Gene Vol. 17, 107 (1982), and the like are used.

Bacteria of *Bacillus genus* can be transformed according to, for example, the method published in Molecular & General Genetics Vol. 168, 111 (1979) and the like.

Yeast can be transformed according to, for example, the methods published in Methods in Enzymology Vol. 194, 182-187 (1991).

Insect cells and insects can be transformed according to, for example, the method published in Bio/Technology, 6, 47-55 (1988) and the like.

Animal cells can be transformed by, for example, the methods described in Cell Technology (Saibo Kogaku) Separate Vol. 8, New Cell Technology Experimental Protocol, 263-267 (1995) (Shujun-sha).

As a method for introducing an expression vector into a cell, for example, calcium phosphate method [Graham F. L. and van der Eb A. J., Virology, 52, 456-467 (1973)], DEAE-dextran method [Sompayrac L.M. and Danna K.J., Proc. Natl. Acad. Sci. USA, 78, 7575-7578, 1981], lipofection method [Malone R.W. et al., (Proc. Natl. Acad. Sci. USA, 86, 6077-6081, 1989)], electroporation method [Nuemann E. et al., EMBO J., 1, 841-845 (1982)] and the like can be mentioned.

In this way, a transformant transformed with an expression vector containing a DNA encoding the protein of this invention can be obtained.

As a method for stably expressing the protein of this invention using animal cells, a method comprising selection of a cell by clonal selection, wherein an expression vector introduced into the above-mentioned animal cell is incorporated into chromosome, can be mentioned. Specifically, a transformant can be selected using the above-mentioned selection marker as an index,. Moreover, repeated clonal selections of animal cells obtained by using a selection marker give a stable animal cell line having highly capable of expressing the protein of this invention.

When a dhfr gene is used as a selection marker, a MIX concentration may be gradually raised during cultivation and a resistant strain is selected. Consequently, a DNA encoding the protein of this invention can be amplified in the cell along with the dhfr gene to give an animal cell line capable of higher expression.

By culturing the above-mentioned transformant under conditions where a DNA encoding the protein of this invention or a partial peptide thereof can be expressed to produce and accumulate the protein of this invention or a partial peptide thereof, the protein of this invention, a partial peptide thereof or a salt thereof can be produced.

For the medium for culturing the trasnformants using the bacterua of *Escherichia* genus or *Bacillus* genus as a host, liquid medium is suitable as the medium used for culture, in which carbon source, nitrogen source, inorganic compounds, and other substances necessary for the growth of the transformants are contained. The carbon source includes glucose, dextrin, soluble starch, sucrose and the like. The nitrogen source includes inorganic and organic compounds such as ammonium salts, nitrates, corn steep liquor, peptone, casein, meat extract, soybean cake, potato extract and the like. The inorganic compounds include calcium chloride, sodium dihydrogen phosphate, magnesium chloride and the like. Yeast extracts, vitamins, growth-stimulating factor and the like may be added. The pH about 5 - 8 is desirable for the medium.

As a medium for culture of bacteria belonging to the Escherichia genus, for example, M9 medium [Miller, Journal of Experiments in Molecular Genetics, 431 - 433, Cold Spring Harbor Laboratory, New York 1972] supplemented with glucose and casamino acid is preferable. For efficient action of the promoter, for example, an agent such as 3β-indolyl acrylic acid can be added as necessary.

When the host is the bacteria belonging to the Escherichia genus, culture is done at generally about 15 - 43°C for about 3 - 24 hr, and where necessary, aeration and agitation may be applied.

When the host is the bacteria belonging to the genus Bacillus genus, culture is done at generally about 30-40°C for about 6-24 hr, and where necessary, aeration and agitation may be applied.

For the medium for culturing the transformant of yeast host, for example, Burkholder minimum medium [Bostian, K.L. et al., Proc. Natl. Acad. Sci. USA Vol. 77, 4505 (1980)] and SD medium containing 0.5% casamino acid [Bitter, G.A. et al., Proc. Natl. Acad. Sci. USA Vol. 81, 5330 (1984)] are used. The pH of the medium is preferably adjusted to about 5 - 8. The culture are generally performed at about 20 - 35°C for about 24 - 72 hours, and aeration or agitation may be added to the culture, when necessary.

When the transformants in insect cell host are cultured, Grace's insect medium (Grace, T.C.C., Nature, 195, 788 (1962)) containing appropriate supplements such as inactivated 10% bovine serum is used as a medium. The pH of the medium is preferably adjusted to about 6.2 - 6.4. Usually, the culture is performed at 27°C for about 3 - 5 days, and aeration or agitation may be added to the culture, when necessary.

When the transformants in animal cell host are cultured, for example, MEM medium containing about 5 - 20% fetal calf serum [(Sience Vol. 122, 501 (1952)), DMEM medium [Virology vol. 8, 396 (1959)], RPMI 1640 medium [The Journal of the American Medical Association vol. 199, 519 (1967)], 199 medium [Proceeding of the Society for the Biological Medicine Vol. 73, 1 (1950)] and the like are used as a medium. The pH is preferably about 6 - 8. Usually, the culture is performed at about 30 - 40°C for about 15 - 72 hours, and aeration or agitation may be added to the culture, when necessary.

Particularly , CHO ($dhfr^-$) cell and dhfr gene are used as selection markers, DMEM medium containing fetal bovine serum dialyze, which does not substancially contain thymidine is preferably used.

Separation and purification of the protein of this invention from the above-mentioned culture product can be performed by, for example, the following method.

When the protein of this invention is extracted from the cultured bacteria or cells, the bacteria or cells are collected after culture by a publicly known method, and suspended in appropriate buffer. Then, the bacteria or cells are disrupted using ultrasonication, lysozymes, and/or by freezing-thawing, and the crude extract of protein is obtained by centrifugation or filtration. The buffer may contain protein-denaturants such as urea and gauanidine hydrochloride and a surfactant such as Triton X-100 (a registered trademark, hereinafter sometimes to be abbreviated as TM).

When the protein is secreted into the culture medium, the supernatant can be separated from the bacteria or cells after culture and collected using a publicly known method. Purification of the protein from the culture supernatant or the extract obtained as described above can be performed by appropriate combination of publicly known methods for separation and purification. These publicly known methods for separation and purification include methods utilizing differences in solubility such as salting out and solvent precipitation, methods mainly utilising differences in molecular weight such as dialysis, ultrafiltration, gel filtration, and SDS-polyacrylamide gel electophoresis, methods utilizing differences in electric charge such as ion-exchange chromatography, methods utilizing specific affinity such as affinity chromatography, methods utilizing differences in hydrophobicity such as hydrophobic chromatography and reversed-phase high performance liquid chromatography, and methods utilizing differences in isoelectric point such as isoelectric focusing.

When the protein of this invention obtained as described above is obtained in the free form, it can be converted to salt by publicly known methods or its modified methods. On the other hand, when the protein is obtained in a salt, the salt can be converted to the free form or other salt by publicly known methods or its modified methods.

The protein produced by transformants can be optionally modified or the partial polypeptide can be removed from the protein by treating the protein with an appropriate protein-modifying enzyme before or after purification. For the protein-modifying enzyme, for example, trypsin, chymotrypsin, arginylendopeptidase, protein kinase, glycosidase and the like are used.

The existence of the protein of this invention produced as described above can be detected by an enzyme immunoassay using specific antibody, and the like.

Antibodies against the protein, its partial peptides, and their salts of this invention may be either polycloncal antibodies or a monoclonal antibody that recognize the protein of this invention, its partial peptides, and their salts (hereinafter sometimes to be briefly referred to as the protein of this invention).

Antibodies against the protein of this invention (hereinafter sometimes to be briefly referred to as the antibody of this invention) can be manufactured according to publicly known methods for manufacturing antibodies and antiserum using the proteins of this invention as antigens.

[Production of a monoclonal antibody]

(a) Establishment of monoclonal antibody-producing cells

The protein of this invention is administered with or without carrier and diluent to sites by which antibody production is induced in warm-blooded animals. To increase the productivity of antibodies, Freund's complete or incomplete adjuvant may be administered. Usually, the administration is performed every 2 - 6 weeks, 2 - 10 times in total. Warm-blooded animals used include monkeys, rabbits, dogs, guinea pigs, mice, rats, sheeps, goats and chickens. Among them, mice and rats are preferably used.

In production of monoclonal antibody-producing cells, animals in which antibody titer are observed are selected from warm-blooded animals immunized with antigen such as mice, and the spleen or lymph nodes are excised 2 - 5 days after the final immunization. Monoclonal antibody-producing hybridomas can be produced by fusing the antibody-producing cells contained in the excised organ with myeloma cells. Antibody titer in an antiserum can be measured by, for example, reacting the labeled protein described below with the antiserum, followed by measurement of the activity of the label bound to the antibodies. The cell fusion can be performed according to known methods such as the method by Köhler and Milstein [Nature Vol. 256, 495 (1975)]. As the fusion-promoting agent, for example, polyethyleneglycol (PEG) and Sendai virus are used, and PEG is preferred.

For myeloma cells, for example, NS-1, P3U1, SP2/0, AP-1 and the like are included, and P3U1 is preferably used. The preferable ratio of the number of antibody-producing cells (spleen cells) to that of myeloma cells is about 1: 1 - 20:1. PEG (preferably PEG1000 - PEG6000) is added at the concentration of about 10 - 80% and the cells are incubated at about 20 - 40°C, preferably about 30 - 37°C, for about 1 - 10 minutes, then, an efficient cell fusion can be performed.

Various methods can be used for screening monoclonal antibody-producing hybridomas. For example, hybridoma culture supernatant is added to the proteinous antigen adsorbed on a solid phase (e.g. microplate) directly or with carrier, and anti-immunoglobulin antibody labeled with radioactive substance, enzyme or the like (when the cells for fusion are mouse cells, anti-mouse immunogloblin antibody is used) or protein A is added, then the monoclonal antibody bound to the solid phase is detected. In other method, hybridoma culture supernatant is added to anti-immunoglobulin antibody or protein A adsorbed on a solid phase, and the protein labeled with radioactive substance, enzyme or the like is added, then the monoclonal antibody bound to the solid phase is detected.

Monoclonal antibody can be selected by publicly known methods or its modified methods. Usually, medium for animal cells supplemented with HAT (hypoxanthine, aminopterin, thymidine) can be used. Any medium in which hybridoma can grow is used for selection and clonal growth. For example, RPMI 1640 medium containing 1 - 20%, preferably 10 - 20% fetal calf serum, GIT medium (Wako Pure Cheimcal Industries, Ltd.) containing 1 - 10% fetal calf serum, serum-free medium for hybridoma culture (SFM-101, Nissui Pharmaceutical Co., Ltd.) and the like can be used. The temperature for culture is usually 20 - 40°C, preferably about 37°C. The duration of culture is usually 5 days to 3 weeks, preferably 1 - 2 weeks. Usually, the cells can be cultured under 5% $CO_2$ gas. The antibody titer in hybridoma culture supernatant can be measured by the same procedure as that for antibody titer in antiserum described above.

(b) Purification of monoclonal antibody

The monoclonal antibodies can be separated and purified by the publicy known methods, for example, the methods for purification of the immunoglobulins [e.g. salting out, alcohol precipitation, isoelectric precipitation, electrophoresis, adsorption-desorption method using ion exchangers (e.g. DEAE), ultracentrifugation, gel filtration, specific purification methods in which only antibody is collected using an active adsorbent such as antigen-bound solid phase, protein A, and protein G, and the antibody is obtained by dissociating the binding].

[Production of polyclonal antibodies]

The polyclonal antibodies of this invention can be manufactured by publicly known methods or its modified methods. For example, a conjugate of an immunogen (proteinous antigen) and a carrier protein is prepared, and warm-blooded animals are immunized by the same method as described for production of monoclonal antibody, and the material containing the polyclonal antibodies of this invention against the protein are collected from said immunized animals, and the antibodies are purified.

Regarding the complex of immnogen and carrier protein for immunizing warm-blooded animals, any kind of carrier protein can be used at any conjugation ratio of the carrier to the hapten if the antibody is efficiently produced against the hapten crosslinked to the carrier for immunization. For example, bovine serum albumin, bovine thyroglobulin, keyhole limpet hemocyanin or the like is coupled to hapten at a weight ratio of carrier to hapten of about 0.1 - 20, preferably about 1 - 5.

Various condensation agents can be used for coupling of carrier to hapten. Active ester reagents containing glutaraldehyde, carbodiimide, maleimide active ester, thiol group, dithiopyridyl group or the like are used.

The condensation product is administered with or without carrier and diluent to the site at which antibody can be produced in warm-blooded animals. To increase the productivity of antibodies, Freund's complete or incomplete adjuvant may be administered when the condensation product is administered. Usually, the condensation product may be administered every once per about 2 - 6 weeks, about 3 - 10 times in total.

The polyclonal antibody can be collected from the blood, ascites and the like, preferably from the blood of warm-blooded animals immunized by the method described above.

The polyclonal antibody titer in antiseurm can be measured by the same procedure as that for the serum antibody titer described above. The antibody can be separated and purified according to the same purification method for immunoglobulin as that of the monoclonal antibody described above.

As antisense DNA having a base sequence complementary to DNA or mRNA encoding the protein or partial peptide of this invention, any antisense DNA can be used as long as it is an oligonucleotide or a derivative thereof having a base sequence complementary to a base sequence or a partial base sequence of a DNA or mRNA encoding the protein or partial peptide of this invention and having an action to suppress expression of the protein or the partial peptide.

By the complementary base sequence is meant, for example, a base sequence having a homology of not less than about 40%, preferably not less than about 60%, more preferably not less than about 80%, more preferably not less than about 90% with a total base sequence or partial base sequence of a DNA or mRNA encoding the protein or the partial peptide of this invention, and the like. Particularly, of the total base sequence of DNA or mRNA of this invention, antisense DNA having a homology of not less than about 40%, preferably not less than about 60%, more preferably not less than about 80%, more preferably not less than about 90% with the base sequence of the portion encoding the N-terminal of the protein of this invention (e.g., base sequence near initiation codon and the like) is preferable. Such antisense DNA can be produced by using a known DNA synthesis apparatus and the like.

The protein of this invention, a partial peptide thereof or a salt thereof has actions such as phosphaturic activity, hypophosphatemia-inducing activity, $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity in kidney cell, and the like. Therefore, the protein of this invention, a partial peptide thereof or a salt thereof can be used for various uses.

In the following, use of the protein of this invention, a partial peptide thereof or a salt thereof (hereinafter sometimes to be briefly referred to as the protein of this invention), a DNA encoding the protein of this invention (hereinafter to be briefly referred to as DNA of the present invention), antibody against the protein of this invention (hereinafter to be briefly referred to as antibody of this invention) and antisense DNA is explained.

(1) Pharmaceutical agents such as an agent for treatment or prophylaxis of various diseases

The protein of this invention and DNA of this invention are useful as a pharmaceutical agent such as an agent for the prophylaxis or treatment of hyperphosphatemia, arteriosclerosis, acute coronary syndrome, heart failure, stroke, chronic glomerulonephritis, diabetic nephropathy, kidney failure and the like.

When the protein of this invention or the DNA of this invention is used as the above-mentioned pharmaceutical agent, it can be used orally as, for example, tablet coated with sugar as necessary, capsule, elixir, microcapsule and the like, or non-orally in the form of injection with water or other pharmacologically acceptable liquid such as asceptic solution and suspension. For example, they can be produced by admixing the protein or DNA of this invention with a physiologically admissible carrier, flavor, excipient, vehicle, preservative, stabilizer, binder and the like in the unit dose form required for generally admitted practice of preparation. The amount of active ingredient in these preparations affords a suitable dose within the indicated range. When the DNA of this invention is used, it can be administered according to a conventional method as the DNA alone or after insertion into a suitable vector such as retrovirus vector, adenovirus vector, adenovirus associated viral vector and the like.

For the additive that can be mixed in tablets, capsules and the like, for example, binders such as gelatin, cornstarch, tragacanth, and gum arabic, excipients such as crystalline cellulose, imbibers such as cornstarch, gelatin, alginic acid and the like, lubricants such as magnesium stearate, sweeteners such as sucrose, lactose and saccharin, and flavors such as peppermint, akamono oil and cherry are used. When the unit-dosage form is capsule, liquid carrier such as oils and fat can be contained. Aseptic compositions for injection can be formulated according to the usual preparation procedure such as dissolving or suspending the active substance in vehicle, e.g. water for injection, natural plant oils e.g. sesame oil and coconut oil and the like. For the aqueous solution for injection, for example, physiological saline and isotonic solutions containing glucose and other supplement (e.g. D-sorbitol, D-mannitol, sodium hydrochloride and the like)are used. Appropriate dissolution-assisting agents, for example, alcohol (e.g. ethanol and the like), polyalcohol (e.g. propylene glycol, polyethylene glycol and the like), nonionic surfactant (e.g. polysorbate 80™, HCO-50 and the like) and the like may be used in combination. For the oily solution, for example, sesame oil, soybean oil and the like are used, and dissolution-assisting agents such as benzyl benzoate and benzyl alcohol may be used in combination. It may be combined with, for example, buffers (e.g. phosphate buffer, sodium acetate buffer, and the like), analgesics (e.g. benzalkonium chloride, procaine hydrochloride and the like), stabilizers (e.g. human serum albumin, polyethylene glycol and the like), preservatives (e.g. benzyl alcohol, phenol and the like), antioxidants and the like. The preparated solution for injection is usually filled in appropriate ampules.

Since the preparations obtained as described above are safe and low toxic, they can be administered to, for example, humans and warm-blooded animals (e.g. rats, mice, guinea pigs, rabbits, birds, sheeps, pigs, bovines, cats, dogs, monkeys).

The dosage of said protein or DNA differs depending on the symptoms and the like. When it is administered orally, in general, for adult patients (60 kg body weight), for example, about 0.1 mg - 100 mg per day, preferably about 1.0 mg - 50 mg per day, more preferably about 1.0 mg - 20 mg per day is administered. When it is administered non-orally, the dosage per dosing differs depending on the target individual, target organ, symptom, administration method and the like. For example, in case of injection, to adult patients (60 kg body weight), for example, it is desirable to intravenously inject about 0.01 - 30 mg per day, preferably about 0.1 - 20 mg per day, more preferably about 0.1 - 10 mg per day. The protein can be administered to other animals, too, in a dose corresponding to the dosage converted for use for 60 kg.

(2) Gene diagnostic agent

Since, using the DNA of this invention as a probe, abnormalities (gene aberration) in the DNA encoding the protein or its partial peptides of this invention can be detected in humans and warm-blooded animals (e.g., rats, mice, guinea pigs, rabbits, sheep, pigs, bovines, horses, cats, dogs, monkeys and the like), the DNA is useful as gene diagnostic agents for diseases in which the protein of this invention is involved.

For example, when damage or deficiency of a DNA or mRNA encoding the protein of this invention or a partial peptide thereof, or decrease in the expression of the protein is detected, for example, the disease may be diagnosed as hyperphosphatemia, arteriosclerosis, acute coronary syndrome, heart failure, stroke, chronic glomerulonephritis, diabetic nephropathy, kidney failure and the like.

On the other hand, when increase of DNA or mRNA encoding the protein of this invention or a partial peptide thereof, or increase in expression of the protein is detected, for example, the disease may be diagnosed as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure and the like.

The gene diagnosis using the DNA of this invention described above can be performed by currently known methods, such as, northern hybridization and PCR-SSCP (Genomics Vol. 5, 874-879 (1989); Proceedings of the National Academy of Sciences of the United States of America Vol. 86, 2766-2770 (1989)) or the like.

(3) Quantification of the protein, its partial peptides, and their salts of this invention

Since the antibodies of this invention specifically recognize the protein of this invention, the antibodies can be used to quantify the protein of this invention in test solutions, especially for a quantification of said protein by the sandwich immunoassay.

This invention provides, for example, the following quantification methods:

(i) Method for quantifying the protein of this invention in test solutions in which antibody of this invention, test solution, and labeled protein of this invention are competitively reacted, and the ratio of the labeled protein of this invention bound to the antibody is measured.

(ii) Method for quantifying the protein of this invention in test solutions in which test solution, antibody of this invention immobilized on carrier, and labeled antibody of this invention are simultaneously or sequentially reacted, and the activity of the label on the immobilized carrier is measured.

In (ii), it is preferable that one antibody recognizes the N-terminal region of the protein of this invention, and the other antibody reacts with the C-terminal region of the protein of this invention.

Using monoclonal antibody against the protein of this invention (hereinafter sometimes to be briefly referred to as monoclonal antibody), quantification of the protein of this invention, and also detection of said protein by tissue staining or the like can be performed. For these purposes, whole antibody molecules may be used, and $F(ab')_2$, Fab', and Fab fractions of the antibody molecule may also be used.

Quantification methods using antibodies against the protein of this invention are not restricted. Any measurement method can be used in which the amount of antibody, antigen, or antibody-antigen complex corresponding to the amount of antigen (e.g. the amount of the protein) in the test solution is detected by chemical or physical technique and the antigen amount is calculated from a standard curve prepared from standard solutions containing known amount of the antigen. For example, nephrometry, competitive method, immunometric method, and sandwich method are appropriately used, and the sandwich method described below is most preferable in regard to sensitivity and specificity.

For the labeling agent for the measurement methods using labeled substances, for example, radioisotopes, enzymes, fluorescent substances, and luminescent substances are used. For the radioisotope, for example, [$^{125}$I], [$^{131}$I], [$^{3}$H], [$^{14}$C] and the like are prefered. As the enzyme described above, stable enzymes with high specific activity are preferred, for example, β-galactosidase, β-glucosidase, alkaline phsophatase, peroxidase, malate dehydrogenase and the like are used. For the fluorescent substance, for example, fluorescamine, fluorescein isothiocyanate and the like are used. For the luminescent substance, for example, luminol, luminol derivatives, luciferin, lucigenin and the like are used. The biotin-avidin system may be used for the binding of labeling agents to antibody or antigen.

For immobilization of antigen or antibody, physical adsorption may be used, and chemical coupling methods usually used for immobilization or fixing proteins, enzymes and the like may also be used. As the carrier, for example, insoluble polysaccharides such as agarose, dextran, cellulose, synthetic resin such as polystyrene, polyacrylamide and silicon, glass, and the like are used.

In the sandwich method, immobilized monoclonal antibody is reacted with test solution (primary reaction), then, with labeled monoclonal antibody (secondary reaction), and the amount of the protein of this invention in the test solution can be quantified by measuring the activity of the label on the carrier. The order of the primary and secondary reactions may be reversed, and the reactions may also be performed simultaneously or sequentially. The labeling agents and the methods for immobilization can follow those described above. In the immunoassay by the sandwich method, the antibody used for immobilized and labeled antibodies is not necessarily one species, and a mixture of two or more species of antibody may be used to increase the measurement sensitivity.

In the methods for measuring the protein of this invention by the sandwich method of this invention, for the monoclonal antibodies of this invention used in the primary and secondary reactions, antibodies that bind to different sites of the protein and the like are preferred. For the antibodies for the primary and secondary reactions are, for example, when antibody used in the secondary reaction recognizes the C-terminal region of the protein of this invention, it is preferable to use antibody recognizing the region other than the C-terminal region for the primary reaction, for example, antibody recognizing the N-terminal region.

Monoclonal antibodies of this invention can be used for measurement systems other than the sandwich method, for example, competitive method, immunometric method, nephrometry and the like. In the competitive method, antigen in test solution and the labeled antigen are competitively reacted with antibody, and the non-reacted labeled antigen (F) and the labeled antigen bound to the antibody (B) are separated (B/F separation). The amount of the label in B or F is measured, and the amount of the antigen in the test solution is quantified.

For the reaction method, a liquid phase method using a soluble antibody, polyethylene glycol for B/F separation, and the secondary antibody against the soluble antibody, or an immobilized method using immobilized antibody as the primary antibody or soluble antibody as the primary antibody and an immobilized antibody as the secondary antibody is used.

In the immunometric method, antigen in test solution and immobilized antigen are competitively reacted with a specified amount of labeled antibody, then the solid phase and liquid phase are separated, or antigen in test solution and an excess amount of labeled antibody are reacted, then immobilized antigen is added to bind to the non-reacted labeled antibody to the solid phase, and the solid phase and liquid phases are separated. Then, the amount of the label in either phase is measured to quantify the antigen in the test solution.

In the nephrometry, insoluble precipitate produced after antigen-antibody reaction in gel or in solution is quantified. When the amount of antigen in the test solution is small and only a small amount of precipitate is obtained, for example, laser nephrometry using scattering of laser is appropriately used.

For applying these immunological methods to the quantification methods for this invention, no specific conditions, procedures or the like are necessary. Systems for measuring the protein of this invention are constructed by adding the usual technical consideration in the art to the conventional conditions and procedures. The details of these general technical means can be referred to reviews and texts.

For example, Irie, H. ed. 'Radioimmunoassay' (Kodansha, 1974), Irie, H. ed. 'Sequel to the Radioimmunoassay' (Kodansha, 1979), Ishikawa, E. et al. ed. 'Immunoenzyme assay' (Igakushoin, 1978), Ishikawa, E. et al. ed. 'Immunoenzyme assay' (2nd ed.) (Igakushoin, 1982), Ishikawa, E. et al. ed. 'Immunoenzyme assay' (3rd ed.) (Igakushoin, 1987), Methods in ENZYMOLOGY Vol. 70 (Immunochemical Techniques (Part A)), Vol. 73 (Immunochemical Techniques (Part B)), Vol. 74 (Immunochemical Techniques (Part C)), Vol. 84 (Immunochemical Techniques (Part D: Selected Immunoassays)), Vol. 92 (Immunochemical Techniques (Part E: Monoclonal Antibodies and General Immunoassay Methods)), Vol. 121 (Immunochemical Techniques (Part I: Hybridoma Technology and Monoclonal Antibodies)) (Academic Press Publishing) and the like can be referred.

By using the antibodies of this invention as mentioned above, the protein and the like of this invention can be quantified with high sensitivity.

Moreover, by quantitative determination of the concentration of the protein of this invention using the antibody of this invention, various diseases involving the protein of this invention can be diagnosed.

For example, when the concentration of the protein of this invention shows a decrease, the disease may be diagnosed as hyperphosphatemia, arteriosclerosis, acute coronary syndrome, heart failure, stroke, chronic glomerulonephritis, diabetic nephropathy, kidney failure and the like.

In contrast, when the concentration of the protein of this invention shows an increase, the disease may be diagnosed as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure and the like.

In addition, among the antibodies of this invention, the antibody capable of neutralizing the activity of the protein of this invention can be used as a pharmaceutical agent such as an agent for the prophylaxis or treatment of a disease such as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure and the like, and the like.

Furthermore, the antibody of this invention can be used to detect the protein of this invention and the like present in a test sample such as body fluid, tissue and the like. In addition, it can be used for the preparation of an antibody column to be used for purification of the protein of this invention and the like, and detection of the protein of this invention and the like in each fraction during the purification.

(4) Screening of candidate compound for pharmaceutical agent

(A) Screening method for receptor agonist or antagonist

The protein of this invention can specifically bind to a phosphatonin receptor (hereinafter to be briefly referred to as receptor) present on a renal tubular cell. Therefore, by constructing a ligand·receptor binding assay system using the protein of this invention and said receptor, screening of a candidate compound for pharmaceutical agent, which has a similar action with the protein of this invention and screening of a candidate compound for pharmaceutical agent, which inhibits the action of the protein of this invention, can be performed. Thus, this invention provides a screening method for a receptor agonist or antagonist using the protein of this invention.

More specifically, this invention provides

(1) a screening method for a receptor agonist or antagonist, which comprises comparing between (i) a case where the protein of this invention is brought into contact with the receptor or a partial peptide thereof, and (ii) a case where the protein of this invention and the like and a test compound are brought into contact with the receptor or a partial peptide thereof, and

(2) a screening method for a receptor agonist or antagonist, which comprises comparing between (i) a case where the protein of this invention and the like is brought into contact with a cell containing a receptor or cell membrane fraction thereof, and (ii) a case where the protein of this invention and a test compound are brought into contact with a cell containing a receptor or cell membrane fraction thereof.

To be specific, the screening method of this invention comprises measurement and comparison of the amount of the protein of this invention which binds to a receptor or a cell containing a receptor and the like, and the cell stimulating activity via said receptor in a cell containing the receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hydroxy vitamin $D_3$-$1_\alpha$-hydroxylase activity, 25-hydroxy vitamin $D_3$-24-hydroxylase activity and the like, between the cases of (i) and (ii).
More specifically, this invention provides

(1a) a screening method for a receptor agonist or antagonist, which comprises measuring and comparing the amount of the labeled protein of this invention bound to a receptor or a partial peptide thereof and their salts, between (i) a case where the labeled protein of this invention is brought into contact with said receptor or a partial peptide thereof, and (ii) a case where the labeled protein of this invention and a test compound are brought into contact with said receptor or a partial peptide thereof, and
(2a) a screening method for a receptor agonist or antagonist, which comprises measuring and comparing the amount of the labeled protein of this invention bound to a cell containing a receptor or the cell membrane fraction thereof, between (i) a case where the labeled protein of this protein is brought into contact with the cell containing the receptor or the cell membrane fraction thereof, and (ii) a case where the labeled protein of this invention and a test compound are brought into contact with the cell containing the receptor or the cell membrane fraction thereof, and
(2b) a screening method for a receptor agonist or antagonist, which comprises measuring and comparing a cell stimulating activity via the receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hydroxy vitamin $D_3$-$1_\alpha$-hydroxylase activity, 25-hydroxy vitamin $D_3$-24-hydroxylase activity and the like, between (i) a case where the protein of this invention is brought into contact with the cell containing said receptor, and (ii) a case where the protein of this invention and a test compound are brought into contact with the cell containing said receptor.

In the screening methods of the above-mentioned (1a) and (2a), a compound that binds to a receptor and inhibits the binding of the protein of this invention to the receptor can be selected as a receptor agonist or antagonist.
In the screening method of the above-mentioned (2b), a compound that binds to a receptor and has a cell stimulating activity via a receptor in a cell containing said receptor (e.g., activity to promote release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity and the like can be selected as a receptor agonist. On the other hand, a compound having an action such as an activity to suppress said cell stimulating activity, $Na^+$-Pi transport-promoting activity, 25-hydroxy vitamin $D_3$-$1_\alpha$-hydroxylase-promoting activity, 25-hydroxy vitamin $D_3$-24-hydroxylase inhibitory activity and the like can be selected as a receptor antagonist.
Furthermore, in the screening method of the above-mentioned (1a) or (2a), among the test compounds in which an inhibitory activity on the binding of the protein of this invention to a receptor were approved, a compound having an activity such as phosphaturic activity, hypophosphatemia-inducing activity, cell stimulating activity via a receptor in a cell containing said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity and the like in a kidney cell, can be selected as a receptor agonist and a compound without such activity can be selected as a receptor antagonist.
As a receptor to be used for the screening method of this invention, a phosphatonin receptor and the like, which is expressed in a renal proximal tubule cell of human or warm-blooded animals can be used.
These receptors and a receptor for the protein of this invention can be obtained according to a publicly known purification method for proteins. Alternatively, an objective receptor can be also obtained by cloning a DNA encoding said receptor according to a publicly known genetic engineering technique, and then by the aforementioned expression method of the protein of this invention.
As a partial peptide of said receptor, a partial peptide obtained by appropriately cleaving the full-length receptor can be used.
As the labeled protein of this invention, for example, the protein of this invention labeled with [$^3$H], [$^{125}$I], [$^{14}$C],

[$^{35}S$] and the like, and the like can be used.

As a cell containing the above-mentioned receptor, which is used for the screening method of this invention, those similar to the ones recited as the aforementioned host cell to be used for the expression of protein of this invention can be used, with preference given to CHO cells and the like. The cell containing a receptor can be produced using a DNA encoding the receptor according to publicly known method(s), such as the aforementioned method for expressing the protein of this invention, and the like. As a cell containing the above-mentioned receptor, a cell line such as CL8 cell (BONE, 18, 159-169, 1996), OK cell (AMERICAN JOURNAL OF PHYSIOLOGY, 253, E221-E227, 1987) and the like can be also used.

When a cell containing a receptor is used for the screening method of this invention, said cell can be immobilized with glutaraldehyde, formalin and the like. The immobilization can be done by publicly known method(s).

Cell membrane fraction is a fraction abundant in cell membranes obtained by publicly known methods after disruption of the cells. The cell disruption methods include crush of the cells with a Potter-Elvehjem homogenizer, crush using a Waring blender or polytron (Kinematica Co.), disruption by ultrasonication, and disruption by passing the cells through a narrow nozzle with compressing the cells using a French Press. For the cell membrane fractionation, fractionation based on centrifugal force such as a centrifugation for fractionation and a density gradient centrifugation are mainly used. For example, disrupted cell suspension is centrifuged at a low speed (500 - 3,000 rpm) for a short time (usually about 1 - 10 minutes), then the supernatant is centrifuged at a high speed (15,000 - 30,000 rpm) for usually 30 minutes - 2 hours, and the obtained precipitate is used as the membrane fraction. The membrane fraction rich in membrane components such as the expressed receptor and the protein of this invention, and phospholipids and membrane proteins derived from the cells.

For the amount of the receptor expressed on the cells containing the receptor or the membrane fraction thereof, $10^3$ - $10^8$ molecules per cell is preferred, and $10^5$ - $10^7$ molecules per cell is appropriate. As the expression level increases, the ligand-binding activity (specific activity) of the membrane fraction increases, which allows not only construction of a highly sensitive screening system but also measurement of a large number of samples using the same lot.

For the test compounds, for example, proteins, non-proteinous compounds, synthetic compounds, fermentation products, cell extracts, plant extracts, and animal tissue extracts, and the like are used, and these compounds may be novel or publicly known compounds.

In the screening method of this invention, the reaction between the protein of this invention and a receptor can be performed generally in about 37°C for several hours.

Specifically, in order to perform the screening method of the above-mentioned (1a) or (2a), at first, a cell containing a receptor or a cell membrane fraction thereof, a receptor or a partial peptide thereof of this invention is suspended in a buffer suitable for screening to give a receptor preparation. For the buffer, any buffer that does not inhibit the binding of the protein of this invention to the receptor, such as phosphate buffer and Tris-hydrochloride buffer (pH ca. 4 - 10, preferably pH ca. 6 - 8) can be used. To reduce non-specific binding, a surfactant such as CHAPS, Tween-80™ (Kao-Atras Co.), digitonin, and deoxycholate may be added to the buffer. Furthermore, in order to suppress degradation of the receptor and its ligands by proteases, protease inhibitors such as PMSF, leupeptin, bacitracin, aprotinin, E-64 (Institute for Protein Research), and pepstatin and the like may be added. On the other hand, when the cell is an adhesive cell, the protein of this invention and the like can be bound to the receptor by using a cell adhered on a culture vessel, that is the condition the cell is alive, or by using a cell fixed on the culture vessel with glutaraldehyde or paraformaldehyde.

In these cases, medium, Hanks' solution and the like are used as said buffer. A specified amount (e.g., about 10,000 - 1,000,000 cpm in the case of 2000Ci/mmol) of labeled protein of this invention and the like (e.g., [$^{125}I$] labeled protein of this invention) is added to 0.01 - 10 ml of the receptor solution, with simultaneous addition of $10^{-4}$ M - $10^{-10}$ M test compound. To examine the non-specific binding (NSB), reaction tubes containing a highly excess amount of the non-labeled protein of this invention are also prepared. The reaction is performed at 0°C - 50°C, preferably 4°C - 37°C, for 20 minutes - 24 hours, preferably for 30 minutes - 3 hours. After the reaction, the reaction mixture is filtrated through a glass fiber filter and the filter was washed with an appropriate volume of the same buffer. The radioactivity (e.g., amount of [$^{125}I$]) remaining on the glass fiber filter is measured by using a liquid scintillation counter or γ-counter. For filtration, Manifold and cell harvester can be used, wherein use of a cell harvester is desirable to achieve higher efficiency. Regarding the count obtained by subtracting the amount of non-specific binding (NSB) from the count obtained in the absence of competitive substance ($B_0$) as 100%, when the amount of specific binding (B-NSB) is, for example, 50% or less of the count ($B_0$-NSB), the test compound can be selected as a candidate compound for agonist or antagonist.

Furthermore, to perform the screening method of the above-mentioned (2b), the cell stimulating activity via a receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hy-

droxy vitamin $D_3$-$1_\alpha$-hydroxylase activity, 25-hydroxy vitamin $D_3$-24-hydroxylase activity and the like) can be measured according to a publicly known method or its modified method.

Specifically, first, cells containing the receptor are cultured in multiwell plates and the like. Before screening, the culture medium is exchanged to a fresh medium or appropriate buffer that exhibits no toxicity for the cells, and then the test compounds and the like are added. After the cells are incubated with test compound for a specified time, the cells are extracted or the supernatant is collected, and the product is quantified according to the corresponding method. When it is difficult to examine the production of indicator substance for the cell-stimulating activity (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) due to degrading enzymes contained in the cells, inhibitors of the degrading enzymes may be added to the assay system. The inhibitory activity on cAMP production and the like can be detected as the inhibitory effect on the cells whose baseline production amount has been increased with forskolin and the like.

The measurement of the $Na^+$-Pi transport activity can be performed according to, for example, a method of Cole J.A. *et al.* (AMERICAN JOURNAL OF PHYSIOLOGY, 253, E221-E227, 1987).

The 25-hydroxy vitamin $D_3$-$1_\alpha$-hydroxylase activity and 25-hydroxy vitamin $D_3$-24-hydroxylase activity can be measured according to, for example, a method such as Miyauchi A. and the like, (JOURNAL OF CLINICAL ENDOCRINOLOGY AND METABOLISM, 67, 46-53, 1988).

As a cell to be used for these measurements, for example, an established cell line such as the aforementioned CL8 cell line and OK cell line and the like.

In the screening method of the above-mentioned (2b), when the cell containing a receptor shows enhanced cell stimulating activity via said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), suppressed $Na^+$-Pi transport activity, suppressed 25-hydroxy vitamin $D_3$-$1_\alpha$-hydroxylase activity, enhanced 25-hydroxy vitamin $D_3$-24-hydroxylase activity and the like, upon addition of a test compound, the test compound can be selected as a candidate compound for a receptor agonist. In addition, when the cell containing a receptor shows suppressed cell stimulating activity via said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), enhanced $Na^+$-Pi transport activity, enhanced 25-hydroxy vitamin $D_3$-$1_\alpha$-hydroxylase activity, suppressed 25-hydroxy vitamin $D_3$-24-hydroxylase activity, and the like, upon addition of a test compound, the test compound can be selected as a candidate compound for a receptor antagonist.

The screening kit of this invention contains the protein of this invention, and preferably further contains a cell containing a receptor or a cell membrane fraction thereof, and the like.

Examples of the screening kit of this invention include the following.

[Reagents for screening]

(1) Buffer solution for measurement and washing

Hanks' balanced salt solution (Gibco Co.) supplemented with 0.05% bovine serum albumin (Sigma Co.). The solution is sterilized by filtration through a 0.45 μm (pore size) filter, and stored at 4°C or may be prepared at use.

(2) Receptor preparation

CHO cells containing the receptor to the protein of this invention and the like which were seeded in 12-well plates at a density of $5 \times 10^5$ cells/well and cultured at 37°C under 5% $CO_2$ and 95% air for two days.

(3) Preparation of the labeled protein of this invention

The protein of this invention, a partial peptide thereof or a salt thereof labeled with [$^3$H], [$^{125}$I], [$^{14}$C], [$^{35}$S] and the like.

(4) The standard solution of the protein of this invention

The protein of this invention, a partial peptide thereof or a salt thereof which were dissolved in and adjusted to 0.1 mM with PBS containing 0.1% bovine serum albumin (Sigma Co.) and stored at -20°C.

[Measurement method]

(i) CHO cells containing a recombinant receptor are cultured in 12-well culture plates and washed twice with 1 ml of the measurement buffer, and 490 μl of the measurement buffer is added to each well.
(ii) After adding 5 μl of $10^{-3}$ - $10^{-10}$ M test compound solution, 5 μl of labeled protein of this invention (5 nM) is added, and the cells are incubated at room temperature for one hour. In order to estimate the non-specific binding, 5 μl of the protein of this invention ($10^{-4}$ M) is added in place of the test compound.
(iii) The reaction solution is removed, and the wells are washed three times with 1 ml of the washing buffer. The labeled protein of this invention bound to the cells is dissolved with 0.5 ml of 0.2N NaOH-1% SDS, and mixed with 4 ml of liquid scintillator A (Wako Pure Chemical Industries, Ltd.)
(iv) The radioactivity is measured using a liquid scintillation counter (Beckman Co.), and the percent maximum binding (PMB) is calculated by the equation [Equation 1] below. When labeled with [$^{125}$I], it can be directly measured on a gamma counter, without mixing with a liquid scintillator.

[Equation 1]

$$PMB = [(B - NSB)/(B_0 - NSB)] \times 100$$

PMB : Percent maximum binding
B : Binding amount obtained in the presence of test compound
NSB : Non-specific binding
$B_0$ : Maximum binding

As mentioned above, the protein of this invention is useful as a reagent for screening a receptor agonist or antagonist.
The compound or a salt thereof obtained using the screening method or screening kit of this invention is the compound which inhibits binding the protein of this invention to its receptor, specifically, the compound or a salt thereof (so-called, receptor agonist) having an action such as the cell stimulating activity via said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity and the like, or the compound , or a salt thereof (so-called, receptor antagonist) without an action such as the cell stimulating activity via a receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity and the like.
Because a receptor agonist has the entire or partial physiological activity that the protein of this invention has, it is useful as a safe and low toxic pharmaceutical agent depending on said physiological activity. For example, it is useful for pharmaceutical agent such as an agent for the prophylaxis or treatment and the like of a disease (e.g., hyperphosphatemia, arteriosclerosis, acute coronary syndrome, heart failure, stroke, chronic glomerulonephritis, diabetic nephropathy, kidney failure and the like).
In contrast, a receptor antagonist can suppress the entire or partial physiological activity that the protein of this invention has. Therefore, it is useful as a safe and low toxic pharmaceutical agent that suppresses said physiological activity. For example, it is useful for a pharmaceutical agent such as an agent for the prophylaxis or treatment and the like of a disease (oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure and the like).

(B) Screening method or a screening kit for an inhibitor of a proteinase that degrades the protein of this invention

The protein of this invention or a salt thereof is considered to be inactivated upon cleavage by a proteinase present in biological organisms. Therefore, the use of the protein of this invention and proteinase that degrades the protein of this invention enables selection of a compound having an inhibitory activity on proteinase that degrades the protein of this invention.

A compound having an inhibitory activity on said proteinase can promote the activity of the protein of this invention, which is independent of contact between cells, by preventing inactivation of the protein of this invention in biological organisms. Thus, it is expected to be a pharmaceutical agent such as an agent for the prophylaxis or treatment of a disease such as hyperphosphatemia, arteriosclerosis, acute coronary syndrome, heart failure, stroke, chronic glomerulonephritis, diabetic nephropathy, kidney failure and the like.

Thus, this invention provides a screening method of a compound or a salt thereof having an inhibitory activity on proteinase that degrades the protein of this invention, which comprises using the protein of this invention.

More specifically, this invention provides

(1) a screening method of a compound or a salt thereof having an inhibitory activity on a proteinase that degrades the protein of this invention, which comprises comparing (i) a case where a proteinase that degrades the protein of this invention and the protein of this invention are incubated and brought into contact with a cell containing a receptor, with (ii) a case where a proteinase that degrades the protein of this invention, a test compound and the protein of this invention are incubated and brought into contact with a cell containing a receptor.

Specifically, the screening method of this invention comprises measuring and comparing activities such as the cell stimulating activity via a receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hydroxy vitamin $D_3$-$1_\alpha$-hydroxylase activity, 25-hydroxy vitamin $D_3$-24-hydroxylase activity and the like between the cases of (i) and (ii).

More specifically, this invention provides

(1a) a screening method of a compound or a salt thereof having an inhibitory activity on a proteinase that degrades the protein of this invention, which comprises measuring and comparing activities such as a cell stimulating activity via a receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hydroxy vitamin $D_3$-$1_\alpha$-hydroxylase activity, 25-hydroxy vitamin $D_3$-24-hydroxylase activity and the like, between (i) a case where a proteinase that degrades the protein of this invention and the protein of this invention are incubated and brought into contact with a cell containing a receptor, and (ii) a case where a proteinase that degrades the protein of this invention a test compound and the protein of this invention are incubated and brought into contact with a cell containing a receptor.

In the above-mentioned screening method, a test compound that promotes activities such as a cell stimulating activity via said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity and the like, can be selected as a compound or a salt thereof having an inhibitory activity on proteinase that degrades the protein of this invention.

As a receptor to be used for the screening method of this invention, a phosphatonin receptor that expresses in a renal proximal tubule cell of human or warm-blooded animal and the like can be used.

The receptor to the protein of this invention can be obtained according to a publicly known purification method for proteins. Alternatively, an objective receptor can be also obtained by cloning a DNA encoding said receptor according to a publicly known genetic engineering technique, and then by the aforementioned expression method of the protein of this invention.

As a proteinase that degrades the protein of this invention, for example, zinc metallopeptidase, PHEX gene product and the like are used.

As a cell containing the above-mentioned receptor to be used for the screening method of this invention, those similar to the ones recited above as a host cell to be used for the expression of the protein of this invention can be used, with preference given to CHO cell and the like. Of those recited, CHO cell and the like are preferable. The cell containing a receptor can be produced using a DNA encoding the receptor according to publicly known method(s), such as the aforementioned method for expressing the protein of this invention, and the like. As a cell containing the above-mentioned receptor, a cell line such as CL8 cell (BONE, 18, 159-169, 1996), OK cell (AMERICAN JOURNAL OF PHYSIOLOGY, 253, E221-E227, 1987) and the like can be also used.

When a cell containing a receptor is used for the screening method of this invention, said cell can be immobilized with glutaraldehyde, formalin and the like. The immobilization can be done by publicly known method(s).

As a cell membrane fraction of the cell containing the above-mentioned receptor, those similar to the ones recited above can be used.

For the test compound, for example, protein, non-proteinous compound, synthetic compound, fermentation product, cell extract, plant extract, animal tissue extract and the like are used, and these compounds may be novel or publicly known compounds.

In the screening method of this invention, incubation of proteinase and the protein of this invention can be done generally for several hours at about 37°C. The reaction of this reaction mixture with a cell containing a receptor can be performed generally for several hours at about 37°C.

The cell stimulating activity via a receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hydroxy vitamin $D_3$-$1_\alpha$-hydroxylase activity, 25-hydroxy vitamin $D_3$-24-hydroxylase activity and the like can be measured in the same manner as in the aforementioned.

The screening kit of this invention contains the protein of this invention and a proteinase that degrades the protein of this invention, and preferably further contains a cell containing a receptor.

Examples of the screening kit of this invention include the following.

[Reagent for screening]

(1) Buffers for measurement and washing

Hanks' balanced salt solution (Gibco Co.) supplemented with 0.05% bovine serum albumin (Sigma Co.). The solution is sterilized by filtration through a 0.45 μm (pore size) filter, and stored at 4°C or may be prepared at use.

(2) Receptor preparation

CHO cells containing the receptor to the protein of this invention and the like which were seeded in 12-well plates at a density of 5 x $10^5$ cells/well and cultured at 37°C under 5% $CO_2$ and 95% air for two days.

(3) Preparation of the protein of this invention

The protein of this invention, a partial peptide thereof or a salt thereof.

(4) Preparation of proteinase that degrades the protein of this invention

A proteinase that degrades the protein of this invention.

[Measurement method]

(1) A proteinase that degrades the protein of this invention and the protein of this invention are incubated at about 37°C for several hours.
(2) A proteinase that degrades the protein of this invention, a test compound and the protein of this invention are incubated at about 37°C for several hours.
(3) The reaction mixtures obtained in the above-mentioned (1) and (2) are respectively cultured together with a cell containing a receptor to the protein of this invention at about 37°C for several hours.
(4) Then, the cell stimulating activity via said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like), $Na^+$-Pi transport activity, 25-hydroxy vitamin $D_3$-$1_\alpha$-hydroxylase activity, 25-hydroxy vitamin $D_3$-24-hydroxylase activity and the like are measured according to the aforementioned method.

As mentioned above, the protein of this invention is useful as a reagent for screening a compound or a salt thereof having an inhibitory activity on a proteinase that degrades the protein of this invention.

The compound or a salt thereof obtained using the screening method or screening kit of this invention is the compound that inhibits a proteinase that degrades the protein of this invention, and suppresses inactivation of the protein of this invention by said protease. Therefore, said compound can promote activities such as a cell stimulating activity via said receptor (e.g., release of arachidonic acid, release of acetylcholine, change in intracellular $Ca^{2+}$ con-

centration, intracellular production of cAMP, intracellular production of cGMP, production of inositol phosphate, change in cell membrane potential, phosphorylation of intracellular protein, lowering of pH and the like), $Na^+$-Pi transport inhibitory activity, 25-hydroxy vitamin $D_3$-$1_\alpha$-hydroxylase inhibitory activity, 25-hydroxy vitamin $D_3$-24-hydroxylase-promoting activity and the like, which are independent of contact between cells and afforded by the protein of this invention, and the compound is useful as a safe and low toxic pharmaceutical agent for hyperphosphatemia, arteriosclerosis, acute coronary syndrome, heart failure, stroke, chronic glomerulonephritis, diabetic nephropathy, kidney failure and the like.

When a compound obtained using the screening method or screening kit of this invention is used as the aforementioned agent for treatment or prophylaxis, it can be used in the same manner as in the aforementioned receptor agonist/antagonist. (C) Screening method of a compound or a salt thereof that promotes or inhibits intracellular signal transduction after binding of the protein of this invention to its receptor

The protein of this invention can specifically bind to a phosphatonin receptor (hereinafter to be briefly referred to as a receptor) expressed in renal proximal tubule cell of human or warm-blooded animals. Therefore, by constructing a ligand-receptor binding assay system using the protein of this invention and said receptor, screening of a compound, which promotes or inhibits intracellular signal transduction after the binding of the protein of this invention to said receptor, can be performed.

Thus, this invention provides a screening method of a compound or a salt thereof that promotes or inhibits intracellular signal transduction after binding of the protein of this invention to the receptor, which comprises using the protein of this invention.

More specifically, this invention provides

(1) a screening method of a compound or a salt thereof that promotes or inhibits intracellular signal transduction after binding of the protein of this invention to the receptor, which comprises comparing (i) a case where the protein of this invention is brought into contact with a cell containing a receptor, with (ii) a case where the protein of this invention and a test compound are brought into contact with a cell containing the receptor.

Specifically, the screening method of this invention comprises measuring and comparing an intracellular signal transduction after binding of the protein of this invention to the receptor between the cases (i) and (ii).

More specifically, this invention provides

(1a) a screening method of a compound or a salt thereof that promotes or inhibits intracellular signal transduction after binding of the protein of this invention to the receptor, which comprises measuring and comparing the cell stimulating activity via a receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) between (i) a case where the protein of this invention is brought into contact with a cell containing the receptor, and (ii) a case where the protein of this invention and a test compound are brought into contact with a cell containing the receptor.

In the screening method of the above-mentioned (1a), a compound that does not inhibit the binding of the protein of this invention to a receptor but promotes the cell stimulating activity induced by the protein of this invention via said receptor (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) can be selected as a compound or a salt thereof which promotes or inhibits intracellular signal transduction after binding of the protein of this invention to the receptor. In contrast, a compound that does not inhibit the binding of the protein of this invention to a receptor but inhibits the cell stimulating activity induced via a receptor by the protein of this invention (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) can be selected as a compound that inhibits intracellular signal transduction after binding of the protein of this invention to the receptor.

That is, this screening method is for selection of a compound that regulates (promotes or suppresses) intracellular signal transduction after binding of the protein of this invention to the receptor, without an influence on the binding the protein of this invention to the receptor. Therefore, as a test compound to be used for this screening method, a compound that was not selected as a receptor agonist or antagonist in the aforementioned screening method of the receptor agonist/antagonist is desirable.

As the receptor to be used for the screening method of this invention, phosphatonin receptor that expresses in renal proximal tubule cell of human or warm-blooded animal and the like are used.

The receptor to the protein of this invention can be obtained according to a publicly known purification method for proteins. Alternatively, an objective receptor can be also obtained by cloning a DNA encoding said receptor according to a publicly known genetic engineering technique, and then by the aforementioned expression method of the protein of this invention.

As a partial peptide of the receptor, a partial peptide obtained by appropriately cleaving the full-length receptor can be used.

As a cell containing the above-mentioned receptor, which is used for the screening method of this invention, those similar to the ones recited above as the aforementioned host cell to be used for the expression of protein of this invention can be used. Of those recited, CHO cells and the like are preferable. The cell containing a receptor can be produced using a DNA encoding the receptor according to publicly known method(s), such as the aforementioned method for expressing the protein of this invention, and the like. As a cell containing the above-mentioned receptor, a cell line such as CL8 cell (BONE, 18, 159-169, 1996), OK cell (AMERICAN JOURNAL OF PHYSIOLOGY, 253, E221-E227, 1987) and the like can be also used.

When a cell containing a receptor is used for the screening method of this invention, said cell can be immobilized with glutaraldehyde, formalin and the like. The immobilization can be done by publicly known method(s).

As a cell membrane fraction containing the above-mentioned receptor, those similar to the ones recited above can be used.

For the test compounds, for example, proteins, non-proteinous compounds, synthetic compounds, fermentation products, cell extracts, plant extracts, animal tissue extracts and the like are used, and these compounds may be novel or publicly known compounds.

In the screening method of this invention, the reaction between the protein of this invention and a receptor can be performed generally in about 37°C for several hours.

In the screening method of the above-mentioned (1a), the cell stimulating activity (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) induced by the protein of this invention via a receptor, can be measure in the same manner as above.

In the screening method of the above-mentioned (1a), when the cell stimulating activity (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) induced by the protein of this invention via said receptor, is promoted by the addition of a test compound, said test compound can be selected as a compound or a salt thereof that promotes intracellular signal transduction after the binding of the protein of this invention to the receptor. On the other hand, when the cell stimulating activity (e.g., release of arachidonic acid, release of acetylcholine, change in intracellular $Ca^{2+}$ concentration, intracellular production of cAMP, intracellular production of cGMP, production of inositol phosphate, change incell membrane potential, phosphorylation of intracellular protein, lowering of pH and the like) induced by the protein of this invention via a receptor is inhibited by the addition of a test compound, said test compound can be selected as a compound or a salt thereof that promotes intracellular signal transduction after the binding of the protein of this invention to the receptor.

The screening kit of this invention contains the protein of this invention, and preferably further contains a cell containing a receptor.

Examples of the screening kit of this invention include the following.

[Reagent for screening]

(1) Buffers for measurement and washing

Hanks' balanced salt solution (Gibco Co.) supplemented with 0.05% bovine serum albumin (Sigma Co.). The solution is sterilized by filtration through a 0.45 μm (pore size) filter, and stored at 4°C or may be prepared at use.

(2) Receptor preparation

CHO cells containing the receptor to the protein of this invention which are seeded in 12-well plates at a density of 5 x $10^5$ cells/well and cultured at 37°C under 5% $CO_2$ and 95% air for two days.

(3) Preparation of the protein of this invention

The protein of this invention, a partial peptide thereof or a salt thereof.

[Measurement method]

The cell stimulating activity (e.g., release of arachidonic acid, release of acetylcholine, change in intracellular $Ca^{2+}$ concentration, intracellular production of cAMP, intracellular production of cGMP, production of inositol phosphate, change in cell membrane potential, phosphorylation of intracellular protein, lowering of pH and the like) is assayed according to the aforementioned method.

As mentioned above, the protein of this invention is useful as a reagent for screening a compound or a salt thereof that promote or inhibit intracellular signal transduction after binding of the protein to its receptor.

The compound or a salt thereof obtained by the screening method or screening kit of this invention is a compound or a salt thereof that promotes a cell stimulating activity (e.g., release of arachidonic acid, release of acetylcholine, variation of intracellular $Ca^{2+}$ concentration, intracellular production of cAMP, intracellular production of cGMP, production of inositol phosphate, variation of cell membrane potential, phosphorylation of intracellular protein, lowering of pH and the like) induced by the protein of this invention via the receptor after binding of the protein of this invention to the receptor, or that inhibits the cell stimulating activity.

The compound or a salt thereof that promotes the intracellular signal transduction after binding of the protein of this invention to a receptor is useful as a safe and low toxic pharmaceutical agent for the prophylaxis or treatment of, for example, diseases such as hyperphosphatemia, arteriosclerosis, acute coronary syndrome, heart failure, stroke, chronic glomerulonephritis, diabetic nephropathy, kidney failure and the like.

The compound or a salt thereof that inhibits the intracellular signal transduction after binding of the protein of this invention to a receptor is useful as a safe and low toxic pharmaceutical agent for the prophylaxis or treatment of, for example, diseases such as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure and the like.

When a compound obtained using the screening method or screening kit of this invention is used as the aforementioned agent for treatment or prophylaxis, it can be used in the same manner as in the aforementioned receptor agonist/antagonist.

(4) Antisense DNA

An antisense DNA that can complementarily bind with DNA or mRNA encoding the protein of this invention and suppress expression of said DNA or mRNA or the protein of this invention can suppress functions of the protein of this invention or the DNA encoding the same to exhibit the above-mentioned action in biological organisms. Therefore, said antisense DNA can be used as a pharmaceutical agent for the prophylaxis or treatment of, for example, diseases such as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi' s syndrome, renal tubular acidosis, cystic fibrosis, fibrous cystic ostitis, kidney failure and the like.

When the antisense DNA is used as the aforementioned agent for the above-mentioned treatment or prophylaxis, it can be used in the same manner as in the aforementioned agent for treatment·prophylaxis of various diseases, which contains the protein of this invention or DNA.

In addition, said antisense DNA can also be used as a diagnostic oligonucleotide probe to examine the presence and expression levels of DNA of this invention in tissues and cells.

(5) Preparation of a transgenic animal of this invention

Furthermore, the present invention provides a non-human mammal having a DNA encoding a novel protein (phosphatonin) (hereinafter to be briefly referred to as exogenous DNA of this invention) having a phosphaturic activity and/or hypophosphatemia-inducing activity or a mutant DNA thereof (sometimes to be briefly referred to as exogenous mutant DNA of this invention).

That is, the present invention provides

(1) a non-human mammal having an exogenous DNA of this invention or a mutant DNA thereof,
(2) the animal of (1), wherein the non-human mammal is a rodent,
(3) the animal of (2), wherein the rodent is a mouse or rat, and
(4) a recombinant vector containing an exogenous DNA of the present invention or a mutant DNA thereof, which is capable of expression in mammal, and
(5) a pharmaceutical agent for gene therapy, containing the recombinant vector of (4), and the like.

A non-human mammal having an exogenous DNA of the present invention or a mutant DNA thereof (hereinafter to be briefly referred to as a transgenic animal of this invention) can be created by transferring the objective exogenous DNA of this invention into an unfertilized egg, a fertilized egg, sperm, a germinal cell including a progenitor cell thereof and the like, preferably in the stage of embryogenesis in a non-human mammal (more preferably in the stage of single cell or fertilized ovum, and generally before 8 cell stage) according to calcium phosphate method, electric pulse method, lipofection method, aggregation method, microinjection method, particle gun method, DEAE-dextran method or the like. By transferring the objective exogenous DNA of this invention to somatic cell, organs of organisms, tissue cell and the like by said DNA transfer method, they can be utilized for cell culture, tissue culture and the like. Moreover, a transgenic animal of this invention can also be created by fusing these cells with the aforementioned germinal cells according to publicly known cell fusion method.

As the non-human mammal, for example, bovine, pig, sheep, goat, rabbit, dog, cat, guinea pig, hamster, mouse, rat and the like are used. Among them, from the aspects of preparation of pathological animal models, rodent whose developing period and life cycle is comparatively short and that are easily bred, particularly mice (e.g., as pure strain, C57BL/6 strain, DBA2 strain and the like, and as cross strain, B6C3F1 strain, BDF1 strain, B6D2F1 strain, BALB/c strain, ICR strain and the like) or rats (e.g., Wistar, SD and the like) and the like are preferable.

As the "mammal" for the expression of the recombinant vector, human and the like can be mentioned besides the above-mentioned non-human mammals.

The exogenous DNA of this invention is not a DNA of this invention that non-human mammals have, but DNA of the present invention, which is once isolated and extracted from the mammal.

The mutant DNA of this invention is one that shows a variation in the base sequence of DNA of this invention (e.g., mutant and the like), which is specifically a DNA wherein addition, deletion of bases and substitution by different base, and the like occurred, and the like, including abnormal DNA.

As said abnormal DNA, a DNA that expresses abnormal protein of this invention, for example, a DNA that expresses a protein that suppresses normal function of the protein of this invention, and the like are used.

The exogenous DNA of this invention may be derived from a mammal homogeneous or heterogeneous to the target animal. For transfer of the DNA of this invention to a target animal, it is generally beneficial to use a DNA construct wherein the DNA is ligated to the downstream of a promoter that can express said DNA in an animal cell. For example, when human DNA of this invention is transferred, a DNA construct (e.g., vector and the like) wherein the human DNA of this invention is ligated to the downstream of various promoters that can express DNA derived from various mammals (e.g., rabbit, dog, cat, guinea pig, hamster, rat, mouse and the like) possessing the DNA of this invention having high homology therewith, is microinjected into a fertilized egg of the target mammal, for example, mouse fertilized egg, whereby a transgenic mammal that is capable of high expression of the DNA of this invention can be created.

As the expression vector of the protein of this invention, plasmids derived from *Escherichia coli,* plasmids derived from *Bacillus subtilis*, plasmids derived from yeast, bacteriophages such as λ phage, retroviruses such as Moloney leukemia virus, animal viruses such as vaccinia virus and baculoviruses, and the like are preferably used. Among them, plasmids derived from *Escherichia coli,* plasmids derived from *Bacillus subtilis* and plasmids derived from yeast and the like are preferably used.

As the promoter to regulate the expression of the above-mentioned DNA, for example, promoters of DNA derived from virus (e.g., simian virus, cytomegalovirus, Moloney leukemia virus, JC virus, breast cancer virus, poliovirus and the like) and as those derived from various mammals (human, rabbit, dog, cat, guinea pig, hamster, rat, mouse and the like) and avians (chicken and the like), promoters of albumin, insulin II, uroplakin II, elastase, erythropoietin, endothelin, muscle creatine kinase, glial fiber acidic protein, glutation S-transferase, platelet-derived growth factor β, keratin K1, K10 and K14, type I and type II collagen, cyclic AMP-dependent protein kinase βI subunit, dystrophin, tartaric acid-resistant alkaline phosphatase, atrial natriuretic factor, endothelial receptor tyrosine kinase (generally abbreviated as Tie2), sodium potassium adenosine triphosphatase (Na, K-ATPase), neurofilament light chain, metallothionein I and IIA, metalloproteinase 1, tissue inhibitor, MHC class I antigen (H-2L), H-ras, renin, dopamine β-hydroxylase, thyroid peroxidase (TPO), polypeptide chain elongation factor $1_\alpha$ (EF-$1_\alpha$), β-actin, α and β myosin heavy chain, myosin light chain 1 and 2, myelin basic protein, thyloglobulin, Thy-1, immunoglobulin, H-chain variable region (VNP), serum amyloid P component, myoglobin, troponin C, smooth muscle α actin, preproenkephalin A, vasopressin and the like, are used. Preferably, cytomegalovirus promoter, promoter of human polypeptide chain elongation factor $1_\alpha$ (EF-$1_\alpha$), human and chicken β actin promoter and the like, which are capable of systemic high expression of the target DNA, can be used.

The above-mentioned vector preferably contains a sequence (generally called a terminator) that terminates transcription of the objective messenger RNA in a transgenic mammal. For example, virus-derived, various mammals and avians-derived respective DNA sequences can be used, and preferably, SV40 terminator of simian virus and the like are used.

In addition, with the aim of affording still higher expression of the objective DNA, splicing signal, enhancer region, partial intron of eucaryotic DNA and the like of each DNA may be connected to 5' upstream of promoter region, between promoter region and translation region or 3' downstream of translation region, depending on the object.

The translational region of normal protein of this invention can be obtained from a DNA derived from liver, kidney, thyroid cell, and fibroblast of various mammals (e.g., rabbit, dog, cat, guinea pig, hamster, rat, mouse, human and the like) and commercially available various genomic DNA libraries, as the whole or part of the genomic DNA, or by using complementary DNA prepared from RNA derived from liver, kidney, thyroid cell, and fibroblast by a known method, as a starting material. In addition, an exogenous abnormal DNA can be prepared by mutating a translational region of normal protein obtained from the above-mentioned cell or tissue, by a point mutagenesis method.

A DNA construct capable of expression of the protein of this invention in the transgenic animal can be prepared by connecting said translational region to the downstream of the aforementioned promoter and, where desired, to the upstream of the transcription termination site by using general DNA engineering techniques.

The transfer of the DNA of this invention in the stage of fertilized ovum is ensured to be present in every germinal cell and somatic cell of the target mammal. The existence of DNA of this invention in the germinal cell of created animal after DNA transfer means that every germinal cell and every somatic cell in every progeny of the created animal retain the DNA of this invention.

The offspring of this kind of animal that inherited DNA has the DNA of this invention in every germinal cell and every somatic cell.

The non-human mammal, to which the exogenous normal DNA of this invention is transferred, can be bred over generations in typical breeding environment as an animal retaining said DNA, upon confirmation of stable retention of the DNA by mating. The transfer of the DNA of this invention in the stage of fertilized ovum is ensured to be present in excess in every germinal cell and every somatic cell of the target mammal. The existence of DNA of this invention in excess in the germinal cell of created animal after DNA transfer means that every germinal cell and every somatic cell in every progeny of the created animal retain the DNA of this invention in excess. The offspring of this kind of animal that inherited DNA has DNA of this invention in excess in every germinal cell and every somatic cell. By obtaining a homozygote animals having the introduced DNA in both the homologous chromosomes and mating male and female of the animals, every offspring can be bred over generations such that the DNA is retained in excess.

A non-human mammal having the normal DNA of this invention highly expresses the normal DNA of this invention, and by promoting the function of endogeneous normal DNA, the hyperergasia of the protein of this invention may ultimately occur. Thus, it can be used as a pathological model animal. For example, using a transgenic animal of the present invention, to which the normal DNA has been transferred, it is possible to elucidate the pathological mechanism of the hyperergasia of the protein of this invention and the disease involved by the protein of this invention, and to study the therapeutic method of these diseases.

Because a mammal, to which exogenous normal DNA of this invention has been transferred, shows increase in the liberated protein of this invention, it can be used for the screening test of a therapeutic medicine of the diseases related to the protein of this invention.

In the meantime, the non-human mammal, to which the exogenous normal DNA of this invention is transferred, can be bred over generations in typical breeding environment as an animal retaining the DNA, upon confirmation of stable retention of the DNA by mating. Moreover, upon incorporation of the objective DNA in the aforementioned plasmid, it can be used as a starting material. A DNA construct containing a promoter can be prepared according to general DNA engineering technique. The transfer of the abnormal DNA of this invention in the stage of fertilized ovum is ensured to be present in excess in every germinal cell and every somatic cell of the target mammal. The existence of abnormal DNA of this invention in the germinal cell of created animal after DNA transfer means that every germinal cell and every somatic cell in every progeny of the created animal retain the DNA of the present invention. The offspring of this kind of animal that inherited DNA has DNA of this invention in every germinal cell and every somatic cell. By obtaining homozygote animals having the introduced DNA in both the homologous chromosomes and mating male and female of the animals, every offspring can be bred over generations such that the DNA is retained.

A non-human mammal having the abnormal DNA of this invention highly expresses the abnormal DNA of this invention, and inhibition of the function of the endogeneous normal DNA sometimes causes ultimately functionally inactive adiaphoria to the protein of this invention. Thus, it can be utilized as a pathological model animal. For example, using an animal, to which the normal DNA of this invention has been transferred, elucidation of the pathological mechanism of functionally inactive adiaphoria to the protein of this invention and consideration of the treatment method of these diseases can be afforded.

As a concrete possibility of use, an animal that highly expresses the abnormal DNA of this invention can be a model to clarify the functional inhibition (dominant negative action) of normal protein by abnormal protein of this invention in functionally inactive adiaphoria to the protein of this invention.

Because a mammal, to which the extrageneous abnormal DNA of this invention has been transferred, shows condition of increase in the liberated protein of this invention, it can be utilized for screening test of a therapeutic drug of functionally inactive adiaphoria to the protein of this invention.

As a possibility of other use of the above-mentioned two kinds of the transgenic animals of this invention, for example,

(1) use as cell source for tissue culture,
(2) analysis of relationship with a protein that is specifically expressed or activated due to the protein of this invention, by a direct analysis of DNA or RNA in the tissue of the transgenic animals of this invention or by analysis of protein expressed by the DNA in the tissue,
(3) study of cell function from tissue generally difficult to culture, by culturing cells of tissue having a DNA by general tissue culture technique and using them,
(4) screening of a pharmaceutical agent that enhances the function of cells by the use of the cell described in the above-mentioned (3), and
(5) isolation and purification of mutant protein of this invention and production of its antibody, and the like.

Furthermore, clinical condition of the diseases relating to the protein of this invention, including functionally inactive adiaphoria to the protein of this invention can be investigated using the transgenic animal of this invention, and detailed pathological findings in each organ of the disease model relating to the protein of this invention can be obtained, thus contributing to the development of a new therapeutic method, and study and therapy of secondary disease due to said disease.
Establishment of cultured cell is also possible by removing each organ from the transgenic animal of this invention, followed by dicing, liberating DNA-transferred cell by a protease such as trypsin, and culturing thereof. Moreover, characterization of a cell producing the protein of this invention, relationship with hypophosphatemia, or signal transduction mechanism thereof can be examined and look for abnormality therein and the like, thus providing effective research material for the elucidation of the protein of this invention and its action.
Furthermore, for the development of a therapeutic medicine of diseases relating to the protein of this invention, including functionally inactive adiaphoria to the protein of this invention, by the use of the transgenic animal of this invention, an effective and rapid screening method of a said therapeutic drug of the disease can be provided, using the aforementioned test method, quantification method and the like. In addition, using the transgenic animal of this invention or an exogenous DNA expression vector of this invention, a DNA therapy of diseases relating to the protein of this invention can be studied and developed.

(6) Preparation of knockout animal

The present invention further provides a non-human mammal embryonic stem cell where the DNA of this invention is inactivated and non-human mammal deficient in expression of DNA of this invention.
Accordingly, the present invention provides:

(1) a non-human mammal embryonic stem cell where the DNA of this invention is inactivated,
(2) the embryonic stem cell of (1), which is a cell having β-galactosidase gene derived from *Escherichia coli*,
(3) the embryonic stem cell of (1), which is neomycin-resistant,
(4) the embryonic stem cell of (1), wherein the non-human mammal is a rodent,
(5) the embryonic stem cell of (4), wherein the rodent is a mouse,
(6) Non-human mammal deficient in expression of DNA of the present invention,
(7) the animal of (6), wherein a reporter gene can be expressed under regulation of promoter of the protein of this invention,
(8) the animal of (7), wherein the reporter gene is β-galactosidase gene derived from *Escherichia coli*,
(9) the animal of (6), wherein the non-human mammal is a rodent,
(10) the animal of (7), wherein the rodent is a mouse, and
(11) a method for screening a test compound or a salt thereof that promotes a promoter activity of the protein of this invention, which comprises administering the compound to the animal of (7) and detecting expression of the reporter gene.

The non-human mammal embryonic stem cell where the DNA of this invention is inactivated means an embryonic stem cell (hereinafter to be briefly referred to as ES cell) of a non-human mammal, wherein the DNA does not substantially have the expression capability of the protein of this invention (hereinafter sometimes to be referred to as knockout DNA of the present invention), which is achieved by artificially introducing a mutation to the DNA of this invention possessed by the non-human mammal to suppress expression capability of DNA, or by substantially obliterating the activity of the protein of this invention that the DNA codes for.
As the non-human mammal, those similar to the aforementioned can be used.
As a method for artificially introducing a mutation to the DNA of the present invention, for example, a part or the entire DNA sequence can be deleted, or different DNA can be inserted or substituted by genetic engineering technique. With these mutations, a knockout DNA of this invention can be prepared, for example, by shifting the reading frame of codon or destroying the function of promoter or exon.

Specific examples of the non-human mammal embryonic stem cell where the DNA of this invention is inactivated (hereinafter to be briefly referred to as DNA-inactivated ES cell of this invention or knockout ES cell of this invention) can be obtained as follows. First, the DNA of this invention that the objective non-human mammal possesses is isolated, and a drug resistant gene represented by neomycin resistant gene and hygromycin resistant gene, a reporter gene represented by lacZ (β-galactosidase gene) and cat (chloramphenicol acetyl transferase gene) or the like is inserted into the exon portion of the DNA to destroy its function, or a DNA sequence (e.g., poly A-adding signal and the like) that terminates transcription of gene into an intron portion between the exons, in order to construct a DNA chain (hereinafter to be briefly referred to as targeting vector) having a DNA sequence constructed to consequently destroy gene by preventing synthesis of complete messenger RNA. Then, the DNA chain is transferred into the chromosome of the animal by, for example, homologous recombination. The knockout ES cell of the present invention is established by analyzing the obtained ES cell by southern hybridization analysis using the DNA sequence on the DNA of this invention or in the vicinity thereof as a probe, or by PCR using the DNA sequence on a targeting vector, and a DNA sequence in the vicinity of the DNA of the present invention that is other than the DNA of the present invention and is used for preparation of the targeting vector, as primers.

The original ES cell in which the DNA of this invention is inactivated by homologous recombination and the like, may be already established one as aforementioned, or even a new ES cell established according to the known method of Evans and Kaufma. For example, in the case of mouse ES cell, ES cell of 129 strain is generally used at present. However, since the immunological background of the cell of 129 strain is unclear, the ES cell which is established by the use of C57BL/6 mouse, BDF1 mouse that has been established by crossing C57BL/6 with DBA/2 (F1 of C57BL/6 and DBA/2) to increase the number of eggs obtained from C57BL/6, and the like can be also alternatively used, with the aim of obtaining an ES cell from pure strain and having clear immunologically and genetic background, and the like. BDF1 mouse advantageously produces many eggs that are strong, and in addition, it is derived from C57BL/6 mouse. Therefore, when a pathological model mouse is created from ES cell obtained from the BDF1 mouse, it is advantageous that the genetic background of the BDF1 mouse can be changed to that of C57BL/6 mouse by backcrossing with C57BL/6 mouse.

For the establishment of ES cell, blastocyst at day 3.5 after fertilization is generally used. In addition, many early embryos can be efficiently obtained by getting 8 cell embryo and culturing it up to blastocyst.

While either male or female ES cell can be used, generally, male ES cell is more convenient for creating a chimera of germ line than a female one. For eliminating complicated culture procedure, moreover, it is desirable to judge the sexuality of the cell as early as possible.

A method for judging sexuality of ES cells comprises, for example, a method comprising amplifying and detecting a gene in the sex determining region on Y chromosome by PCR.

Using this method, about the number of ES cells in one colony (about 50) is enough for karyotype analysis, though conventional method required about $10^6$ cells. Thus, primary selection of ES cells in the early stage of culture can be made based on judgment of sexuality. The selection of male cells in the early stage drastically reduces labor in the early stage of the culture.

The secondary selection can be made by, for example, confirmation of the number of chromosomes by G-binding method and the like. Although the number of chromosomes in the obtained ES cells is desirably 100% of the normal number, if it is difficult to achieve 100% due to physiological manipulation for establishment and the like, the gene of ES cell is desirably re-cloned into a normal cell (e.g., cell wherein number of chromosome is 2n = 40 for mouse) after the knocking out.

Although the embryonic stem cell line obtained in this manner generally shows highly superior proliferation performance, careful subculture thereof is necessary, because it easily loses the ability of ontogeny. For example, the cell is cultured according to a method comprising culture on a suitable feeder cell such as STO fibroblast in the presence of LIF (1 - 10000 U/ml) in a $CO_2$ culture vessel (preferably 5% $CO_2$, 95% air or 5% oxygen, 5% $CO_2$, 90% air) at about 37°C, or other method, and for subculture, for example, a method is employed which comprises a treatment with a trypsin/EDTA solution (generally 0.001 - 0.5% trypsin/0.1 - 5 mM EDTA, preferably about 0.1% trypsin/1 mM EDTA) to give a single cell, and seeded on a newly prepared feeder cell, and the like. Such subculture is done generally every 1 to 3 days. On this occasion, the cells are observed and when a morphologically abnormal cell is found, the culture cell is desirably discarded.

ES cells can be differentiated to various types of cells of, for example, vertex muscle, visceral muscle, cardiac muscle and the like by single layer culture until they reach high density or by float culture until cell agglomeration is formed under suitable conditions [M. J. Evans and M. H. Kaufman, Nature, vol. 292, p. 154 (1981); G. R. Martin, Proc. Natl. Acad. Sci. U.S.A., vol. 78, p. 7634 (1981); T. C. Doetschman et. al., Journal of Embryology and Experimental Morphology, vol. 87, p. 27 (1985)]. The cell deficient in expression of the DNA of the present invention, which is obtained by differentiating the ES cell of the present invention, is useful for cell biological investigation of the protein of this invention *in vitro.*

Non-human mammal deficient in expression of the DNA of this invention can be distinguished from normal animals by measuring and indirectly comparing the expression level of the mRNA of said animal by a publicly known method.

As the non-human mammal, those similar to the aforementioned can be used.

The non-human mammal which is deficient in the expression of DNA of the present invention can be produced as follows. For example, a targeting vector prepared as mentioned above is introduced into a mouse embryonic stem cell or mouse ovum, and as a result of the introduction, a DNA sequence in the targeting vector in which the DNA of this invention is inactivated, is replaced with the DNA of this invention on the chromosome of the mouse embryonic stem cell or mouse ovum, by homologous recombination, whereby the DNA of this invention can be knocked out.

The cell wherein the DNA of this invention is knocked out can be judged by southern hybridization analysis using a DNA sequence on the DNA of this invention or in the vicinity thereof as a probe, or by PCR using, as primers, the DNA sequence on a targeting vector and a DNA sequence in the vicinity that is other than the DNA of the present invention and was used as the targeting vector. When a non-human mammal embryonic stem cell is used, a cell line wherein the DNA of this invention is inactivated by gene homologous recombination is cloned, and the cells are injected at a suitable stage, for example, into 8 cell embryo or blastocyst of non-human mammal, and the chimeric embryo prepared is transplanted into the uterus of the pseudopregnant non-human mammal. The created animal is a chimeric animal consisting of cells having a normal locus of the DNA of the present invention and cells having locus of artificially mutated DNA of the present invention.

When part of the germ cells of the chimeric animal has the locus of mutant DNA of the present invention, such chimeric individual and a normal individual are mated to give individual group, from which an individual whose entire tissues consist of cells having the locus of DNA of the present invention in which artificial mutation was added, can be obtained by, for example, judgment of coatcolor and the like. The thus-obtained individual is generally a heterozygote which is deficient in the expression of the protein of this invention. The heterozygotes, which is deficient in the expression of the protein of this invention are mated each other and the homozygote which is deficient in the expression of the protein of this invention, can be obtained from their offspring.

When an ovum is used, for example, a transgenic non-human mammal incorporating a targeting vector in chromosome can be obtained by injecting a DNA solution into an ovum nucleus by a microinjection method, and selecting one that has a mutation in the locus of DNA of this invention by gene homologous recombination, as compared to such transgenic non-human mammal.

An individual in which the DNA of this invention is knocked-out can be bred over generations in ordinary breeding environment, upon confirmation of knocked-out of said DNA in the individual animal obtained by mating.

Moreover, establishment and maintenance of germ line can be performed by following the conventional methods. That is, a homozygote animal having said inactivated DNA in both the homologous chromosomes can be obtained by mating male and female animal retaining said inactivated DNA. The homozygote animal thus obtained can be reproduced efficiently by breeding in the state where normal individual is 1 and homozygote are plural relative to a mother animal. By mating male and female heterozygote animals, homozygote and heterozygote animals having said inactivated DNA can be bred over generations.

Non-human mammal embryonic stem cell wherein the DNA of this invention is inactivated is highly useful for creating the non-human mammal deficient in expression of DNA of the present invention. In addition, a mouse deficient in expression of the protein of this invention lacks various biological activities that can be induced by the protein of this invention. Since it can be a model of the disease caused by inactivation of the biological activity of the protein of this invention, it is useful for the investigation of the cause of such disease and consideration of the treatment methods.

Moreover, in an animal that expresses the protein of this invention wherein the structural gene of the protein of this invention is substituted by a reporter gene, the reporter gene is present under the control of the promoter of the protein of this invention, so that the activity of the promoter of the protein of this invention can be detected by tracing the expression of the substance that the reporter gene encodes. For example, when a part of a DNA region encoding the protein of this invention is substituted by β-galactosidase gene (lacZ) derived from *Escherichia coli*, β-galactosidase expresses instead of the protein of this invention in a tissue where the protein of this invention inherently expresses. Accordingly, for example, the expression manner of the protein of this invention in an animal in vivo can be conveniently observed by staining using a reagent that becomes a substrate of β-galactosidase such as 5-bromo-4-chloro-3-indolyl-β-galactopyranoside (X-gal). To be specific, a mouse deficient in the protein of this invention or the tissue section is immobilized with glutaraldehyde and the like. After washing with Dulbecco's phosphate buffer saline (PBS) and reacting with a staining solution containing X-gal at room temperature or around 7°C for about 30 min to 1 hr, the tissue sample is washed with a 1 mM EDTA/PBS solution to terminate the β-galactosidase reaction and the color development may be observed. In addition, mRNA encoding lacZ may be detected according to a conventional method.

Such animal deficient in expression of the protein of this invention is extremely useful for screening a substance that activates or inactivates the promoter of the protein of this invention, and can greatly contribute to the clarification of the cause of various diseases due to deficient expression of the protein of the present invention or the development of the therapeutic drug for the diseases.

In this specification and drawings, when bases and amino acids are expressed using abbreviations, they follow IUPAC-IUB Commission on Biochemical Nomenclature or common abbreviations in the art. The examples are shown below. For amino acids that may have an optical isomer, L form is presented unless it is specified.

DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine

Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : aspargine
Gln : glutamine
pGlu : pyroglutamic acid
Me : methyl group
Et : ethyl group
Bu : butyl group
Ph : phenyl group
TC : thiazolidine-4(R)-carboxyamide group

Substituents, protecting groups, and reagents generally used in this specification are presented in the following abbreviations.

Tos : p-toluenesulfonyl
CHO : formyl
Bzl : benzyl
$Cl_2Bzl$ : 2,6-dichlorobenzyl
Bom : benzyloxymethyl

Z : benzyloxycarbonyl
Cl-Z : 2-chlorobenzyl oxycarbonyl
Br-Z : 2-bromobenzyl oxycarbonyl
Boc : t-butoxycarbonyl

DNP : dinitrophenol
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboxyimide
DCC : N,N'-dicyclorohexylcarbodiimide

The SEQ IDs in the sequence listing of this specification present the following sequences.

[SEQ ID:1]

The amino acid sequence of the protein of this invention, which is derived from human.

[SEQ ID:2]

The base sequence of a DNA encoding the protein of this invention, which is derived from human.

[SEQ ID:3]

The base sequence of a DNA containing a DNA encoding the protein of this invention derived from human, which is inserted in plasmid pCR-PHOS.

[SEQ ID:4]

The base sequence of a primer used for cloning a DNA encoding the protein of this invention, which is derived from human.

[SEQ ID:5]

The base sequence of a primer used for cloning a DNA encoding the protein of this invention derived from human.

[SEQ ID:6]

The base sequence of a primer used for cloning a DNA encoding the protein of this invention derived from human.

[SEQ ID:7]

The base sequence of an Oligo$(dT)_{18}$ linker-primer containing XhoI site used in Example 1.

[SEQ ID:8]

The base sequence of a primer used for cloning a DNA encoding the protein of this invention derived from human.

[SEQ ID:9]

The base sequence of a primer used for cloning a DNA encoding the protein of this invention derived from human.

[SEQ ID:10]

The base sequence of a primer used for cloning a DNA encoding the protein of this invention derived from human.

*Escherichia coli* transformant, TOP10/pCR-PHOS obtained in Example 1 described below was deposited at the Ministry of International Trade and Industry, Agency of Industrial Science and Technology, National Institute of Bioscience and Human Technology (NIBH) as deposit number FERM BP-7172 on May 29, 2000 and at Institute for Fermentation, Osaka (IFO) as deposit number IFO 16431 on May 11, 2000.

*Escherichia coli* transformant, MM294 (DE3)/pTCII-mPHOS-2 obtained in Example 2 described below was deposited at the International Patent Organism Depository National Institute of Advanced Industrial Science and Technology, AIST Tsukuba Central 6, 1-1 Higashi 1-chome Tsukuba-shi, Ibaraki-ken Japan, as deposit number FERM BP-7625 on June 7, 2001 and at Institute for Fermentation, Osaka (IFO), 2-17-85, Jyusohonmachi, Yodogawa-ku, Osaka-shi, Osaka Japan, as deposit number IFO 16646 on May 31, 2001.

*Escherichia coli* transformant, JM109/pT-PHOSF-11 obtained in Example 6 described below was deposited at the International Patent Organism Depository National Institute of Advanced Industrial Science and Technology, AIST Tsukuba Central 6, 1-1 Higashi 1-chome Tsukuba-shi, Ibaraki-ken Japan, as deposit number FERM BP-7626 on June 7, 2001 and at Institute for Fermentation, Osaka (IFO), 2-17-85, Jyusohonmachi, Yodogawa-ku, Osaka-shi, Osaka Japan, as deposit number IFO 16647 on May 31, 2001.

## Examples

The present invention is explained in more detail by referring to examples, which are not to be construed as limitative. The genetic engineering procedures using *Escherichia coli* were performed according to the methods described in the Molecular Cloning.

[Example 1] Cloning of a cDNA encoding novel protein, phosphatonin, having phosphaturic activity and/or hypophosphatemia-inducing activity

A cDNA library derived from tumor of OHO patients was prepared as in the following. That is, a total RNA was obtained from a paranasal sinus tumor tissue of OHO patients according to phenol-chloroform-isoamyl alcohol, LiCl precipitation method (PLANT CELL PHYSIOLOGY, 36, 85-93, 1995). About 6 μg of poly(A)$^+$ RNA was obtained from the extracted total RNA (about 480 μg) using Oligotex $(dT)_{30}$-Super (Takara Shuzo Co., Ltd).

The cDNA library was prepared according to the method of Gubler and Hoffman. The first strand DNA was synthesized by using poly(A)$^+$ RNA (2.5 μg), Oligo$(dT)_{18}$-linker primer ($(GA)_{10}$ ACGCGTCGACTCGAGCGGCCGCG-GA CCG$(T)_{18}$)(SEQ ID:7) containing XhoI site, 5-methyl dCTP, dATP, dGTP, dTTP, RAV-2 reverse transcriptase and SuperscriptII reverse transcriptase. The second strand DNA was synthesized by reacting RNase H, DNA polymerase I and dNTP mixture to it. The second strand DNA was blunt-ended, and was ligated with EcoRI-NotI-BamHI adaptor (Takara Shuzo Co., Ltd) by using T4 DNA ligase. This was cleaved with restriction enzyme XhoI, followed by removal of a low molecular weight DNA by using a spin column and a ligation with λZAPII (EcoRI-XhoI cleaved)(Stratagene) using T4 DNA ligase. The thus obtained λ phage vector was incorporated into a λ phage precursor protein using a *in vitro* packaging kit (Stratagene) to give a λ phage library. The titer of the library was measured by using *Escherichia coli* XL1 Blue MRF' as a host. The titer of the primary λ phage library was 1.0 x $10^6$ pfu/ml.

The primary λ phage library was amplified at 4°C overnight by using *Escherichia coli* XL1 Blue MRF' as a host,. About 5 x $10^4$ plaques were formed per one dish (diameter 150 mm) and SM buffer (10 ml) was layered thereon. Dimethyl sulfoxide was added to the recovered SM buffer to a final concentration of 7% and preserved at -80°C. The titer of the amplified λ phage library was not less than 1.0 x $10^9$ pfu/ml.

Cloning of phosphatonin cDNA was performed according to PCR method. The λ phage library derived from tumor of OHO patients, which was prepared in the above, was used as a template, the synthetic oligo DNA of SEQ ID:4 on the 5' side of multicloning site of the host vector, pBluescript SK(+/-) that was used for the preparation of cDNA library, was used as a forward primer, and the synthetic oligo DNA of SEQ ID:5 on the 3' side of the stop codon of a phosphatonin gene sequence described in international publication WO 99/60017 of international patent application by Rowe P.S.N. was used as a reverse primer.

5'-GGAAACAGCTATGACCATG-3'

(SEQ ID:4)

5'-TCAGGTGGCTCTCCTCTACATCAACTCACA-3'

(SEQ ID:5)

The PCR reaction was carried out in a system containing TaKaRa Ex Taq (Takara Shuzo Co., Ltd), and using a Thermal Cycler (GeneAmp PCR System, PE Applied Biosystems) under the conditions of 1 cycle at 95°C for 3 min, 30 cycles at 95°C for 45 sec, 58°C for 45 sec and 72°C for 3 min, 1 cycle at 72°C for 5 min, and standing at 4°C.

The amplified fragment thus obtained was subjected to an electrophoresis using 1% agarose gel, and the PCR products in a main band was extracted and purified, inserted into pCRII-TOPO vector (Invitrogen) using a TOPO TA cloning kit (Invitrogen) and introduced into *Escherichia coli* TOP10 strain.

Plasmid DNA was extracted from the obtained transformant, subjected to PCR reaction using BigDye Terminator Cycle Sequence Ready Reaction Kit (PE Applied Biosystems), and the base sequence of the cDNA fragment was determined by ABI PRISM™ 377 DNA sequencer (PE Applied Biosystems).

The plasmid DNA retained by the obtained clone #1 had a sequence (1713 bases) containing essentially the same base sequence as the sequence (1290 bases) encoding Val (1st) to Asp (430th) of phosphatonin described in international publication WO 99/60017 of international patent application by Rowe P.S.N., and encoded phosphatonin protein consisting of 525 amino acids.

The insert sequence of plasmid DNA retained by clone #1 was different by several bases from the insert sequence of phosphatonin gene sequence described in international publication WO 99/60017 of international patent application by Rowe P.S.N. and that of plasmid DNA retained by simultaneously obtained other clones. To determine the true phosphatonin gene sequence, therefore, PCR was performed using Pyrobest DNA polymerase with a higher fidelity. The λ phage library prepared in the above, which was derived from tumor of OHO patients was used as a template and as a primer, synthetic oligo DNA of SEQ ID:6, which starts from the 5' side of initiation codon ATG of the above-mentioned clone A was used as a forward primer and synthetic oligo DNA of SEQ ID:5 was used as a reverse primer.

5'-CTCAAAGATGCGAGTTTTCTGTGTGGGA-3'

(SEQ ID:6)

The PCR reaction was carried out using a Thermal Cycler (GeneAmp PCR System, PE Applied Biosystems) under the conditions of 30 cycles at 98°C for 10 sec, 56°C for 45 sec and 72°C for 3 min, and standing at 4°C.

The amplified fragment thus obtained was subjected to a gel electrophoresis using 1% agarose gel, and the PCR products in a main band was extracted and purified, inserted into pCR-Blunt vector (Invitrogen) using a Zero Blunt PCR Cloning Kit (Invitrogen) and introduced into *Escherichia coli* TOP10 strain.

Plasmid DNA was extracted from the obtained transformed bacteria, subjected to PCR reaction using BigDye Terminator Cycle Sequence Ready Reaction Kit (PE Applied Biosystems), and the base sequence of cDNA fragment was determined by ABI PRISM™ 377 DNA sequencer (PE Applied Biosystems). As a result, a transformant: *Escherichia coli* TOP10/pCR-PHOS (clone #9) containing plasmid pCR-PHOS retaining a DNA encoding the phosphatonin protein of this invention was obtained.

The plasmid DNA retained by the obtained TOP10/pCR-PHOS (clone #9) had a sequence (1662 bases) presented by SEQ ID:3 containing the sequence (1575 bases) presented by SEQ ID:2, and encoded phosphatonin protein consisting of 525 amino acids presented by SEQ ID:1 (Figs. 1-4). The molecular weight of the protein portion (including signal peptide) of the protein of this invention was 58.4 kDa as deduced from the amino acid sequence.

The base sequence presented by SEQ ID:3 had a base sequence essentially identical to a sequence (1290 bases) encoding Val (1st) to Asp (430th) of phosphatonin described in international publication WO 99/60017 of international patent application by Rowe P.S.N. and had a novel sequence (285 bases) encoding 95 amino acid sequence starting with Met in the 5' region thereof. In the sequence common to base sequence presented by SEQ ID:3 and base sequence of phosphatonin described in international publication WO 99/60017 of international patent application by Rowe P.S.N., only the 293rd base was different (Rowe P.S.N. publication : $G^1$ → SEQ ID:3: $C^{293}$), along with which the amino acid sequence presented by SEQ ID:1 was different in one residue (Rowe P.S.N. publication: $Val^1$ → SEQ ID:1: $Leu^{96}$).

The hydrophobicity of the amino acid sequence presented by SEQ ID:1 was assumed by the Kite Doolittle method. As a result, the presence of signal sequence was suggested (Fig. 5) because N-terminal region starting with Met clearly showed topically high hydrophobicity. Because a sequence ($Ala^{17}$ - $Thr^{19}$), which is identical to the N-terminal sequence (Ala-Pro-Thr-) of interleukin-2 as the secreted protein, is also present in SEQ ID:1, $Met^{1}$ - $Ala^{16}$ was assumed to be a signal sequence. As the region of the amino acid sequence presented by SEQ ID:1 except $Met^{1}$ - $Ala^{16}$ showed extremely high hydrophilicity and was free of transmembrane region, the protein of this invention was suggested to be a protein secreted extracellularly. This matches well with the fact that phosphatonin is a humoral factor.

The motif found in the amino acid sequence presented by SEQ ID:1 by using a high speed homology search tool "Gapped BLAST", is shown in Table 1.

[Table 1]

| Motif | Site (SEQ ID:1) | Amino acid seq. |
|---|---|---|
| Amidation | 50-53 | LGKR |
| | 465-468 | HGRK |
| Asn-glycosylation | 477-480 | NNST |
| | 478-481 | NSTR |
| Glycosaminoglycan attachment | 256-259 | SGDG |
| RGD | 247-249 | RGD |
| Myristoylation | 82-87 | GSSKSQ |
| | 111-116 | GLRMSI |
| | 238-243 | GSGYTD |
| | 314-319 | GNTIGT |
| | 361-366 | GSQNAH |
| | 386-391 | GSSDAA |
| | 410-415 | GVDHSN |
| | 484-489 | GMPQGK |
| Protein kinase C phosphorylation | 24-26 | TEK |
| | 59-61 | SSK |
| | 83-85 | SSK |
| | 91-93 | TNR |
| | 103-105 | SNK |
| | 172-174 | TPR |
| | 213-215 | THR |
| | 298-300 | TKK |
| | 323-325 | TAK |
| | 406-408 | STR |
| | 414-416 | SNR |
| | 479-481 | STR |
| | 498-500 | SNR |
| | 503-505 | SSR |

[Table 1] (continued)

| Motif | Site (SEQ ID:1) | Amino acid seq. |
|---|---|---|
| Casein kinase II phosphorylation | 30-33 | SCVE |
| | 59-62 | SSKE |
| | 73-76 | SLSE |
| | 103-106 | SNKE |
| | 234-237 | SDFE |
| | 272-275 | TGPD |
| | 289-292 | SEAE |
| | 294-297 | THLD |
| | 319-322 | TRDE |
| | 323-326 | TAKE |
| | 333-336 | SLVE |
| | 420-423 | TLNE |
| | 518-521 | SSSE |
| | 520-523 | SESD |
| | 522-525 | SDGD |
| cAMP-dependent protein kinase phosphorylation | 501-503 | RRFS |
| Tyrosine kinase phosphorylation | 135-142 | KLHDQEEY |

The protein of this invention presented by SEQ ID:1 had a sugar chain binding site in $Asn^{477}$ - $Thr^{480}$ and $Asn^{478}$ - $Arg^{481}$, and a glycosaminoglycan binding site in $Ser^{256}$ - $Gly^{259}$. The protein of this invention presented by SEQ ID:1 had RGD sequence ($Arg^{247}$ - $Asp^{249}$) involved in cell adhesion. The RGD sequence is known to be present in collagen, vitronectin, fibrinogen, von Willebrand factor and the like, and expected to contribute to the interaction between the protein of this invention and a cell in as well.

The protein of this invention presented by SEQ ID:1 has many phosphorylation site for protein kinase C, casein kinase II, cAMP-dependent protein kinase and tyrosine kinase (Table 1), and these sites were considered to play some role in the biological activity of the protein of this invention and a partial peptide thereof. In addition, the protein of this invention presented by SEQ ID:1 had many myristoylation site characteristic of phosphorylated glycoproteins such as collagen, vitronectin, fibronectin, osteopontin, dentin-sialophosphoprotein (DSSP) and the like (Table 1). The C-terminal region of the protein of this invention contains a sequence rich in aspartic acid and serine ($Asp^{509}$ - $Ser^{522}$; DSSESSDSGSSSES), which showed high 79% homology with a sequence repeatedly seen in DSSP, such as $Asp^{686}$- $Ser^{699}$; DSSDSSDSSSSSDS. A similar sequence is also present in osteopontin ($Asp^{101}$ - $Asp^{116}$; DDSHQS-DESHHHSDESD), suggesting a involvement of the protein of this invention in bone formation.

While phosphatonin is considered to be cleaved by a PHEX gene product, the amino acid sequence presented by SEQ ID:1 has an amino acid sequence ($Ala^{327}$ - $Ser^{333}$; ADAVDVS) that matches with the substrate specificity of zinc metalloendopeptidase, so that the site ($Val^{330}$ - $Asp^{331}$) is expected to be cleaved by a PHEX gene product.

[Example 2] Expression of recombinant Phosphatonin in *Escherichia coli*

A expression plasmid for a mature phosphatonin without a signal sequence ($Met^{1}$ - $Ala^{16}$) was constructed and expressed in *Escherichia coli.*

First, a DNA fragment encoding mature phosphatonin was amplified by PCR (98°C × 45 sec, 56°C × 45 sec, 72°C × 3 min, 30 cycles) using pyrobest DNA polymerase (Takara Shuzo Co., Ltd) using oligo DNA comprising 5' terminal sequence of the mature phosphatonin which was added Nde I site and initiation codon ATG, as a forward primer (5'-CATATGGCACCAACATTTCAACCACAGA-3', SEQ ID:8), oligo DNA having a DNA sequence of 3' side of a stop codon as a reverse primer (5'-CTCTCGTCGACATCAACTCACA-3', SEQ ID:9), and plasmid pCR-PHOS-9 for animal cell expression (Example 1) as a template. The obtained PCR product was purified by an electrophoresis using 1% agarose gel, ligated to a pCR Blant vector (Invitrogen), and transformed to TOP10 competent cell. Plasmid DNA (pCR-mPHOS) was prepared from 12 positive strains obtained by colony PCR, and 3 strains having an insert in the reverse direction, which was confirmed by a SacI digestion, was selected. All of these plasmids had a correct DNA sequence. These three kinds of plasmid DNAs were digested with Nde I and Bam HI by utilizing Bam HI site on the 5' side of the insert in a host vector, and the insert was purified by an electrophoresis using 1% agarose gel. pTCII (Nde I/Bam HI) arm prepared in the same manner and these inserts were ligated and transformed to JM109 competent cell. A plasmid DNA was prepared from thus obtained transformant (JM109/pTCII-mPHOS-2), and the presence of an insert was confirmed by Nde I/Bam HI digestion. pTCII-mPHOS-2 (Fig. 6) had a DNA sequence that encodes the correct mature phosphatonin.

Then, pTCII-mPHOS-2 was transfected to *Escherichia coli* (MM294 (DE3)) competent cell having T7 RNA polymerase gene (under regulation of lac promoter) to give *Escherichia coli* MM294 (DE3)/pTCII-mPHOS-2.

This transformant was cultured in a 2 L flask containing 1 L of LB medium (1% pepton, 0.5% yeast extract, 0.5% sodium chloride) supplemented with 10 μg/ml of tetracycline at 37°C for 8 hr with shaking. The culture medium thus obtained was transferred into a 50 L fermentation tank charged with 19 L of a main fermentation medium (1.68% dibasic sodium phosphate, 0.3% potassium dihydrogen phosphate, 0.1% ammonium chloride, 0.05% sodium chloride, 0.05% magnesium sulfate, 0.02% defoaming agent, 0.00025% ferrous sulfate, 0.0005% thyamine hydrochloride, 1.5% glucose and 1.5% casamino acid) containing 5 μg/ml of tetracycline, and the culture with aerating and stirring was started at 37°C. When the turbidity of the culture medium reached about 500 Klett unit, isopropyl-β-D-thiogalactopyranoside (IPTG) was added to the medium to give a final concentration of 75 μM, and the culture was continued further for 3.5 hr to reach 1530 Klett. By centrifugation and separation of this culture medium ultimately gave about 300 g of wet bacterial cell, which was frozen and kept at -80°C.

The amount of Phosphatonin expressed by this bacterial cell was estimated to be about 15 mg/g wet bacterial cells (300 mg/L), judging from the degree of stain of 60 Kd band derived from Phosphatonin on the SDS-PAGE of the bacterial cell extract. These bacterial cells were sequentially subjected to ultrasonication in 20 mM EDTA (pH 6) and 20 mM Tris-HCl/8M Urea (pH 8), to examine the expression manner of mature phosphatonin. As a result, all of them were present in the soluble fractions.

[Example 3] Purification of recombinant Phosphatonin

Two liters of 50 mM 2-(N-morpholino)ethanesulfonic acid (MES)/NaOH containing 1 mM p-amidinophenylmethanesulfonylide hydrochloride (APMSF hydrochloride)(pH 6.0) was added to the bacterial cell (50 g) obtained in Example 2, and the bacterial cells disrupted three times at 4°C for 10 min by using a sonicator (Sonifer 450) (Branson), followed by a centrifugation (8000 rpm, 30 min) to give a supernatant. This supernatant was concentrated and substituted with 50 mM MES/NaOH buffer (pH 6.0) by using an ultrafiltration membrane (regenerated cellulose membrane, molecular weight cut off; 10K (k dalton), membrane area; 0.1 $m^2$ X 2 sheets)(Sartorius), and subjected to centrifugation (6000 rpm, 20 min) to give a supernatant (1000 mL). The supernatant (500 mL) was adsorbed on SP-Toyopearl 550C (5cm I.D. X 20cm L, 400mL)(Tosoh Corp.) equilibrated with 50 mM MES/NaOH (pH 6.0). It was washed with 0.5M NaCl 50 mM MES/NaOH (pH 6.0) at a flow rate of 20 mL/min, and Phosphatonin was eluted with 50 mM MES/NaOH containing 0.8M NaCl (pH 6.0). This step was repeated for the remaining half of the supernatant. The eluate was concentrated and substituted with 50 mM MES/NaOH (pH 6.0) by using the aforementioned ultrafiltration membrane, adsorbed on CM-5PW (21.5 mm I.D. X 150 mm L, 13 μm)(Tosoh Corp.) equilibrated with 50 mM MES/NaOH (pH 6.0), and eluted by linear gradient of 0 - 50% B (B = 50 mM MES/NaOH containing 1.5M NaCl (pH 6.0)) at a flow rate of 5 ml/min for 40 min, and the fraction containing Phosphatonin was pooled. This eluate was concentrated with ultrafiltration membrane (Vivaspin 20, molecular weight cut off; 10K (k dalton))(Sartorius). The concentrate was finally applied to Superdex 200 (10 mm I.D. X 30 mm L, 10 μm)(Amersham Pharmacia Biotech) equilibrated with PBS, and eluted at a flow rate of 0.5 mL/min. The elution fraction containing Phosphatonin was pooled to give about 30 mg of a sample.

With the aim of measuring the purity of the thus-obtained Phosphatonin sample, SDS polyacrylamide gel electrophoresis was applied. The sample was suspended in a sample buffer [Laemmli, Nature, 227, 680 (1979)] supplemented with 100 mM DTT, and heated at 95°C for 1 min, which was subjected to electrophoresis using Multi gel 10/20 (Daiichi Pure Chemicals). Staining of the gel with Coomassie brilliant blue after the electrophoresis showed a single band of protein at about 60 Kd. From this results, the Phosphatonin sample was proved to be homogenious and to have a quite high purity (Fig. 7).

With the aim of measuring, the purity of Phosphatonin was analyzed by an ion exchange and a reversed phase HPLC using a GilsonHPLC system (Gilson). For the ion exchange HPLC, Phosphatonin (30 μg) was injected into CM-5PW (7.5 mm I.D. X 75 mm L, 10 μm)(Tosoh Corp.) equilibrated with 50 mM MES/NaOH (pH 6.0) and eluted by linear gradient of 20 - 70% B (B=50 mM MES/NaOH containing 1.5M NaCl (pH 5.8),) at a flow rate of 0.8 ml/min for 30 min. For the reversed phase HPLC, Phosphatonin (15 μg) was injected into C4P-50 (4.6 mm I.D. X 250 mm L, 5 μm)(Showa Denko K. K.) equilibrated with 30%B (A = 0.1% trifluoroacetic acid (TFA), B = 80% acetonitrile/0.1% TFA), and eluted by linear gradient of 10 - 50% B at a flow rate of 0.5 ml/min for 40 min. The detection was performed at a wavelength of 280 nm, and the obtained data was subjected to waveform treatment with a Chromatocorder 21 (System Instruments Co., Ltd.) and the purity was calculated. As a result, Phosphatonin showed a single peak. From this, the Phosphatonin sample was proved to be homogenious peak and to have a quite high purity (Fig. 8).

The amino acid composition of the obtained Phosphatonin was determined by an amino acid analyzer (HITACHI L-8500A). The results are shown in [Table 2].

The measurements of the sample matched with the theoretical value of the amino acid composition of Phosphatonin having Met on the N-terminal.

[Table 2]

| Amino acid | Value expected from base sequence of Phosphatonin | Number of residue per mole |
|---|---|---|
| Asx | 74 | 70.4 |
| Thr1) | 23 | 21.9 |
| Ser1) | 56 | 39.9 |
| Glx | 70 | 71.7 |
| Pro | 28 | 29.2 |
| Gly | 46 | 45.0 |
| Ala | 22 | 21.5 |
| Cys2) | 1 | N.D |
| Val | 13 | 13.2 |
| Met | 7 | 7.4 |
| Ile | 25 | 23.1 |
| Leu | 21 | 21 |
| Tyr | 12 | 12.0 |
| Phe | 13 | 12.1 |
| Lys | 50 | 48.1 |
| His | 18 | 17.8 |
| Trp | 1 | 0.6 |
| Arg | 29 | 25.5 |

Acid hydrolysis (6N HCl-4% thioglycolic acid, 110°C, average value of 24 and 48 hr of hydrolysis)
1) value extrapolated to 0 hr
2) not detected

Analysis was done by using about 10 µg.

The N-terminal amino acid sequence was determined using a gas phase protein sequencer (Applied Biosystems model 492). As a result, while the obtained Phosphatonin was a mixture of proteins with and without the N-terminal methionine, the sequence matched with the N-terminal amino acid sequence of Phosphatonin assumed from the base sequence (Fig. 9).

As shown in Example 1, Phosphatonin has a motif susceptible to modification with phosphorylation. Therefore, phosphorylation with casein kinase II was done. The aforementioned purified Phosphatonin (7.5 mg) was diluted with 20 mM N-2-hydroxyethylpiperazin-N'-2-ethanesulfonic acid (HEPES)/NaOH containing 15 mM NaCl, 15 mM $MgCl_2$, and 0.3 mM ATP (pH 7.5) and pre-incubated in a thermobath at 37°C for 5 min. Then casein kinase II (Calbiochem, 47 mU) was added and the mixture was reacted at 37°C for 60 min. After the completion of the reaction, the mixture was ice-cooled and concentrated by ultrafiltration membrane (Vivaspin 20, molecular weight cut off; 10K (k dalton)) (Sartorius). The concentrated solution was applied to Superdex 200 (10 mm I.D. X 30mm L, 13 µm)(Amersham Pharmacia Biotech) equilibrated with PBS, eluted at a flow rate of 0.5 mL/min and the Phosphatonin-containing fraction was pooled.

With the aim of confirming phosphorylation of Phosphatonin, the aforementioned SDS polyacrylamide gel electrophoresis was performed (multigel 12.5 (Daiichi Pure Chemicals)). The gel after electrophoresis was stained with Coomassie brilliant blue, and as a result, changes in the molecular weight were observed before and after the reaction, suggesting phosphorylation of Phosphatonin (Fig. 10).

[Example 4] Preparation of polyclonal antibody

The Phosphatonin obtained in Example 3 was emulsified with Freund's complete adjuvant or incomplete adjuvant, and for immunization, the emulsion containing 1 mg of protein was subcutaneously injected to each Kbl:JW rabbit biweekly for 10 weeks. At week 10, total blood was recovered from the rabbits via the carotid artery, followed by a centrifugation to obtain antiserum (about 70 mL). After adding preservative (0.05% sodium azide), the antiserum was kept at 4°C.

Prior to purification of antibody from the antiserum, a Phosphatonin NHS Hitrap antigen column was prepared by coupling phosphatonin (3 mg) purified in Example 3 to a NHS-Hitrap (1 mL 10 μ)(Amersham Pharmacia Biotech). The coupling yield was 95%.

The aforementioned antiserum (6 mL) was diluted two-fold with an MAPS II binding buffer (Bio-Rad Japan), adsorbed to protein A Sepharose FF (16 mm I.D. X 50 mm L, 50 μm, 10 mL)(Amersham Pharmacia Biotech) equilibrated in advance with the same buffer, and washed with protein A MAPS II binding buffer, after which IgG fraction was eluted with protein A MAPSII elution buffer (Bio-Rad Japan). The obtained fraction was immediately neutralized with 1M Tris/HCl (pH 8.0) and dialyzed against PBS at 4°C overnight. The dialysate was adsorbed on Phosphatonin NHS Hitrap antigen column (1 mL) equilibrated with 0.1M Tris/HCl (pH 8.0), and washed with the same buffer. The non-adsorbed fraction was removed with 0.1M Tris/HCl containing 0.5M NaCl (pH 8.0) and low titer antibody was removed with 0.1M acetate buffer containing 0.5M NaCl (pH 4.5). Then, a high titer antibody was eluted with 0.1M Glycine/HCl containing 0.5M NaCl (pH 2.7), and the eluted fraction was immediately neutralized with 1M Tris/HCl (pH 8.0). The eluted fractions were collected and dialyzed against PBS to purify anti-Phosphatonin antibody (16 mg).

[Example 5] Establishment of ELISA system

An ELISA system was established by using the antibody obtained in Example 4. This ELISA system was based on the sandwich method. Prior to setting up of the system, a biotinylated antibody was prepared from the antibody (3 mg) obtained in Example 4, by using EZ-Link Sulfo-NHS-LC Biotinylation Kit (Pierce). 2.7 mg of the biotinylated antibody was obtained, and the amount of biotin attached to the antibody was 7.5 biotins per 1 molecule of the antibody.

The ELISA was performed using a 96-well microwell plate (MaxiSorp Nunc). First, the anti-Phosphatonin antibody obtained in Example 4 was diluted with 50 mM sodium carbonate buffer (pH 9.6) to a concentration of 5 μg/mL, and it was added to each well by 100 μL and the plate was left standing overnight at 4°C to allow adsorption. Then, the well was washed 4 times with 200 μL of washing buffer composed of 10 mM phosphate buffer, 1 mM EDTA 0.05% (V/V), and Tween 20 (pH 7.4), and 100 μL of blocking solution (25% BlockAce (Dai-nippon Pharmaceutical Co.) diluted with PBS) was added to each well, and the plate was left standing at room temperature for 2 hr. Then, the aforementioned washing step was done, after which a sample solution such as the culture supernatant of CHO cells expressing phosphatonin (described in Example 7), or human serum and the like, diluted with PBS containing 10% BlockAce and 0.1% (V/V) Tween 20, and a standard solution wherein Phosphatonin prepared from *Escherichia coli* as shown in Example 2 was diluted to a concentration of 0.25 - 2 ng/mL, was added at 100 μL/well and allowed to bind to the antibody on the plate at room temperature for 2 hr. After the aforementioned washing step, biotynylated anti-Phosphatonin antibody diluted with PBS containing 10% BlockAce and 0.1% (V/V) Tween 20 to a concentration of 3 μg/mL was added at 100 μL/well and allowed to bind at room temperature for 1 hr. After the aforementioned washing step, horseradish peroxidase-labeled streptoavidine (Pierce) diluted with PBS containing 0.1% (V/V) Tween 20 to a concentration of 0.5 μg/mL was added at 100 μL/well and the plate was left standing at room temperature for 30 min. After the aforementioned washing step, TMB (Bio-Rad Japan) was added, and the mixture was left standing until sufficient color development is sufficiently developed, and IN sulfuric acid was added at 100 μL/well to stop the reaction. Thereafter, absorbance at 450 nm was measured. The detection limit was 0.1 ng/mL and measurable range was 0.1 - 3 ng/mL (Fig. 11). Particularly, for measurement of the supernatant of CHO cells containing a high concentration of phosphatonin, a system having a wide detection range is necessary. Thus, as an enzyme described in the above-mentioned Example, 2 μg/mL of alkaline phosphatase-labeled streptoavidine (Pierce) was used and Blue Phos (KPL) was used as a substrate. The detection limit was 0.5 ng/mL, and the measurable range was 0.5 - 30 ng/mL, in this case.

Using this measurement system, quantification in human serum was conducted. Each serum was diluted 20-fold with the aforementioned PBS containing 10% BlockAce and 0.1% (V/V) Tween 20 and subjected to the ELISA system. Comparing OHO patients and healthy adult subjects, it was found that the OHO patients showed higher values than healthy adult and that when tumor was removed, the numerical values decreased in about 20% (Fig. 12). Thus it was shown that the determination of Phosphatonin by ELISA system is extremely useful as a diagnosis of neoplastic hypophosphatemia.

[Example 6] Secretory expression of recombinant Phosphatonin in CHO cell line

Using plasmid (pCR-PHOS-9) containing the full-length cDNA of the phosphatonin as a template, a forward primer (5'-CTCAAAGATGCGAGTTTTCTGTGTGGGA-3' SEQ ID:6) containing an initiation codon, and a reverse primer (5'-CTACTTATCGTCGTCATCCTTGTAATCGTCACCATCGCTCTCACTTGA-3' SEQ ID:10) wherein a gene encoding a FLAG sequence was inserted before the stop codon, PCR reaction (98°C x 45 sec, 56°C × 45 sec, 72°C × 3 min, 30 cycles) was performed by using Pyrobest DNA polymerase (Takara Shuzo). The PCR product was subjected to 1% agarose gel electrophoresis and a band having the objective size was extracted from the gel, and purified. After adding adenine to the 3' terminal, the PCR product was ligated to pTARGET™ Vector (Promega) and transformed to JM109 competent cell. Plasmid DNA was obtained from two positive strain selected by colony PCR, and insert check by digestion with Sac I (Takara Shuzo) and DNA sequence analysis gave a plasmid (pT-PHOSF-11) having a correct insert in the forward direction (Fig. 13).

After cleaving the expression plasmid for Phohsphatonin-FLAG (pT-PHOSF-11) with restriction enzyme AhdI (Biolabs) at one site, it was introduced into CHO-K1 cell by electropolation method ($1 \times 10^7$ Cells, Plasmid 10 ug/800 ul PBS, 0.4 cm Cuvette, 960 uF-0.25 kV (Bio-Rad)). After culturing overnight in 10 mL of Ham F12-10% FCS medium (Gibco BRL), cloning of the cells by limited dilution with Ham F12-10% FCS medium containing 550 ug/ml of geneticin (Gibco BRL) was performed. The culture supernatant of each clone was screened using the ELISA system described in Example 5 to give a highly stable expression line (CHO-PHOSF-11-34-24).

[Example 7] Purification of recombinant Phosphatonin from CHO

The recombinant Phosphatonin from CHO cells was purified using an anti-Phosphatonin antibody column. Fifty milligram of anti-Phosphatonin antibody (Example 4) was coupled onto HiTrap-NHS column (5 mL, Amarsham Pharmasia) to give an anti-Phosphatonin antibody column. The culture supernatant (3 L) of a highly stable expression line (CHO-PHOSF-11-34-24) was adsorbed on the anti-Phosphatonin antibody column equilibrated with 50 mM Tris-HCl (pH 8.0), washed with Tris-HCl containing 0.5 M NaCl (pH 8) at a flow rate of 4 mL/min. The Phosphatonin was eluted with 0.1 M glycine containing 0.5 M NaCl (pH 3.0), and neutralized with 1M Tris-HCl (pH 8.0). The eluate was concentrated and substituted with PBS by ultrafiltration membrane (Vivaspin 20, molecular weight cut off; 10K (k dalton))(Sartorius) to give a preparation (2.2 mg). In the same manner as in Example 3, SDS polyacrylamide gel electrophoresis was conducted, which showed bands at about 60 Kd and at a position of high molecular weight, which was considered to be sugar chain-added form (Fig. 14).

[Example 8] Measurement of activity of recombinant Phosphatonin

The phosphorus reabsorption inhibitory activity of recombinant Phosphatonin from CHO cell, in the kidney was assayed using a normal human proximal tubule epithelial cell (RPTEC, BioWhittaker). RPTEC ($2 \times 10^4$/well) was cultured in a 24 well plate using REGM (BioWhittaker) at 37°C, 5% $CO_2$ for 3 days, and the medium was substituted by fresh RBGM (BioWhittaker) containing 0.1% BSA, followed by cultivation overnight at 37°C, 5% $CO_2$. The recombinant Phosphatonin from CHO diluted with RBGM containing 0.1% BSA (final concentration 1 μg/mL) was added to the well and the plate was incubated at 37°C, 5% $CO_2$ for 2 hr. The medium was removed and the each well was washed with 1 mL of a washing buffer (10 mM Tris-HEPES containing 137 mM NaCl, 2.8 mM $CaCl_2$, 1.2 mM $MgCl_2$ and 5.4 mM KCl (pH 7.4)). An uptaking buffer (10 mM Tris-HEPES containing 137 mM NaCl, 0.1 mM $KH_2{}^{32}PO_4$ (0.5 mCi/mL), 2.8 mM $CaCl_2$, 1.2 mM $MgCl_2$ and 5.4 mM KCl (pH 7.4)) was added by 0.25 mL and the plate was incubated at 37°C for 5 min. Then, the uptaking buffer was removed and the each well was washed 3 times with 1 mL of ice-cooled stopping buffer (14 mM Tris-HEPES containing 137 mM NaCl (pH 7.4)). The cells were solubilized with 0.5N NaOH (0.5 mL) (10 mL was preserved for protein concentration measurement), and mixed with 3 mL of liquid scintillation solution A, then the radioactivity was measured by liquid scintillation counter. Protein concentration was determined by using BCA Protein Assay Reagent (Pierce). As shown in Fig. 15, recombinant Phosphatonin from CHO cell inhibited cellular uptake of phosphate in RPTEC.

## Industrial Applicability

The protein of this invention, a partial peptide thereof or a salt thereof has actions such as phosphaturic activity, hypophosphatemia-inducing activity, $Na^+$-Pi transport inhibitory activity, 25-hydroxy vitamin $D_3$-$1_\alpha$-hydroxylase inhibitory activity and 25-hydroxy vitamin $D_3$-24-hydroxylase-promoting activity in kidney cell and the like. Therefore, the protein of this invention, a partial peptide thereof or a salt thereof, and DNA of this invention are useful as an agent for the prophylaxis or treatment of diseases such as hyperphosphatemia, arteriosclerosis, acute coronary syndrome, heart failure, stroke, chronic glomerulonephritis, diabetic nephropathy, kidney failure and the like.

In addition, the DNA of this invention is useful as a gene diagnostic agent of diseases such as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure, hyperphosphatemia, arteriosclerosis, acute coronary syndrome, heart failure, stroke, chronic glomerulonephritis, diabetic nephropathy, kidney failure and the like.

Because an antibody against the protein of this invention, a partial peptide thereof or a salt thereof can specifically recognize the protein of this invention, a partial peptide thereof or a salt thereof, it can be used for quantification of the protein of this invention in a test solution, particularly quantificaton by sandwich immunoassay, and the like. Furthermore, an antibody capable of neutralizing the activity of the protein of this invention, a partial peptide thereof or a salt thereof can be used as an agent for the prophylaxis or treatment of diseases such as oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure, hyperphosphatemia, arteriosclerosis, acute coronary syndrome, heart failure, stroke, chronic glomerulonephritis, diabetic nephropathy, kidney failure and the like.

Moreover, the protein of this invention, a partial peptide thereof or a salt thereof is useful as a reagent for screening a receptor agonist/antagonist, a compound having a proteinase inhibitory action or a compound that promotes or inhibits intracellular signal transduction after the binding of the proteins to the receptor.

## Claims

1. A protein comprising an amino acid sequence identical or substantially identical to an amino acid sequence consisting of amino acid Nos. 17 - 525 of the amino acid sequence presented by SEQ ID:1, or a salt thereof.

2. A protein comprising an amino acid sequence consisting of amino acid Nos. 17 - 525 of the amino acid sequence presented by SEQ ID:1, or a salt thereof.

3. The protein of claim 1 or a salt thereof, which is a protein comprising an amino acid sequence identical or substantially identical to the amino acid sequence presented by SEQ ID:1, or a salt thereof.

4. The protein of claim 3 or a salt thereof, which is a protein having the amino acid sequence presented by SEQ ID: 1 or a salt thereof.

5. The protein of claim 1-4 or a salt thereof, which is a protein having a phosphaturic activity and/or a hypophosphatemia-inducing activity.

6. The protein of claim 1-4, which is a protein having at least one activity selected from (1) an activity that suppresses a sodium-dependent phosphorous ($Na^+$-Pi) transport activity in kidney, (2) an activity that suppresses a 25-hydroxy vitamin $D_3$-$1_\alpha$-hydroxylase activity in kidney, and (3) an activity that promotes a 25-hydroxy vitamin $D_3$-24-hydroxylase activity in kidney.

7. A partial peptide of the protein of claim 1, or a salt thereof.

8. A DNA comprising a DNA having a base sequence encoding the protein of claim 1 or the partial peptide of claim 7.

9. The DNA of claim 8, which has a base sequence presented by SEQ ID:2 or SEQ ID:3.

10. A recombinant vector comprising the DNA of claim 8.

11. A transformant retaining the recombinant vector of claim 10.

12. A method for manufacturing the protein of claim 1, the partial peptide of claim 7 or a salt thereof, which comprises culturing the transformant of claim 11 to produce and accumulate the protein of claim 1 or the partial peptide of claim 7 and harvesting the same.

13. A pharmaceutical agent comprising the protein of claim 1, the partial peptide of claim 7 or a salt thereof.

14. A pharmaceutical agent comprising the DNA of claim 8.

15. The pharmaceutical agent of claim 13 or 14, which is capable of regulating and improving abnormal concentration of phosphorus in blood.

16. An antibody against the protein of claim 1, the partial peptide of claim 7 or a salt thereof.

17. A method for quantifying the protein of claim 1, the partial peptide of claim 7 or a salt thereof, which comprises using the antibody of claim 16.

18. A method for diagnosing a disease related to the protein of claim 1, the partial peptide of claim 7 or a salt thereof, which comprises using the quantification method of claim 17.

19. A method for screening a receptor agonist or antagonist, which comprises using the protein of claim 1, the partial peptide of claim 7 or a salt thereof.

20. A screening kit for a receptor agonist or antagonist, which comprises the protein of claim 1, the partial peptide of claim 7 or a salt thereof.

21. A receptor agonist or antagonist which is obtained by the screening method of claim 19 or by the use of the screening kit of claim 20.

22. A method for screening a compound or a salt thereof having an inhibitory action on a proteinase that degrades the protein of claim 1 or the partial peptide of claim 7, which comprises using the protein of claim 1, the partial peptide of claim 7 or a salt thereof.

**23.** A screening kit for a compound or a salt thereof having an inhibitory action on a proteinase that degrades the protein of claim 1 or the partial peptide of claim 7, which comprises the protein of claim 1, the partial peptide of claim 7 or a salt thereof.

**24.** A compound or a salt thereof having an inhibitory action on a proteinase that degrades the protein of claim 1 or the partial peptide of claim 7, which is obtained by the screening method of claim 22 or by the use of the screening kit of claim 23.

**25.** The screening method of claim 19, which comprises measuring and comparing the amount of the protein of claim 1, the partial peptide of claim 7 or a salt thereof bound to a receptor or a partial peptide thereof, between (i) a case where the protein of claim 1, the partial peptide of claim 7 or a salt thereof is brought into contact with the receptor or a partial peptide thereof, and (ii) a case where the protein of claim 1, the partial peptide of claim 7 or a salt thereof and a test compound are brought into contact with the receptor or a partial peptide thereof.

**26.** The screening method of claim 19, which comprises measuring and comparing the amount of the protein of claim 1, the partial peptide of claim 7 or a salt thereof bound to a cell containing a receptor or a cell membrane fraction thereof, between (i) a case where the protein of claim 1, the partial peptide of claim 7 or a salt thereof is brought into contact with the cell containing the receptor or a cell membrane fraction thereof, and (ii) a case where the protein of claim 1, the partial peptide of claim 7 or a salt thereof and a test compound are brought into contact with the cell containing the receptor or a cell membrane fraction thereof.

**27.** The screening method of claim 19, which comprises measuring and comparing a cell stimulating activity via a receptor in a cell containing the receptor, a $Na^+$-Pi transport activity, a 25-hydroxy vitamin $D_3$-$1_\alpha$-hydroxylase activity or a 25-hydroxy vitamin $D_3$-24-hydroxylase activity, between (i) a case where the protein of claim 1, the partial peptide of claim 7 or a salt thereof is brought into contact with the cell containing the receptor, and (ii) a case where the protein of claim 1, the partial peptide of claim 7 or a salt thereof and a test compound are brought into contact with the cell containing the receptor.

**28.** A pharmaceutical agent comprising a receptor agonist obtained by the screening method of any of claim 19, claim 25, claim 26 and claim 27 or by the use of the screening kit of claim 20.

**29.** The pharmaceutical agent of claim 28, which is an agent for the prophylaxis or treatment of hyperphosphatemia, arteriosclerosis, acute coronary syndrome, heart failure, stroke, chronic glomerulonephritis, diabetic nephropathy or kidney failure.

**30.** A pharmaceutical agent comprising a receptor antagonist obtained by the screening method of any of claim 19, claim 25, claim 26 and claim 27 or by the use of the screening kit of claim 20.

**31.** The pharmaceutical agent of claim 30, which is an agent for the prophylaxis or treatment of oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis or kidney failure.

**32.** The screening method of claim 22, which comprises measuring and comparing a cell stimulating activity via a receptor in a cell containing the receptor, $Na^+$-Pi transport activity, a 25-hydroxy vitamin $D_3$-$1_\alpha$-hydroxylase activity or a 25-hydroxy vitamin $D_3$-24-hydroxylase activity, between (i) a case where the protein of claim 1, the partial peptide of claim 7 or a salt thereof is brought into contact with the cell containing the receptor in the presence of a proteinase that degrades the protein of claim 1, the partial peptide of claim 7 or a salt thereof, and (ii) a case where the protein of claim 1, the partial peptide of claim 7 or a salt thereof is brought into contact with the cell containing the receptor in the presence of a proteinase that degrades the protein of claim 1, the partial peptide of claim 7 or a salt thereof and a test compound.

**33.** A pharmaceutical agent comprising a compound or a salt thereof having an inhibitory action on a proteinase that degrades the protein of claim 1 or the partial peptide of claim 7, which is obtained by the screening method of claim 22 or claim 32 or by the use of the screening kit of claim 23.

**34.** A method for screening a compound or a salt thereof that promotes or inhibits intracellular signal transduction after binding of the protein of claim 1, the partial peptide of claim 7 or a salt thereof to a receptor, which comprises using the protein of claim 1, the partial peptide of claim 7 or a salt thereof.

**35.** A screening method of claim 34, which comprises measuring and comparing intracellular signal transduction after binding of the protein of claim 1, the partial peptide of claim 7 or a salt thereof to a receptor, between (i) a case where the protein of claim 1, the partial peptide of claim 7 or a salt thereof is brought into contact with a cell containing the receptor, and (ii) a case where the protein of claim 1, the partial peptide of claim 7 or a salt thereof and a test compound are brought into contact with the cell containing the receptor.

**36.** A screening kit for a compound or a salt thereof that promotes or inhibits intracellular signal transduction after binding of the protein of claim 1, the partial peptide of claim 7 or a salt thereof to a receptor, which comprises the protein of claim 1, the partial peptide of claim 7 or a salt thereof.

**37.** A compound or a salt thereof that promotes or inhibits intracellular signal transduction after binding of the protein of claim 1, the partial peptide of claim 7 or a salt thereof to a receptor, which is obtained by the screening method of claim 34 or claim 35, or by the use of the screening kit of claim 36.

**38.** A pharmaceutical agent comprising a compound or a salt thereof that promotes or inhibits intracellular signal transduction after binding of the protein of claim 1, the partial peptide of claim 7 or a salt thereof to a receptor, which is obtained by the screening method of claim 34 or claim 35, or by the use of the screening kit of claim 36.

**39.** The pharmaceutical agent of claim 38, which is an agent for the prophylaxis or treatment of oncogenic hypophosphatemic osteomalacia (OHO), X-linked hypophosphatemia (XLH), autosomal dominant hypophosphatemic rickets (ADHR), hereditary hypophosphatemic rickets with hypercalciuria (HHRH), vitamin D-resistant rachitis, osteomalacia, osteoporosis, renal osteodystrophy, secondary hyperparathyroidism, Paget's disease, renal Fanconi's syndrome, renal tubular acidosis, cystic fibrosis, fibrous cystic ostitis, kidney failure, hyperphosphatemia, arteriosclerosis, acute coronary syndrome, heart failure, stroke, chronic glomerulonephritis, diabetic nephropathy or kidney failure.

# FIG. 1

9 18 27 36 45 54

ATG CGA GTT TTC TGT GTG GGA CTA CTC CTT TTC AGT GTG ACC TGG GCA GCA CCA

Met Arg Val Phe Cys Val Gly Leu Leu Leu Phe Ser Val Thr Trp Ala Ala Pro

63 72 81 90 99 108

ACA TTT CAA CCA CAG ACT GAG AAA ACT AAG CAA AGC TGT GTG GAA GAG CAG AGG

Thr Phe Gln Pro Gln Thr Glu Lys Thr Lys Gln Ser Cys Val Glu Glu Gln Arg

117 126 135 144 153 162

CAG GAA GAA AAA AAC AAA GAC AAT ATT GGT TTT CAC CAT TTG GGC AAG AGA ATA

Gln Glu Glu Lys Asn Lys Asp Asn Ile Gly Phe His His Leu Gly Lys Arg Ile

171 180 189 198 207 216

AAT CAA GAG CTA TCA TCT AAA GAA AAT ATT GTC CAG GAA AGA AAG AAA GAT TTG

Asn Gln Glu Leu Ser Ser Lys Glu Asn Ile Val Gln Glu Arg Lys Lys Asp Leu

225 234 243 252 261 270

TCC CTT TCT GAA GCC AGT GAG AAT AAG GGA AGT AGT AAA TCT CAA AAT TAT TTC

Ser Leu Ser Glu Ala Ser Glu Asn Lys Gly Ser Ser Lys Ser Gln Asn Tyr Phe

279 288 297 306 315 324

ACA AAT AGA CAG AGA CTG AAT AAA GAA TAT AGT ATC AGT AAC AAA GAG AAT ACT

Thr Asn Arg Gln Arg Leu Asn Lys Glu Tyr Ser Ile Ser Asn Lys Glu Asn Thr

333 342 351 360 369 378

CAC AAT GGC CTG AGG ATG TCA ATT TAT CCT AAG TCA ACT GGG AAT AAA GGG TTT

His Asn Gly Leu Arg Met Ser Ile Tyr Pro Lys Ser Thr Gly Asn Lys Gly Phe

387 396 405 414 423 432

GAG GAT GGA GAT GAT GCT ATC AGC AAA CTA CAT GAC CAA GAA GAA TAT GGC GCA

Glu Asp Gly Asp Asp Ala Ile Ser Lys Leu His Asp Gln Glu Glu Tyr Gly Ala

441 450 459 468 477 486

GCT CTC ATC AGA AAT AAC ATG CAA CAT ATA ATG GGG CCA GTG ACT GCG ATT AAA

Ala Leu Ile Arg Asn Asn Met Gln His Ile Met Gly Pro Val Thr Ala Ile Lys

# FIG. 2

```
         495          504          513          522          531          540
CTC CTG GGG GAA GAA AAC AAA GAG AAC ACA CCT AGG AAT GTT CTA AAC ATA ATC
Leu Leu Gly Glu Glu Asn Lys Glu Asn Thr Pro Arg Asn Val Leu Asn Ile Ile
         549          558          567          576          585          594
CCA GCA AGT ATG AAT TAT GCT AAA GCA CAC TCG AAG GAT AAA AAG AAG CCT CAA
Pro Ala Ser Met Asn Tyr Ala Lys Ala His Ser Lys Asp Lys Lys Lys Pro Gln
         603          612          621          630          639          648
AGA GAT TCC CAA GCC CAG AAA AGT CCA GTA AAA AGC AAA AGC ACC CAT CGT ATT
Arg Asp Ser Gln Ala Gln Lys Ser Pro Val Lys Ser Lys Ser Thr His Arg Ile
         657          666          675          684          693          702
CAA CAC AAC ATT GAC TAC CTA AAA CAT CTC TCA AAA GTC AAA AAA ATC CCC AGT
Gln His Asn Ile Asp Tyr Leu Lys His Leu Ser Lys Val Lys Lys Ile Pro Ser
         711          720          729          738          747          756
GAT TTT GAA GGC AGC GGT TAT ACA GAT CTT CAA GAG AGA GGG GAC AAT GAT ATA
Asp Phe Glu Gly Ser Gly Tyr Thr Asp Leu Gln Glu Arg Gly Asp Asn Asp Ile
         765          774          783          792          801          810
TCT CCT TTC AGT GGG GAC GGC CAA CCT TTT AAG GAC ATT CCT GGT AAA GGA GAA
Ser Pro Phe Ser Gly Asp Gly Gln Pro Phe Lys Asp Ile Pro Gly Lys Gly Glu
         819          828          837          846          855          864
GCT ACT GGT CCT GAC CTA GAA GGC AAA GAT ATT CAA ACA GGG TTT GCA GGC CCA
Ala Thr Gly Pro Asp Leu Glu Gly Lys Asp Ile Gln Thr Gly Phe Ala Gly Pro
         873          882          891          900          909          918
AGT GAA GCT GAG AGT ACT CAT CTT GAC ACA AAA AAG CCA GGT TAT AAT GAG ATC
Ser Glu Ala Glu Ser Thr His Leu Asp Thr Lys Lys Pro Gly Tyr Asn Glu Ile
```

# FIG. 3

927 936 945 954 963 972
CCA GAG AGA GAA GAA AAT GGT GGA AAT ACC ATT GGA ACT AGG GAT GAA ACT GCG
Pro Glu Arg Glu Glu Asn Gly Gly Asn Thr Ile Gly Thr Arg Asp Glu Thr Ala
981 990 999 1008 1017 1026
AAA GAG GCA GAT GCT GTT GAT GTC AGC CTT GTA GAG GGC AGC AAC GAT ATC ATG
Lys Glu Ala Asp Ala Val Asp Val Ser Leu Val Glu Gly Ser Asn Asp Ile Met
1035 1044 1053 1062 1071 1080
GGT AGT ACC AAT TTT AAG GAG CTC CCT GGA AGA GAA GGA AAC AGA GTG GAT GCT
Gly Ser Thr Asn Phe Lys Glu Leu Pro Gly Arg Glu Gly Asn Arg Val Asp Ala
1089 1098 1107 1116 1125 1134
GGC AGC CAA AAT GCT CAC CAA GGG AAG GTT GAG TTT CAT TAC CCT CCT GCA CCC
Gly Ser Gln Asn Ala His Gln Gly Lys Val Glu Phe His Tyr Pro Pro Ala Pro
1143 1152 1161 1170 1179 1188
TCA AAA GAG AAA AGA AAA GAA GGC AGT AGT GAT GCA GCT GAA AGT ACC AAC TAT
Ser Lys Glu Lys Arg Lys Glu Gly Ser Ser Asp Ala Ala Glu Ser Thr Asn Tyr
1197 1206 1215 1224 1233 1242
AAT GAA ATT CCT AAA AAT GGC AAA GGC AGT ACC AGA AAG GGT GTA GAT CAT TCT
Asn Glu Ile Pro Lys Asn Gly Lys Gly Ser Thr Arg Lys Gly Val Asp His Ser
1251 1260 1269 1278 1287 1296
AAT AGG AAC CAA GCA ACC TTA AAT GAA AAA CAA AGG TTT CCT AGT AAG GGC AAA
Asn Arg Asn Gln Ala Thr Leu Asn Glu Lys Gln Arg Phe Pro Ser Lys Gly Lys
1305 1314 1323 1332 1341 1350
AGT CAG GGC CTG CCC ATT CCT TCT CGT GGT CTT GAT AAT GAA ATC AAA AAC GAA
Ser Gln Gly Leu Pro Ile Pro Ser Arg Gly Leu Asp Asn Glu Ile Lys Asn Glu
1359 1368 1377 1386 1395 1404
ATG GAT TCC TTT AAT GGC CCC AGT CAT GAG AAT ATA ATA ACA CAT GGC AGA AAA
Met Asp Ser Phe Asn Gly Pro Ser His Glu Asn Ile Ile Thr His Gly Arg Lys

# FIG. 4

```
       1413        1422        1431        1440        1449        1458
TAT CAT TAT GTA CCC CAC AGA CAA AAT AAT TCT ACA CGG AAT AAG GGT ATG CCA
Tyr His Tyr Val Pro His Arg Gln Asn Asn Ser Thr Arg Asn Lys Gly Met Pro
       1467        1476        1485        1494        1503        1512
CAA GGG AAA GGC TCC TGG GGT AGA CAA CCC CAT TCC AAC AGG AGG TTT AGT TCC
Gln Gly Lys Gly Ser Trp Gly Arg Gln Pro His Ser Asn Arg Arg Phe Ser Ser
       1521        1530        1539        1548        1557        1566
CGT AGA AGG GAT GAC AGT AGT GAG TCA TCT GAC AGT GGC AGT TCA AGT GAG AGC
Arg Arg Arg Asp Asp Ser Ser Glu Ser Ser Asp Ser Gly Ser Ser Ser Glu Ser
       1575
GAT GGT GAC
Asp Gly Asp
```

# FIG. 5

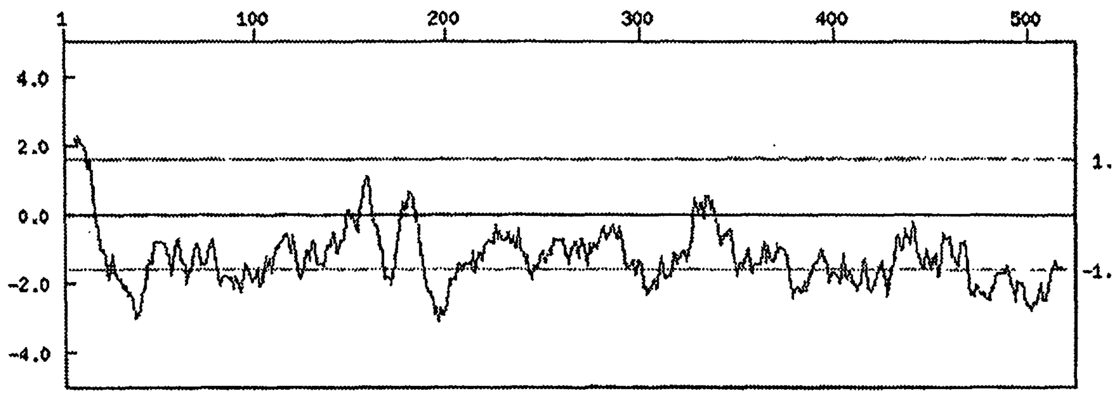

FIG. 6

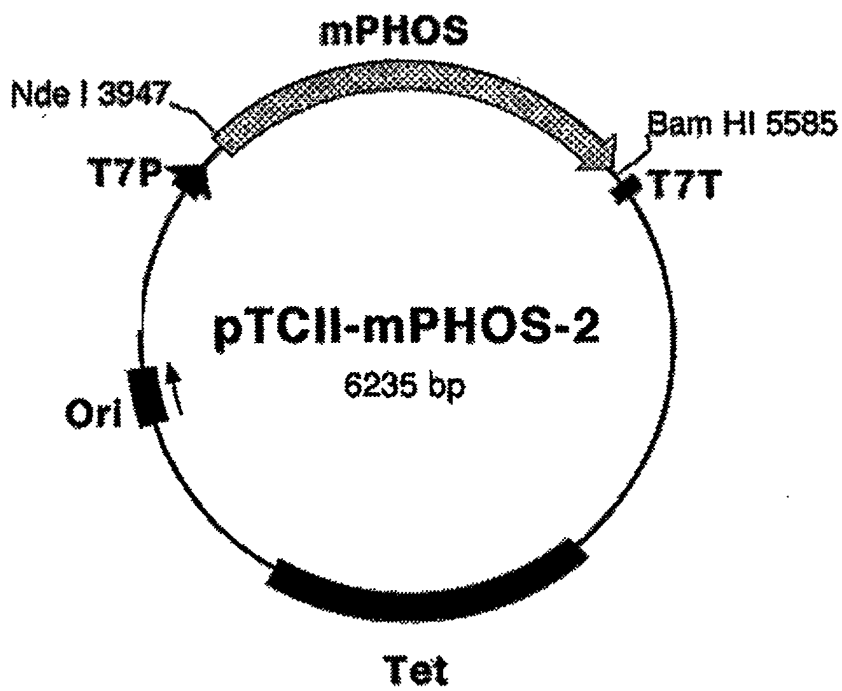
mPHOS
Nde I 3947
Bam HI 5585
T7P
T7T
pTCII-mPHOS-2
6235 bp
Ori
Tet

# FIG. 7

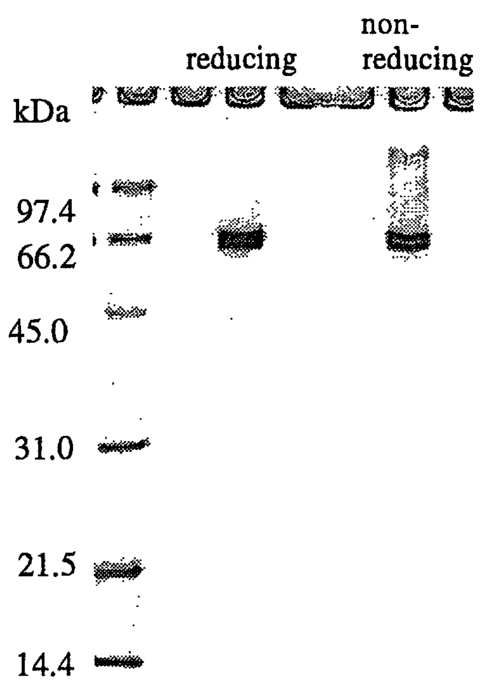


SDS PAGE
Multi gel 10/20
reducing conditions
100mM DTT 100°C
2min
amount applied
1μg each

# FIG. 8

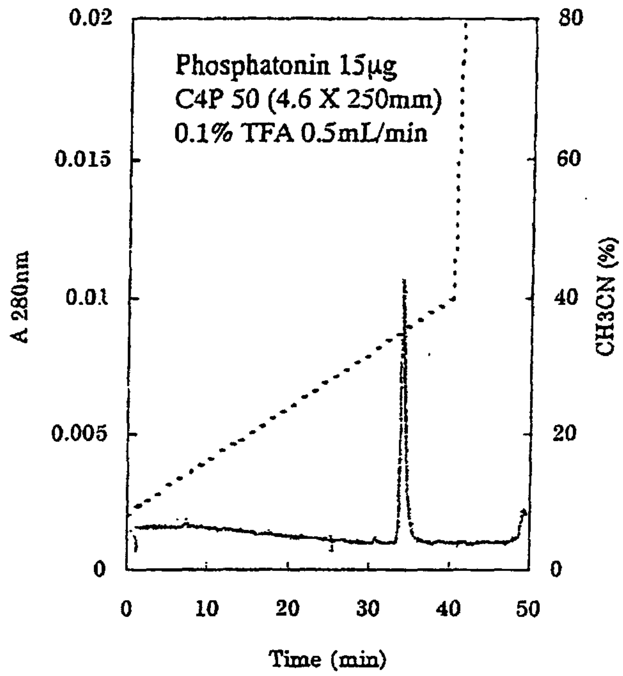
Phosphatonin 15µg
C4P 50 (4.6 X 250mm)
0.1% TFA 0.5mL/min
A 280nm
0.02
0.015
0.01
0.005
0
80
60
40
20
0
CH3CN (%)
0
10
20
30
40
50
Time (min)

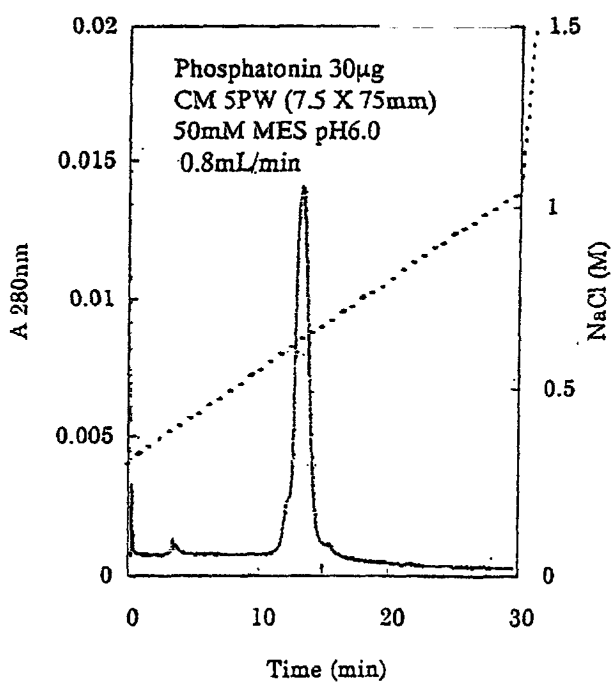
Phosphatonin 30µg
CM 5PW (7.5 X 75mm)
50mM MES pH6.0
0.8mL/min
A 280nm
0.02
0.015
0.01
0.005
0
1.5
1
0.5
0
NaCl (M)
0
10
20
30
Time (min)

# FIG. 9

| | | | | | | | | | | |
|---|---|---|---|---|---|---|---|---|---|---|
| Met | Ala | Pro | Thr | Phe | Gln | Pro | Gln | Thr | Glu | Lys |
| 26.2 | 26.9 | 17.7 | 15.5 | 18.6 | 22.2 | 14.1 | 16.8 | 11.1 | 13.6 | |
| | 13.3 | 8.3 | 7.6 | 9.8 | 9.6 | 7.7 | - | 6.4 | 9.7 | 5.9 |

analyzed by 100pmol Apply ABI Procise 492 (pmol)

# FIG. 10

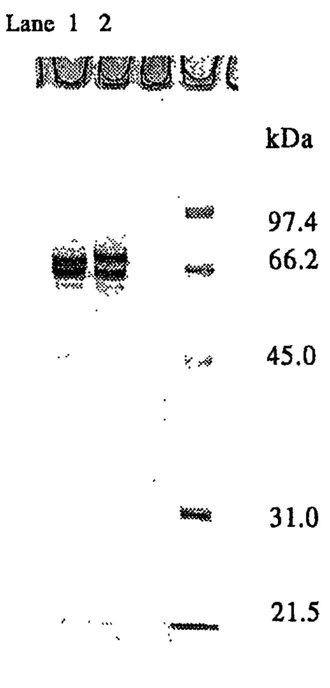


SDS PAGE
Multi gel 12.5
reducing conditions
100mM DTT 100°C 2min
amount applied 2μg each
Lane 1 Phosphatonin
Lane 2 Phosphorylated Phosphatonin

# FIG. 11

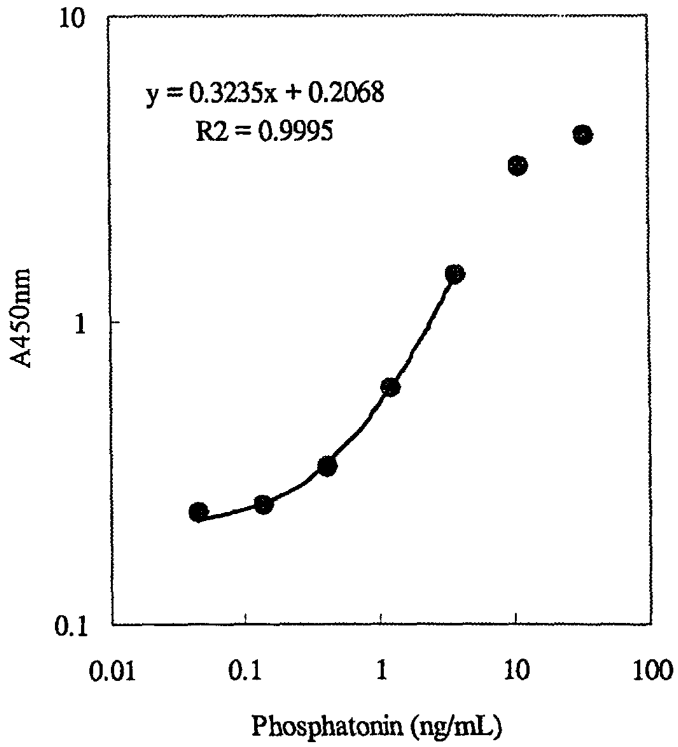


detection limit: 0.1ng/mL (1.8fmol/mL)
detection range: 0.1-3ng/mL (1.8-53fmol/mL)

FIG. 12

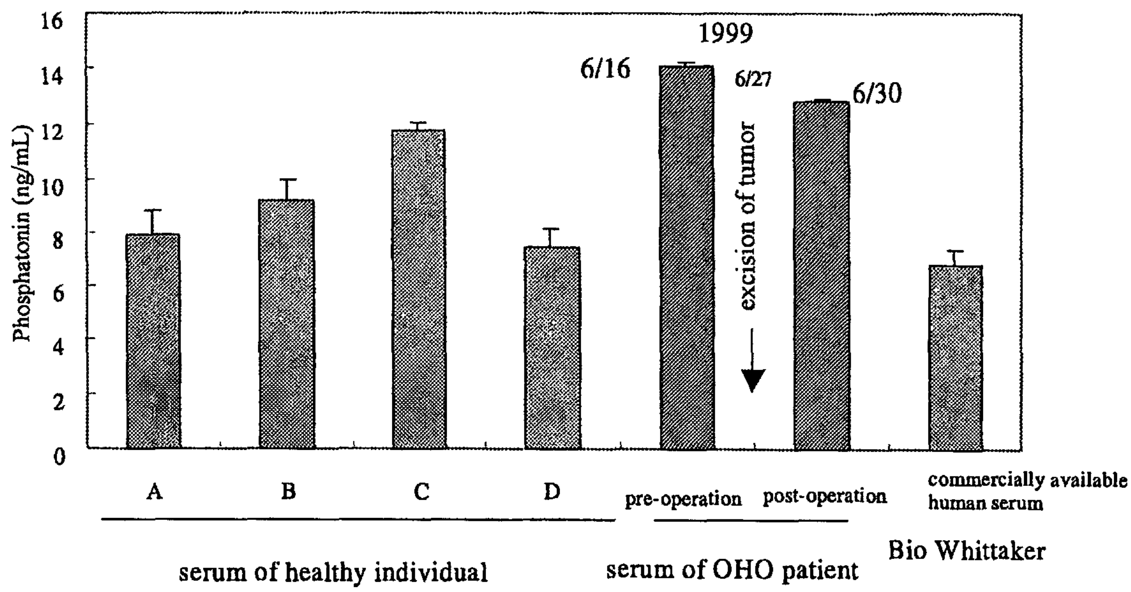
Phosphatonin (ng/mL)
16
14
12
10
8
6
4
2
0
1999
6/16
6/27
6/30
excision of tumor
A
B
C
D
pre-operation
post-operation
commercially available human serum
serum of healthy individual
serum of OHO patient
Bio Whittaker

FIG. 13

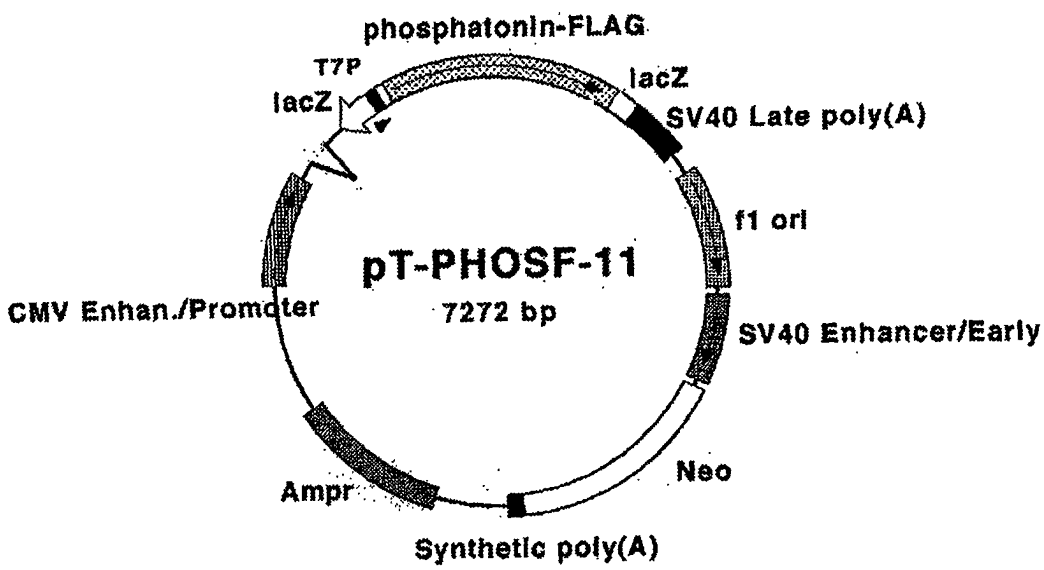
phosphatonin-FLAG
T7P
lacZ
lacZ
SV40 Late poly(A)
f1 ori
pT-PHOSF-11
7272 bp
CMV Enhan./Promoter
SV40 Enhancer/Early
Neo
Ampr
Synthetic poly(A)

# FIG. 14

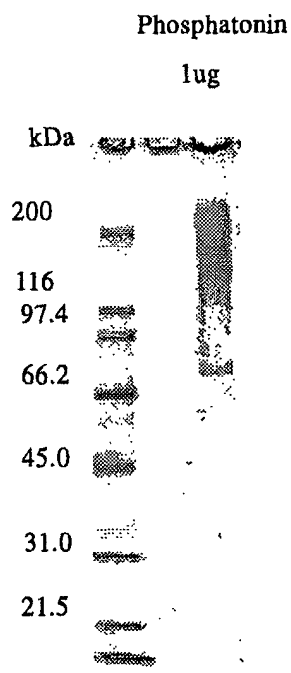


SDS PAGE CBB staining
Multi gel 4/20
reducing conditions
100mM DTT 100°C 2min
amount applied 1μg

FIG. 15

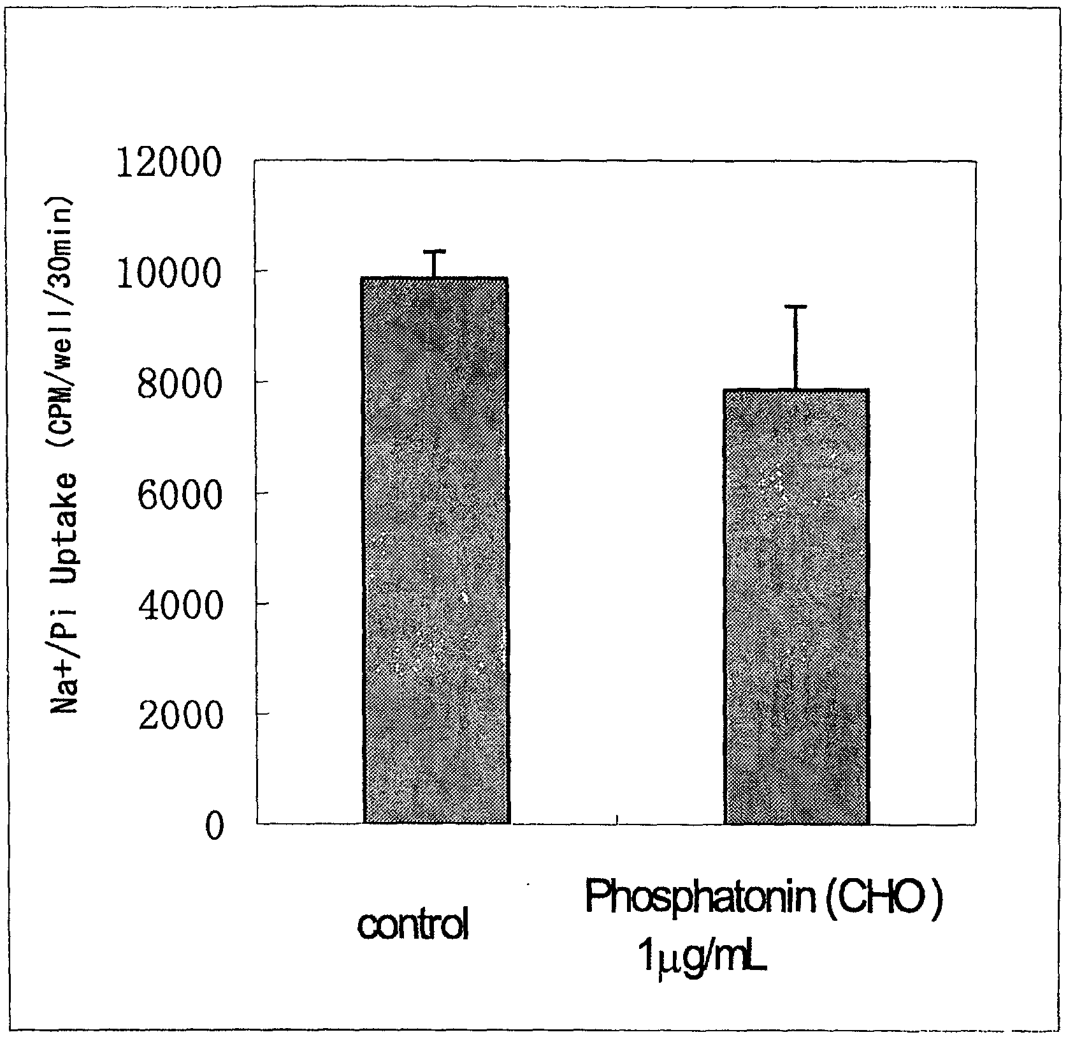
Na+/Pi Uptake (CPM/well/30min)
12000
10000
8000
6000
4000
2000
0
control
Phosphatonin (CHO)
1μg/mL

## INTERNATIONAL SEARCH REPORT

International application No.
PCT/JP01/05263

A. CLASSIFICATION OF SUBJECT MATTER
Int.Cl[7] C12N15/12, C12P21/02, C07K14/47, C12N5/10, C07K16/18, A61K45/00, A61P3/12, G01N33/566, G01N33/50, G01N33/15

According to International Patent Classification (IPC) or to both national classification and IPC

B. FIELDS SEARCHED
Minimum documentation searched (classification system followed by classification symbols)
Int.Cl[7] C12N15/12, C12P21/02, C07K14/47, C12N5/10, C07K16/18, A61K45/00, A61P3/12, G01N33/566, G01N33/50, G01N33/15

Documentation searched other than minimum documentation to the extent that such documents are included in the fields searched

Electronic data base consulted during the international search (name of data base and, where practicable, search terms used)
MEDLINE, CAPLUS (CAS), BIOSIS (CAS), SwissProt/PIR/GeneSeq, Genbank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X<br>Y | WO 99/60017 A2 (ROWE P.), 25 November, 1999 (25.11.99), & EP 1086225 A2 | 1-17,19-39<br>1-6,8-17,19-39 |
| Y | ROWE P. S., "Candidate 56 and 58 kDa protein(s) responsible for mediating the renal defects in oncogenic hypophosphatemic osteomalacia", Bone, (1996), Vol.18, No.2, pages 159 to 169 | 1-6,8-17,19-39 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>05 September, 2001 (05.09.01) | Date of mailing of the international search report<br>18 September, 2001 (18.09.01) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP01/05263

**Box I Observations where certain claims were found unsearchable (Continuation of item 1 of first sheet)**

This international search report has not been established in respect of certain claims under Article 17(2)(a) for the following reasons:

1. ☒ Claims Nos.: 18
because they relate to subject matter not required to be searched by this Authority, namely:

The invention as set forth in claim 18 pertains diagnostic methods to be practiced on the human body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

SEQUENCE LISTING

<110> Takeda Chemical Industries, Ltd.

<120> Novel Protein and its DNA

<130> 2738WOOP

<150> JP 2000-191088

<151> 2000-06-21

<160> 10

<210> 1

<211> 525

<212> PRT

<213> Human

<400> 1

Met Arg Val Phe Cys Val Gly Leu Leu Leu Phe Ser Val Thr Trp Ala
1 5 10 15

Ala Pro Thr Phe Gln Pro Gln Thr Glu Lys Thr Lys Gln Ser Cys Val
20 25 30

Glu Glu Gln Arg Gln Glu Glu Lys Asn Lys Asp Asn Ile Gly Phe His
35 40 45

His Leu Gly Lys Arg Ile Asn Gln Glu Leu Ser Ser Lys Glu Asn Ile
50 55 60

Val Gln Glu Arg Lys Lys Asp Leu Ser Leu Ser Glu Ala Ser Glu Asn
65 70 75 80

Lys Gly Ser Ser Lys Ser Gln Asn Tyr Phe Thr Asn Arg Gln Arg Leu
85 90 95

Asn Lys Glu Tyr Ser Ile Ser Asn Lys Glu Asn Thr His Asn Gly Leu
100 105 110

Arg Met Ser Ile Tyr Pro Lys Ser Thr Gly Asn Lys Gly Phe Glu Asp
115 120 125

Gly Asp Asp Ala Ile Ser Lys Leu His Asp Gln Glu Glu Tyr Gly Ala

130 135 140

Ala Leu Ile Arg Asn Asn Met Gln His Ile Met Gly Pro Val Thr Ala

145 150 155 160

Ile Lys Leu Leu Gly Glu Glu Asn Lys Glu Asn Thr Pro Arg Asn Val

165 170 175

Leu Asn Ile Ile Pro Ala Ser Met Asn Tyr Ala Lys Ala His Ser Lys

180 185 190

Asp Lys Lys Lys Pro Gln Arg Asp Ser Gln Ala Gln Lys Ser Pro Val

195 200 205

Lys Ser Lys Ser Thr His Arg Ile Gln His Asn Ile Asp Tyr Leu Lys

210 215 220

His Leu Ser Lys Val Lys Lys Ile Pro Ser Asp Phe Glu Gly Ser Gly

225 230 235 240

Tyr Thr Asp Leu Gln Glu Arg Gly Asp Asn Asp Ile Ser Pro Phe Ser

245 250 255

Gly Asp Gly Gln Pro Phe Lys Asp Ile Pro Gly Lys Gly Glu Ala Thr

260 265 270

Gly Pro Asp Leu Glu Gly Lys Asp Ile Gln Thr Gly Phe Ala Gly Pro

275 280 285

Ser Glu Ala Glu Ser Thr His Leu Asp Thr Lys Lys Pro Gly Tyr Asn

290 295 300

Glu Ile Pro Glu Arg Glu Glu Asn Gly Gly Asn Thr Ile Gly Thr Arg

305 310 315 320

Asp Glu Thr Ala Lys Glu Ala Asp Ala Val Asp Val Ser Leu Val Glu

325 330 335

Gly Ser Asn Asp Ile Met Gly Ser Thr Asn Phe Lys Glu Leu Pro Gly

340 345 350

Arg Glu Gly Asn Arg Val Asp Ala Gly Ser Gln Asn Ala His Gln Gly

355 360 365

Lys Val Glu Phe His Tyr Pro Pro Ala Pro Ser Lys Glu Lys Arg Lys
370 375 380

Glu Gly Ser Ser Asp Ala Ala Glu Ser Thr Asn Tyr Asn Glu Ile Pro
385 390 395 400

Lys Asn Gly Lys Gly Ser Thr Arg Lys Gly Val Asp His Ser Asn Arg
405 410 415

Asn Gln Ala Thr Leu Asn Glu Lys Gln Arg Phe Pro Ser Lys Gly Lys
420 425 430

Ser Gln Gly Leu Pro Ile Pro Ser Arg Gly Leu Asp Asn Glu Ile Lys
435 440 445

Asn Glu Met Asp Ser Phe Asn Gly Pro Ser His Glu Asn Ile Ile Thr
450 455 460

His Gly Arg Lys Tyr His Tyr Val Pro His Arg Gln Asn Asn Ser Thr
465 470 475 480

Arg Asn Lys Gly Met Pro Gln Gly Lys Gly Ser Trp Gly Arg Gln Pro
485 490 495

His Ser Asn Arg Arg Phe Ser Ser Arg Arg Arg Asp Asp Ser Ser Glu
500 505 510

Ser Ser Asp Ser Gly Ser Ser Ser Glu Ser Asp Gly Asp
515 520 525

<210> 2

<211> 1575

<212> DNA

<213> Human

<400> 2

atgcgagttt tctgtgtggg actactcctt ttcagtgtga cctgggcagc accaacattt 60
caaccacaga ctgagaaaac taagcaaagc tgtgtggaag agcagaggca ggaagaaaaa 120
aacaaagaca atattggttt tcaccatttg ggcaagagaa taaatcaaga gctatcatct 180
aaagaaaata ttgtccagga aagaaagaaa gatttgtccc tttctgaagc cagtgagaat 240

aagggaagta gtaaatctca aaattatttc acaaatagac agagactgaa taaagaatat 300
agtatcagta acaaagagaa tactcacaat ggcctgagga tgtcaattta tcctaagtca 360
actgggaata aagggtttga ggatggagat gatgctatca gcaaactaca tgaccaagaa 420
gaatatggcg cagctctcat cagaaataac atgcaacata taatggggcc agtgactgcg 480
attaaactcc tgggggaaga aaacaaagag aacacaccta ggaatgttct aaacataatc 540
ccagcaagta tgaattatgc taaagcacac tcgaaggata aaaagaagcc tcaaagagat 600
tcccaagccc agaaaagtcc agtaaaaagc aaaagcaccc atcgtattca acacaacatt 660
gactacctaa aacatctctc aaaagtcaaa aaaatcccca gtgattttga aggcagcggt 720
tatacagatc ttcaagagag aggggacaat gatatatctc ctttcagtgg ggacggccaa 780
ccttttaagg acattcctgg taaaggagaa gctactggtc ctgacctaga aggcaaagat 840
attcaaacag ggtttgcagg cccaagtgaa gctgagagta ctcatcttga cacaaaaaag 900
ccaggttata atgagatccc agagagagaa gaaaatggtg gaaataccat tggaactagg 960
gatgaaactg cgaaagaggc agatgctgtt gatgtcagcc ttgtagaggg cagcaacgat 1020
atcatgggta gtaccaattt taaggagctc cctggaagag aaggaaacag agtggatgct 1080
ggcagccaaa atgctcacca agggaaggtt gagtttcatt accctcctgc accctcaaaa 1140
gagaaaagaa aagaaggcag tagtgatgca gctgaaagta ccaactataa tgaaattcct 1200
aaaaatggca aaggcagtac cagaaagggt gtagatcatt ctaataggaa ccaagcaacc 1260
ttaaatgaaa aacaaaggtt tcctagtaag ggcaaaagtc agggcctgcc cattccttct 1320
cgtggtcttg ataatgaaat caaaaacgaa atggattcct ttaatggccc cagtcatgag 1380
aatataataa cacatggcag aaaatatcat tatgtacccc acagacaaaa taattctaca 1440
cggaataagg gtatgccaca agggaaaggc tcctggggta gacaacccca ttccaacagg 1500
aggtttagtt cccgtagaag ggatgacagt agtgagtcat ctgacagtgg cagttcaagt 1560
gagagcgatg gtgac 1575

<210> 3

<211> 1662

<212> DNA

<213> Human

<400> 3

ctcaaagatg cgagtttttct gtgtgggact actcctttttc agtgtgacct gggcagcacc 60

aacatttcaa ccacagactg agaaaactaa gcaaagctgt gtggaagagc agaggcagga 120
agaaaaaaac aaagacaata ttggttttca ccatttgggc aagagaataa atcaagagct 180
atcatctaaa gaaaatattg tccaggaaag aaagaaagat ttgtcccttt ctgaagccag 240
tgagaataag ggaagtagta aatctcaaaa ttatttcaca aatagacaga gactgaataa 300
agaatatagt atcagtaaca aagagaatac tcacaatggc ctgaggatgt caatttatcc 360
taagtcaact gggaataaag ggtttgagga tggagatgat gctatcagca aactacatga 420
ccaagaagaa tatggcgcag ctctcatcag aaataacatg caacatataa tggggccagt 480
gactgcgatt aaactcctgg gggaagaaaa caaagagaac acacctagga atgttctaaa 540
cataatccca gcaagtatga attatgctaa agcacactcg aaggataaaa agaagcctca 600
aagagattcc caagcccaga aaagtccagt aaaaagcaaa agcacccatc gtattcaaca 660
caacattgac tacctaaaac atctctcaaa agtcaaaaaa atccccagtg atttgaagg 720
cagcggttat acagatcttc aagagagagg ggacaatgat atatctcctt tcagtgggga 780
cggccaacct tttaaggaca ttcctggtaa aggagaagct actggtcctg acctagaagg 840
caaagatatt caaacagggt ttgcaggccc aagtgaagct gagagtactc atcttgacac 900
aaaaaagcca ggttataatg agatcccaga gagagaagaa aatggtggaa ataccattgg 960
aactagggat gaaactgcga aagaggcaga tgctgttgat gtcagccttg tagagggcag 1020
caacgatatc atgggtagta ccaattttaa ggagctccct ggaagagaag gaaacagagt 1080
ggatgctggc agccaaaatg ctcaccaagg gaaggttgag tttcattacc ctcctgcacc 1140
ctcaaaagag aaaagaaaag aaggcagtag tgatgcagct gaaagtacca actataatga 1200
aattcctaaa aatggcaaag gcagtaccag aaagggtgta gatcattcta ataggaacca 1260
agcaacctta aatgaaaaac aaaggtttcc tagtaagggc aaaagtcagg gcctgcccat 1320
tccttctcgt ggtcttgata atgaaatcaa aaacgaaatg gattccttta atggccccag 1380
tcatgagaat ataataacac atggcagaaa atatcattat gtaccccaca gacaaaataa 1440
ttctacacgg aataagggta tgccacaagg gaaaggctcc tggggtagac aaccccattc 1500
caacaggagg tttagttccc gtagaaggga tgacagtagt gagtcatctg acagtggcag 1560
ttcaagtgag agcgatggtg actagtccac caggagttcc cagcggggtg acagtctgaa 1620
gacctcgtca cctgtgagtt gatgtagagg agagccacct ga 1662

<210> 4

<211> 19

<212> DNA

<213> Artificial Sequence

<220>

<223> Primer

<400> 4

ggaaacagct atgaccatg 19

<210> 5

<211> 30

<212> DNA

<213> Artificial Sequence

<220>

<223> Primer

<400> 5

tcaggtggct ctcctctaca tcaactcaca 30

<210> 6

<211> 28

<212> DNA

<213> Artificial Sequence

<220>

<223> Primer

<400> 6

ctcaaagatg cgagttttct gtgtggga

<210> 7

<211> 60

<212> DNA

<213> Artificial Sequence

<220>

<223> Oligo(dt)$_{18}$-Linker Primer

<400> 7

gagagagaga gagagagaga acgcgtcgac tcgagcggcc gcggaccgtt ttttttttt 60

<210> 8

<211> 28

<212> DNA

<213> Artificial Sequence

<220>

<223> Primer

<400> 8

catatggcac caacatttca accacaga 28

<210> 9

<211> 22

<212> DNA

<213> Artificial Sequence

<220>

<223> Primer

<400> 9

ctctcgtcga catcaactca ca 22

<210> 10

<211> 48

<212> DNA

<213> Artificial Sequence

<220>

<223> Primer

<400> 10

ctacttatcg tcgtcatcct tgtaatcgtc accatcgctc tcacttga 48